(19) Europäisches Patentamt
European Patent Office
Office européen des brevets

(11) EP 0 391 644 B1

(12) EUROPEAN PATENT SPECIFICATION

(45) Date of publication and mention of the grant of the patent: **19.06.1996 Bulletin 1996/25**

(21) Application number: **90303510.3**

(22) Date of filing: **02.04.1990**

(51) Int Cl.⁶: **C07D 277/14**, C07D 277/36, C07D 207/26, C07D 333/32, C07D 333/48, A61K 31/425, A61K 31/425

# (54) Aryl-substituted rhodanine derivatives

Arylsubstituierte Rhodaninderivate

Dérivés de rhodanine arylsubstitués

(84) Designated Contracting States:
**AT BE CH DE DK ES FR GB GR IT LI NL SE**

(30) Priority: **07.04.1989 US 335063**

(43) Date of publication of application:
**10.10.1990 Bulletin 1990/41**

(73) Proprietor: **ELI LILLY AND COMPANY**
**Indianapolis, Indiana 46285 (US)**

(72) Inventor: **Panetta, Jill Ann**
**Zionsville, Indiana 46077 (US)**

(74) Representative: **Tapping, Kenneth George et al**
**Lilly Industries Limited**
**European Patent Operations**
**Erl Wood Manor**
**Windlesham Surrey GU20 6PH (GB)**

(56) References cited:
**EP-A- 0 211 670** **EP-A- 0 343 643**

- **PATENT ABSTRACTS OF JAPAN, vol. 11, no. 232 (C-437)(2679), 29 July 1987; & JP-A-62 45 553**
- **Chemical and Pharmaceutical Bulletin, vol. 34, no. 4, April 1986, Tokyo, pages 1619-1627; Ikuo Katsumi et al.: "Studies on Styrene Derivatives II.Synthesis and Antiinflammatory Activity of 3,5-Di-tert-butyl-4-hydroxystyrenes"**
- **PATENT ABSTRACTS OF JAPAN, vol. 11, no. 206 (C-433)[2653], 03 July 1987; & JP-A-62 29 570**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 391 644 B1

## Description

This invention relates to novel aryl-substituted rhodanine derivatives which are useful in the treatment of antiinflammatory conditions, ameliorating ischemia-induced cell damage, as well as treating dystrophy in mammals.

Mammals, both humans and animals, are known to suffer from various conditions involving inflammation with concomitant swelling, tenderness, decreased mobility, pain, and fever. While a number of antiinflammatory agents are effective in the symptomatic treatment of such inflammatory conditions as rheumatoid arthritis, rheumatoid spondylitis, osteoarthritis, degenerative joint diseases, and the like, many such agents have a number of undesirable side effects, such as gastric irritation and the like.

The etiology and pathogenesis of rheumatic and arthritic diseases remain obscure. Meanwhile, the need continues for safer, better calibrated drugs which will slow the process and alleviate the symptoms of inflammatory diseases. For example, in rheumatoid arthritis, any agent which reduces the inflammation is important in lessening or delaying the development of crippling.

EP-A-343643, published after the priority date of this application, discloses arylmethylenyl derivatives of thiazolidinones, imidazolidinones and oxazolidinones having anti-allergy and anti-inflamatory activity.

Patent Abstracts of Japan, 11, No.206, (C-433) (2653) discloses 3,5-diisopropylbenzylidine heterocyclic compounds having pharmaceutical activites.

The present invention relates to certain aryl-substituted rhodanine derivatives which are useful as antiinflammatory agents and in slowing the development of arthritic conditions. Accordingly, one aspect of the present invention provides compounds, and pharmaceutically acceptable salts thereof, of the formula (I)

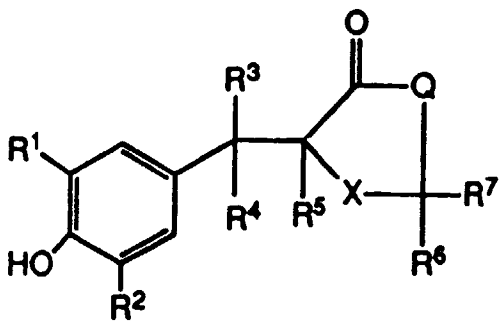

(I)

wherein:

$R^1$ and $R^2$ are each independently hydrogen, $C_1$-$C_6$ alkyl, $C_1$-$C_6$ alkoxy or

$$-C_1-C_4\ alkyl-O-\overset{O}{\overset{\|}{C}}-(C_1-C_4\ alkyl);$$

$R^3$ is hydrogen or $C_1$-$C_6$ alkyl;

$R^4$ and $R^5$ are each hydrogen, or when taken together form a bond;

$R^6$ and $R^7$ are each hydrogen or when taken together are =S, or when one of $R^6$ or $R^7$ is hydrogen, the other is -OH or $-SCH_3$;

X is

$$-\overset{(O)_m}{\overset{\|}{S}}-,$$

where m is 0, 1 or 2; and

Q is $-CH_2-$ -O- or $NR^8$ where $R^8$ is hydrogen, $C_1$-$C_6$ alkyl, $C_3$-$C_8$ cycloalkyl, $C_2$-$C_6$ alkenyl, $-SO_2CH_3$ or $-(CH_2)_n-Y$, where n is an integer from 0 to 3, both inclusive, and Y is cyano, $OR^9$,

$$-\overset{O}{\overset{\|}{C}}R^{10},$$

tetrazolyl, $-NR^{11}R^{12}$, -SH, $-S(C_1-C_4\ alkyl)$ or

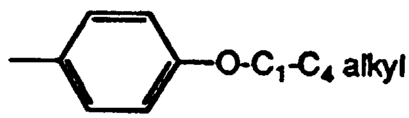

where $R^9$ is hydrogen, $C_1$-$C_4$ alkyl, tosyl or

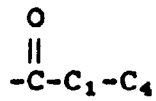

alkyl; $R^{10}$ is $C_1$-$C_4$ alkyl, $C_1$-$C_4$ alkoxy or $-NH_2$; $R^{11}$ and $R^{12}$ are each independently hydrogen, $C_1$-$C_6$ alkyl, $C_2$-$C_6$ alkenyl, $C_2$-$C_6$ alkynyl, $-(CH_2)_qOH$, $-(CH_2)_q-N(C_1-C_4\ alkyl)_2$, $-(CH_2)_q-S(C_1-C_4\ alkyl)$ or

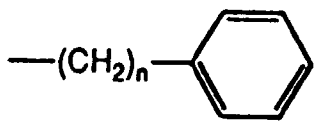

where q is an integer from 1 to 6, both inclusive, and n is as defined above; or $R^{11}$ and $R^{12}$ taken together form a morpholinyl, piperidinyl, piperazinyl or an N-methyl-piperazinyl ring;
with the proviso that when Q is -O- or $NR^8$ ($R^8$ is hydrogen or $C_1$-$C_4$ alkyl), $R^3$ is hydrogen, $R^4$ and $R^5$ are each hydrogen or when taken together form a bond, $R^6$ and $R^7$ are each hydrogen or when taken together are =S and X is

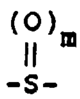

(where m is 0), $R^1$ and $R^2$ cannot both be a t-butyl group;
and when Q is $NR^8$ ($R^8$ is hydrogen), $R^3$ is hydrogen, $R^6$ and $R^7$ taken together are =S and X is $-S(O)_m-$ (where m is 0), $R^4$ and $R^5$ must both be hydrogen;
and when Q is $NR^8$ [$R^8$ is hydrogen or $-(CH_2)_n-Y$, where n is 0 and Y is $CO(C_1-C_4\ alkyl)$], $R^3$ is hydrogen, $R^4$ and $R^5$ taken together form a bond, $R^6$ and $R^7$ are each hydrogen and X is $-S(O)_m-$ (where m is 0), $R^1$ and $R^2$ cannot both be an isopropyl group.

In addition to the compounds of Formula I, a second aspect of the present invention provides a method of treating inflammation and arthritis in a mammal in need of such treatment which comprises administering to said mammal a therapeutically effective amount of a compound, or pharmaceutically acceptable salt thereof, of formula I.

According to a further aspect of the present invention, there is provided a pharmaceutical composition which comprises as active ingredient a compound of formula I as defined above, or a pharmaceutically acceptable salt thereof, in association with one or more pharmaceutically acceptable diluents, excipients or carriers therefor.

Moreover, it has been discovered that compounds of formula II

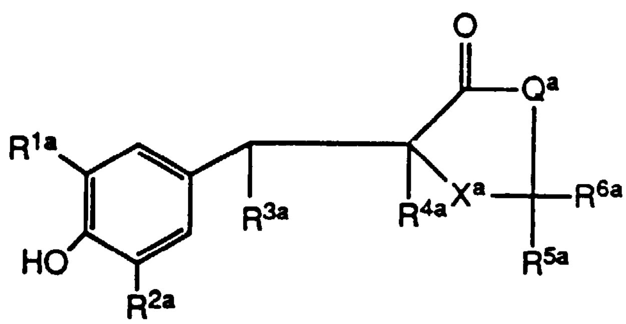

(II)

wherein:

$R^{1a}$ and $R^{2a}$ are each independently $C_1$-$C_6$ alkyl;
$R^{3a}$ and $R^{4a}$ are each hydrogen or when taken together form a bond;
$R^{5a}$ and $R^{6a}$ are each hydrogen or when taken together are =O;
$X^a$ is $-CH_2-$ or

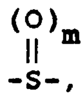

where m is as defined for formula I;

$Q^a$ is $NR^{7a}$;

$R^{7a}$ is hydrogen, $C_1$-$C_6$ alkyl, or $-(CH_2)_n-Y^a$, where n is as defined for formula I and $Y^a$ is cyano, $OR^{8a}$,

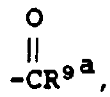

-SH, -S($C_1$-$C_4$ alkyl), tetrazolyl, $-NR^{10a}R^{11a}$ or

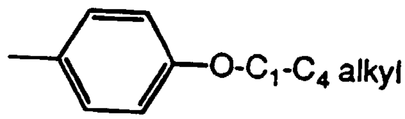

where $R^{8a}$ is hydrogen, $C_1$-$C_4$ alkyl, or

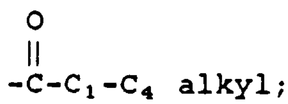

$R^{9a}$ is $-N_2$ or -OH; and $R^{10a}$ and $R^{11a}$ are each independently hydrogen, $C_1$-$C_6$ alkyl, $C_2$-$C_6$ alkenyl or $C_2$-$C_6$ alkynyl;

with the proviso that when $R^{3a}$ and $R^{4a}$ taken together form a bond, $R^{5a}$ and $R^{6a}$ are each hydrogen, $X^a$ is

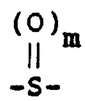

(where m is 0) and $R^{7a}$ is hydrogen or $C_1$-$C_4$ alkyl, $R^{1a}$ and $R^{2a}$ cannot both be a t-butyl group are also useful for preventing ischemia-induced brain damage such as may be caused by strokes, for example. Accordingly, yet another aspect of the present invention provides a method for preventing ischemia-induced cell damage in mammals by administering to a mammal in need thereof an effective ischemia reducing amount of a compound of formula II or a pharmaceutically acceptable salt thereof.

Further, it has been found that the lifespan of dystrophic mice has been prolonged by the administration of certain aryi-substituted rhodanines. Accordingly, another aspect of the present invention provides a method for the treatment of a dystrophic mammal by the administration of an effective amount of a compound of the formula (III)

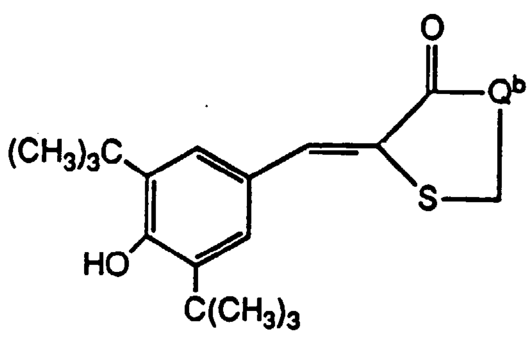

(III)

wherein:

$Q^b$ is -O- or $NR^{7b}$, where $R^{7b}$ is hydrogen, $C_1$-$C_6$ alkyl, $NR^{8b}R^{9b}$ or $-(CH_2)_n$-OH, where $R^{8b}$ and $R^{9b}$ are each independently hydrogen or $C_1$-$C_4$ alkyl and n is as defined for formula I, or a pharmaceutically acceptable salt thereof.

As used herein, the term "$C_1$-$C_6$ alkyl" refers to straight and branched chain aliphatic radicals of 1 to 6 carbon atoms, both inclusive, such as methyl, ethyl, propyl, isopropyl, butyl, isobutyl, sec-butyl, tert-butyl, n-pentane, isopentane, n-hexane, isohexane and the like. The term "$C_1$-$C_6$ alkyl" includes within its definition the term "$C_1$-$C_4$ alkyl".

The term "$C_1$-$C_6$ alkoxy" refers to the alkyl radicals of 1 to 6 carbon atoms, both inclusive, attached to the remainder of the molecule by oxygen and includes methoxy, ethoxy, propoxy, isopropoxy, butoxy, isobutoxy, sec-butoxy, tert-butoxy, pentoxy, hexoxy and the like. The term "$C_1$-$C_6$ alkoxy" includes within its definition the term "$C_1$-$C_4$ alkoxy".

The term "$C_2$-$C_6$ alkenyl" refers to straight and branched chain radicals of 2 to 6 carbon atoms, both inclusive, having a double bond. As such, the term includes ethylene, propylene, isopropylene, 1-butene, 2-butene, 2-methyl-1-propene, 1-pentene, 2-pentene, 2-methyl-2-butene and the like.

The term "$C_2$-$C_6$ alkynyl" refers to straight and branched chain radicals of 2 to 6 carbon atoms, both inclusive, having a triple bond. As such, the term includes acetylene, propyne, 1-butyne, 2-butyne, 1-pentyne, 2-pentyne, 3-methyl-1-butyne, 1-hexyne, 2-hexyne, 3-hexyne and the like.

The term "$C_3$-$C_8$ cycloalkyl" refers to saturated alicyclic rings of 3 to 8 carbon atoms, both inclusive, such as cyclopropyl, methylcyclopropyl, cyclobutyl, cyclopentyl, cyclohexyl and cyclooctyl.

Compounds of formula I, and their pharmaceutically acceptable salts, wherein $R^1$ and $R^2$ are each $C_1$-$C_6$ alkyl; $R^3$ is hydrogen; $R^4$ and $R^5$ taken together form a bond; $R^6$ and $R^7$ are each hydrogen; X is

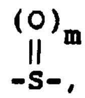

where m is O; and Q is -O- or $NR^8$, where $R^8$ is as defined for formula I are preferred. Of this preferred group of compounds, those compounds, and their pharmaceutically acceptable salts, wherein $R^1$ and $R^2$ are each $C_1$-$C_4$ alkyl; $R^3$ is hydrogen; $R^4$ and $R^5$ taken together form a bond; $R^6$ and $R^7$ are each hydrogen; X is

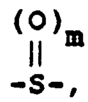

where m is O; and Q is $NR^8$, where $R^8$ is hydrogen, $C_1$-$C_6$ alkyl or $-(CH_2)_n$-Y, where n and Y are as defined for formula I are particularly preferred.

Compounds of formula I, and their pharmaceutically acceptable salts, wherein $R^1$ and $R^2$ are each $C_1$-$C_6$ alkyl; $R^3$ is hydrogen; $R^4$ and $R^5$ taken together form a bond; $R^6$ and $R^7$ are each hydrogen; X is

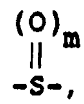

where m is O; and Q is -O- or $NR^8$, where $R^8$ is as defined for formula I are preferred for use in the treatment of inflammation and arthritis as well as for the pharmaceutical compositions of the present invention. Of these preferred compounds, those compounds, and their pharmaceutically acceptable salts, wherein $R^1$ and $R^2$ are each $C_1$-$C_4$ alkyl; $R^3$ is hydrogen; $R^4$ and $R^5$ taken together form a bond; $R^6$ and $R^7$ are each hydrogen; X is

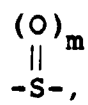

where m is O; and Q is $NR^8$, where $R^8$ is hydrogen, $C_1$-$C_6$ alkyl or $-(CH_2)_n$-Y, where n and Y are as defined for formula I are particularly preferred for use in the treatment of inflammation and arthritis as well as for the pharmaceutical compositions of the present invention.

Compounds, and the pharmaceutically acceptable salts thereof, of formula II wherein $R^{1a}$ and $R^{2a}$ are each independently $C_1$-$C_6$ alkyl; $R^{3a}$ and $R^{4a}$ taken together form a bond; $R^{5a}$ and $R^{6a}$ are each hydrogen; $X^a$ is

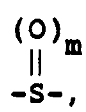

where m is O; and $R^{7a}$ is as defined for formula II are preferred for use in the prevention of ischemia-induced cell damage in mammals. Of this preferred group of compounds, those compounds, and their pharmaceutically acceptable salts, wherein $R^{1a}$ and $R^{2a}$ are each $C_1$-$C_4$-alkyl; $R^{3a}$ and $R^{4a}$ taken together form a bond; $R^{5a}$ and $R^{6a}$ are each hydrogen; $X^a$ is

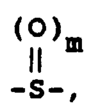

where m is O; and $R^{7a}$ is as defined for formula II are particularly preferred for use in the prevention of ischemia-induced cell damage in mammals.

5-{[3,5-Bis(1,1-dimethylethyl)-4-hydroxyphenyl]methylene}-2-thioxo-4-thiazolidinone (referred to in the following discussion as Compound A); is taught in the art by Teuber et al., Leibigs Ann. Chem., 757 (1978) (as compound V). The compound is prepared by the reaction of 3,5-di-tert-butyl-4-hydroxybenzaldehyde with rhodanine at reflux temperature in glacial acetic acid with fused sodium acetate as a catalyst. 5-{[3,5-Bis(1,1-dimethylethyl)-4-hydroxyphenyl] methylene}-4-thiazolidinone (Compound B); 5-{[3,5-bis(1,1-dimethylethyl)-4-hydroxyphenyl]methyl}-4-thiazolidinone (Compound C); and 5-{[3,5-bis(1,1-dimethylethyl)-4-hydroxyphenyl]methyl}-2-thioxo-4-thiazolidinone (Compound D) can be prepared from Compound A. For example, when Compound A is subjected to catalytic hydrogenation, one obtains both Compounds B and C. The relative proportions of each depends upon the temperature, pressure, and duration of hydrogenation, the solvent employed, and the particular catalyst used. For example, when Compound A is treated with 5% palladium on carbon in ethanol at 100°C for approximately 18 hours, the relative ratios of Compound B:C are approximately 60:40. Alternatively, these transformations may be accomplished by heating Compound A in a mixture of hydrochloric acid and an alcohol such as ethanol in the presence of zinc. Reduction of the thione without affecting the benzylic double bond may be accomplished by heating the thione with a reducing agent such as tri-n-butyl tin hydride in a non-reactive solvent, such as toluene, and preferably in the presence of a free radical initiator, such as azobisisobutyronitrile. However, for such reduction to work an N-substituted rhodanine substrate (i.e., Q cannot be NH) must be employed.

The transformation of Compound A to D may be accomplished by a variety of methods known in the art. A preferred method is that taught by Nakamura et al., Tetrahedron Letters, 25, 3983 (1984). In this reaction, Compound A is treated with a dihydropyridine such as diethyl 2,6-dimethyl-1,4-dihydro-3,5-pyridinedicarboxylate in the presence of silica gel. The reaction is best carried out in the presence of a nonreactive solvent such as benzene or toluene, preferably under an inert atmosphere. The reaction may be accomplished at temperatures from about 25°C up to the reflux temperature of the mixture. At the preferred temperature of approximately 80°C, the reaction is essentially complete after 12-18 hours.

Other thiazolidinones (Q is $NR^8$, $Q^a$ is $NR^{7a}$ or $Q^b$ is $NR^{7b}$) may, depending on the values selected for the various substituents, be prepared in an analogous fashion. For example, compounds of formula I wherein Q is $NR^8$ and $R^8$ is hydrogen, $C_1$-$C_6$ alkyl, $C_3$-$C_8$ cycloalkyl or $-(CH_2)_n$-Y where n is an defined for formula I and Y is cyano or $NR^{11}R^{12}$ where $R^{11}$ and $R^{12}$ are each independently hydrogen or $C_1$-$C_6$ alkyl, may be prepared by the method of Teuber et al. described above, employing the appropriate N-substituted rhodanine and $R^1$, $R^2$-substituted-4-hydroxybenzaldehyde. Alternatively, rhodanine may be used for the condensation with the aldehyde forming those species wherein Q is $NR^8$ and $R^8$ is hydrogen followed by alkylation with the appropriate $R^8$-containing halide, such as an iodide or bromide, to provide N-substituted derivatives; i.e., those compounds of formula I in which $R^8$ is $C_1$-$C_6$ alkyl, $C_2$-$C_6$ alkenyl, $C_3$-$C_8$ cycloalkyl or $-(CH_2)_n$-Y, where Y is cyano, $OR^9$, -SH, -S($C_1$-$C_4$ alkyl), $-NR^{11}R^{12}$ or

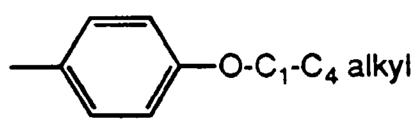

and n, $R^9$, $R^{11}$ and $R^{12}$ are as defined for formula I. The alkylation is usually accomplished in an inert solvent such as tetrahydrofuran (THF) or dimethylformamide (DMF) and in the presence of a strong base such as sodium hydride. In a similar fashion, rhodanine may be used for the condensation with the aldehyde forming those species wherein Q is $NR^8$ and $R^8$ is hydrogen followed by acylation with the appropriate $R^8$-containing halide to provide N-substituted derivatives of formula I in which $R^8$ is $-(CH_2)_n$-Y and Y is

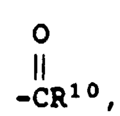

where n and $R^{10}$ are as defined for formula I.

Compounds of formula I wherein Q is $NR^8$ and $R^8$ is $-(CH_2)_n$-Y (Y is $OR^9$ or $NR^{11}R^{12}$, wherein $R^9$ is hydrogen

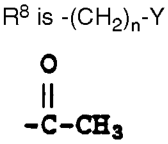

or tosyl and $R^{11}$ and $R^{12}$ are as defined for formula I) may also be prepared according to the following reaction scheme:

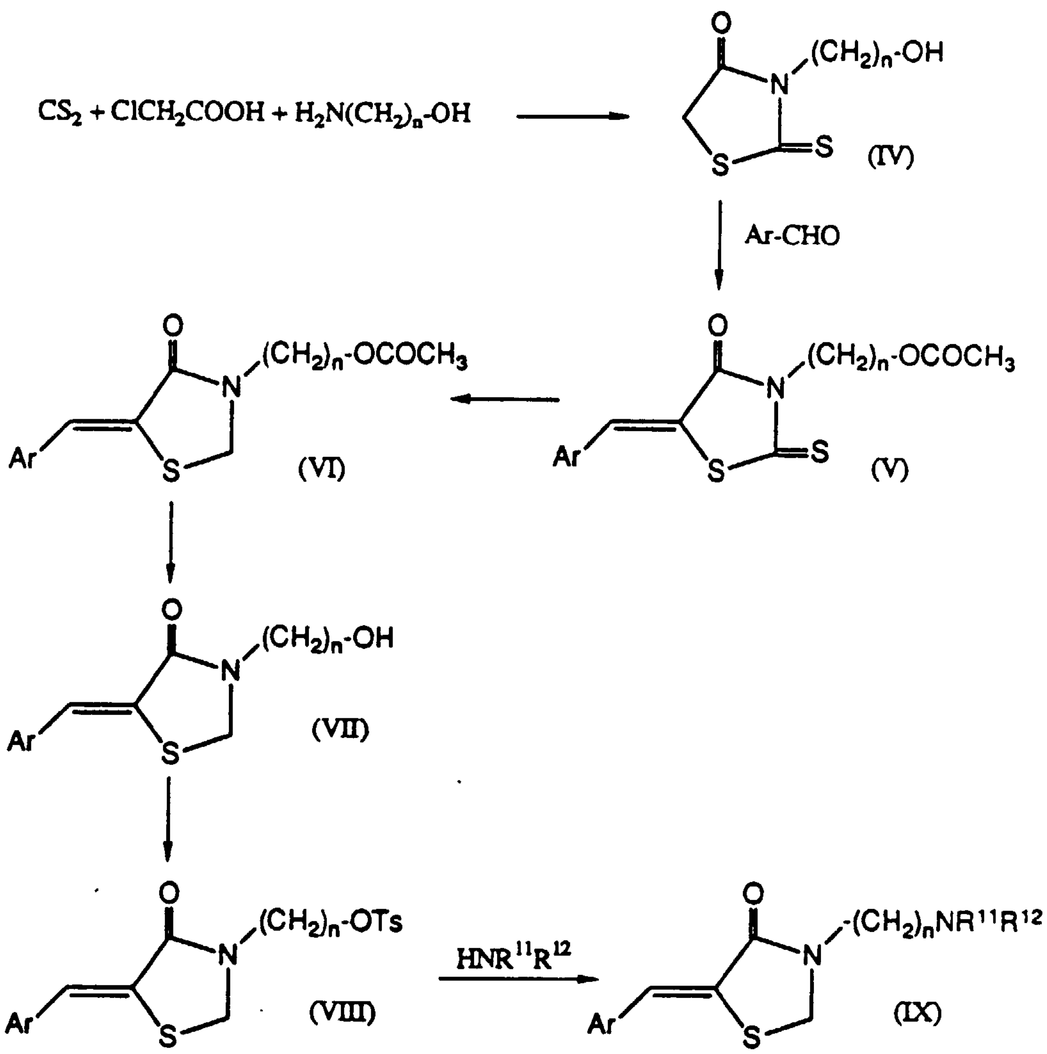


where Ts = Tosyl; Ar =

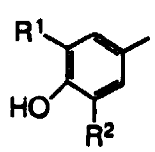

A hydroxyalkyl rhodanine IV is prepared by condensing carbon disulfide, chloroacetic acid, and the appropriate hydroxyalkylamine by standard techniques. When condensed with the appropriate $R^1$,$R^2$-substituted-4-hydroxybenzaldehyde as described above, the resulting product is the condensed 2-thioxo-4-thiazolidinone V which has been transformed into the acetyl derivative. The thioxo compound V may optionally be converted to the methylene compound of formula VI as described above. The acetyl group of intermediate VI may be removed upon treatment with aqueous ammonia in a solvent such as acetonitrile to provide compound VII (i.e., that compound of formula I wherein Q is $NR^8$ and $R^8$ is $-(CH_2)_n$-Y where Y is $OR^9$ and $R^9$ is hydrogen). The hydroxy compound VII is then converted to the tosyl derivative upon treatment with p-toluenesulfonyl chloride in pyridine, preferably at a temperature of around 0°C. The versatile tosyl intermediate VIII may then be transformed into additional compounds of formula I upon treatment with an appropriate $HNR^{11}R^{12}$ amine, where $R^{11}$ and $R^{12}$ are as stated in the preceeding paragraph. This latter transformation is best accomplished by allowing VIII to react in the presence of a molar excess of.the amine. Once again, a solvent such as acetonitrile is useful for accomplishing this transformation.

The corresponding 1,3-oxothiolan-5-ones (Q and $Q^b$ are -O-) may be prepared from β-(3,5-di-t-butyl-4-hydroxyphenyl)-α-mercaptoacrylic acid (X). Compound X may be treated with carbon disulfide to prepare the thione analog

(formula I, Q = -O-, $R^6$ and $R^7$ are =S), while reaction of X with formic acid provides the corresponding desthione (formula I, Q = -O-, $R^6$ and $R^7$ are each hydrogen). Compound X can be prepared by known methods (see, e.g., Campaigne et al., J. Org. Chem., 26, 359 (1961); id., 26, 1326 (1961); Chakrabarti, et al., Tetrahedron, 25 (14), 2781 (1969)), or upon heating Compound A with dilute aqueous base (See Example 17A).

Compounds of formula I wherein Q is $NR^8$ and $R^8$ is $-(CH_2)_n$-Y (n=0) and Y is $NR^{11}R^{12}$ and $R^{11}$ and $R^{12}$ are as defined for formula I may be prepared according to the following reaction sequence:

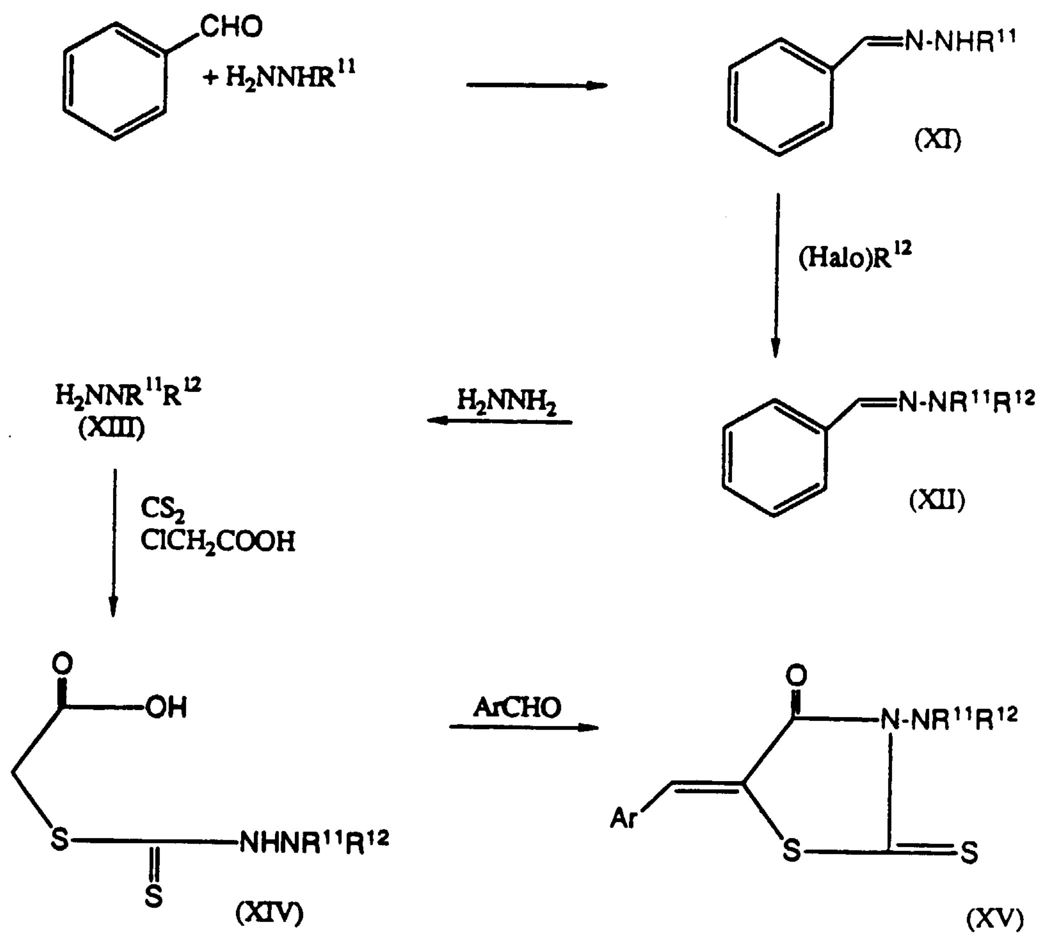


Ar = 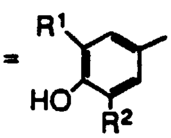

The $R^{11}$-substituted hydrazine is treated with benzaldehyde in an alcoholic (preferably methanol) solvent to yield intermediate XI, which, in turn, is reacted with the appropriate $R^{12}$-halide in the presence of triethylamine and acetonitrile to render intermediate XII. XII is then treated with hydrazine to render the $R^{11}$,$R^{12}$-hydrazine, XIII. XIII may alternatively be prepared by reducing a nitroso-$R^{11}R^{12}$ amine using zinc dust and acetic acid or aluminum and a strong base. The nitroso-$R^{11}R^{12}$ amine itself is prepared from an $R^{11}$,$R^{12}$ amine as described in J. Am. Chem. Soc. 77, 790 (1955) by treatment with sodium nitrite in HCl. The $R^{11}$,$R^{12}$-hydrazine (XIII) is then treated with carbon disulfide, chloroacetic acid and triethylamine to yield intermediate XIV. Condensation of said intermediate XIV with the appropriate $R^1$,$R^2$-substituted-4-hydroxybenzaldehyde (i.e., ArCHO) renders XV. As described previously, the thione may be reduced by treatment with a reducing agent such as tri-n-butyl-tin hydride in a non-reactive solvent such as toluene, preferably in the presence of a free radical initiator such as azobisisobutyronitrile. Preparation of the species wherein one of $R^{11}$ or $R^{12}$ is hydrogen may be effected before or after reduction of the thione, as desired, by heating the disubstituted compound in a mixture of ethanol/water in the presence of a catalyst such as a rhodium catalyst.

Those compounds of formulae I or II where X is

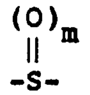

and m is 1 or 2 are readily prepared from the sulfide (i.e., m=0) as by treatment with an oxidizing agent, such as m-chloroperbenzoic acid, in an appropriate organic solvent, such as chloroform, for a time sufficient to effect the desired oxidation.

Compounds of formula I wherein $R^3$ is $C_1$-$C_6$ alkyl are prepared by conventional Friedel-Crafts alkylation of the appropriate $R^1$, $R^2$-substituted phenol, followed by condensation with rhodanine, or the desired N-substituted rhodanine, as described herein or is used as described in other reaction schemes depicted herein.

It will be readily appreciated by one skilled in the art that the aryl portion of the present compounds of formulae I, II and III are either commercially available or may be readily prepared by known techniques from commercially available starting materials. For example, p-hydroxybenzaldehyde may be alkylated under Friedel-Crafts conditions to yield an alkylbenzaldehyde which in turn may itself be alkylated. Similarly, the rhodanine or N-substituted rhodanine starting material is either commercially available or prepared by well known methodology from commercially available starting materials.

Further, the skilled artisan will readily appreciate that the pyrrolidones described for formula II (i.e., $X^a$ = -$CH_2$-) which are useful in a treatment method for preventing ischemia-induced cell damage, are prepared in analogous fashion to that described herein for the thiazolidinones. That is to say, the pyrrolidones are conveniently prepared by the condensation of an appropriately substituted 2-pyrrolidone with the desired $R^1$,$R^2$-substituted aryl moiety as described, for example, by Katsumi et al., Chem. Pharm. Bull. 34 (4), 1619 (1986).

Those compounds of formula I wherein one of $R^6$ or $R^7$ is hydrogen and the other is -OH are conveniently prepared from their respective precursors (i.e., those compounds of formula I where $R^6$ and $R^7$ are both hydrogen and $R^1$, $R^2$, $R^3$, $R^4$ $R^5$, X and Q are as defined in formula I) by the treatment of the precursor with, for example, trifluoroacetic anhydride in an inert solvent (preferably methylene chloride) at reduced temperatures. Similarly, compounds of formulae I and II where, in the definition of Q or $Q^a$, Y or $Y^a$ is cyano are prepared by treating the non-cyanated compounds of those formulae with the desired halo-substituted aliphatic nitrile. From the cyano derivative the tetrazolyl is prepared by treatment with tri-N-butyl tin azide in, for example, ethylene glycol dimethyl ether.

Other compounds of formula I may be prepared as more fully described below from compounds whose synthesis was described generically, supra. The compounds useful in the present methods for preventing ischemia-induced brain damage (compounds of formula II) and treatment of a dystrophic mammal (compounds of formula III) may be prepared in a manner analogous to that described above with respect to the compounds of formula I.

According to a final aspect of the invention there is provided a process for preparing a novel compound of formula I which comprises

(A) reacting a compound of the formula

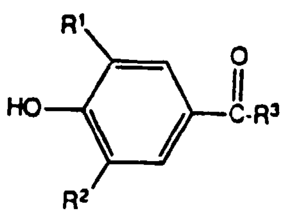

with a compound of the formula

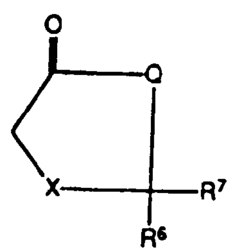

wherein $R^1$, $R^2$, $R^3$ and X are as in formula I, Q is -$CH_2$- or $NR^8$ (where $R^8$ is as defined in formula I) and $R^6$ and $R^7$ taken together are =S, so as to provide a compound of the formula

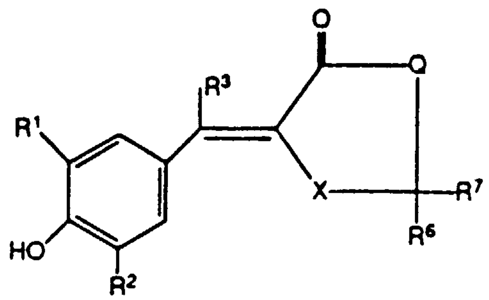

wherein $R^1$, $R^2$, $R^3$, $R^6$, $R^7$, X and Q are as set forth above;

(B) reducing a compound of formula I wherein $R^6$ and $R^7$ taken together are =S so as to prepare a compound of formula I in which $R^6$ and $R^7$ are hydrogen;

(C) reducing a compound of formula I in which $R^4$ and $R^5$ taken together form a bond so as to prepare a compound of formula I in which $R^4$ and $R^5$ are hydrogen;

(D) reducing a compound of formula I in which $R^4$ and $R^5$ taken together form a bond and $R^6$ and $R^7$ taken together are =S so as to prepare a compound of formula I in which $R^4$, $R^5$, $R^6$ and $R^7$ are all hydrogen;

(E) alkylating a compound of formula I in which $R^8$ is hydrogen so as to prepare a compound of formula I in which $R^8$ is $C_1$-$C_6$ alkyl, $C_2$-$C_6$ alkenyl, $C_3$-$C_8$ cycloalkyl or $-(CH_2)_n-Y$ (where n is an integer from 0 to 3, both inclusive, and Y is cyano, $OR^9$, -SH, -S($C_1$-$C_4$ alkyl), $-NR^{11}R^{12}$ or

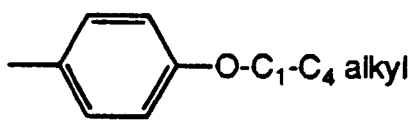

where $R^9$, $R^{11}$ and $R^{12}$ are as defined in formula I);

(F) acylating a compound of formula I in which $R^8$ is hydrogen so as to prepare a compound of formula I in which $R^8$ is $-(CH_2)_n-Y$, where n is an integer from 0 to 3, both inclusive, and Y is

$$-\overset{O}{\overset{\|}{C}}R^{10},$$

where $R^{10}$ is as defined in formula I;

(G) oxidizing a compound of formula I wherein X is

$$-\overset{(O)_m}{\overset{\|}{S}}-,$$

where m is 0, so as to prepare a compound of formula I wherein X is

$$-\overset{(O)_m}{\overset{\|}{S}}-$$

and m is 1;

(H) oxidizing a compound of formula I wherein X is

$$-\overset{(O)_m}{\overset{\|}{S}}-,$$

where m is 0, so as to prepare a compound of formula I wherein X is

$$-\overset{(O)_m}{\overset{\|}{S}}-$$

and m is 2;

(I) oxidizing a compound of formula I wherein X is

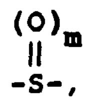

where m is 1, so as to prepare a compound of formula I wherein X is

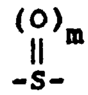

and m is 2;

(J) reacting a compound of the formula

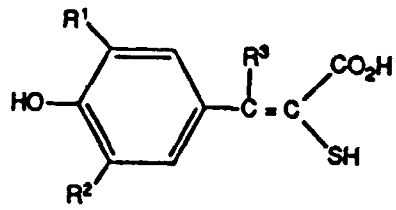

with

i) formic acid, so as to provide a compound of formula I wherein Q is O, $R^4$ and $R^5$ taken together form a bond and $R^6$ and $R^7$ are hydrogen; or
ii) carbon disulfide, so as to provide a compound of formula I wherein Q is O, $R^4$ and $R^5$ taken together form a bond and $R^6$ and $R^7$ taken together are =S;

(K) reacting a compound of the formula

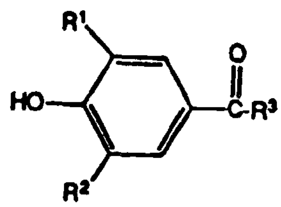

with a compound of the formula

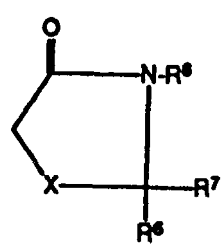

wherein $R^1$, $R^2$, $R^3$ and X are as defined in formula I, $R^6$ and $R^7$ taken together are =S, and $R^8$ is $-(CH_2)_n-Y$ (where n is an integer from 0 to 3, both inclusive, and Y is $OR^9$, where $R^9$ is hydrogen) so as to provide a compound of the formula

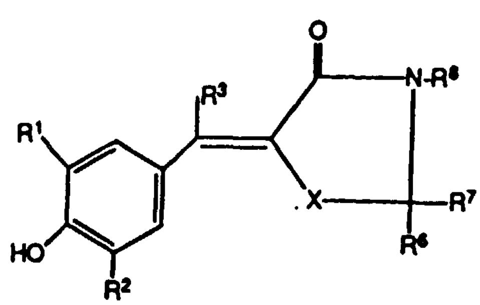

wherein $R^1$, $R^2$, $R^3$, $R^6$, $R^7$ and X are as set forth above and $R^8$ is $-(CH_2)_n-Y$ (where n is an integer from 0 to 3,

both inclusive, and Y is $OR^9$, where $R^9$ is

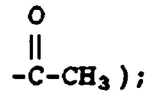

(L) reducing a compound of formula I in which $R^8$ is $-(CH_2)_n-Y$, wherein n is 0 to 3, both inclusive, and Y is $-OR^9$, where $R^9$ is

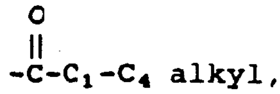

so as to prepare a compound of formula I in which $R^8$ is $-(CH_2)_n-Y$, wherein n is 0 to 3, both inclusive, and Y is $OR^9$, where $R^9$ is hydrogen;

(M) reacting a compound of formula I in which $R^8$ is $-(CH_2)_n-Y$ wherein n is 0 to 3, both inclusive, and Y is $-OR^9$, where $R^9$ is hydrogen, with a tosyl-halide so as to prepare a compound of formula I in which $R^8$ is $-(CH_2)_n-Y$ wherein n is 0 to 3, both inclusive, and Y is $OR^9$, where $R^9$ is tosyl;

(N) reacting a compound of formula I in which $R^8$ is $-(CH_2)_n-Y$, wherein n is 0 to 3, both inclusive, and Y is $-OR^9$, where $R^9$ is tosyl, with an amine of the formula $HNR^{11}R^{12}$ (where $R^{11}$ and $R^{12}$ are as defined for formula I) so as to prepare a compound of formula I in which $R^8$ is $-(CH_2)_n-Y$, wherein n is 0 to 3, both inclusive, and Y is $-NR^{11}R^{12}$;

(O) treating a compound of formula I in which $R^8$ is $-(CH_2)_n-Y$, wherein n is 0 to 3, both inclusive, and Y is cyano with tri-n-butyl tin azide so as to prepare a compound of formula I in which $R^8$ is $-(CH_2)_n-Y$ wherein n is 0 to 3, both inclusive, and Y is tetrazolyl;

(P) reacting a compound of the formula

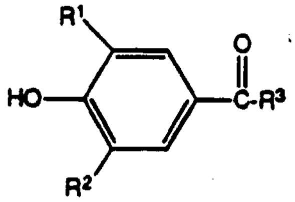

with a compound of the formula

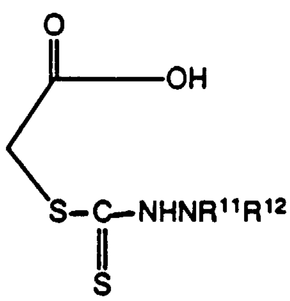

,

wherein $R^1$, $R^2$, $R^3$, $R^{11}$ and $R^{12}$ are as in formula I, so as to provide a compound of the formula

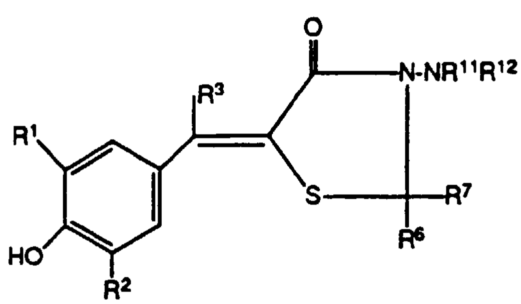

wherein $R^6$ and $R^7$ taken together are =S and $R^1$, $R^2$, $R^3$, $R^{11}$ and $R^{12}$ are as defined in formula I;

(Q) heating a compound of formula I in which $R^8$ is $-(CH_2)_n-Y$ and Y is $-NR^{11}R^{12}$ (neither of $R^{11}$ or $R^{12}$ being hydrogen) in an ethanol/water mixture in the presence of a catalyst so as to prepare a compound of formula I in

which $R^8$ is $-(CH_2)_n$-Y and Y is $-NR^{11}R^{12}$ (where one of $R^{11}$ or $R^{12}$ is hydrogen and the other is not hydrogen);

(R) reacting a compound of formula I in which $R^6$ and $R^7$ are both hydrogen with trifluoroacetic anhydride so as to prepare a compound of formula I in which one of $R^6$ and $R^7$ is hydrogen and the other is -OH;

(S) salifying a compound of formula I by reacting the non-salt form of the compound with either a strong acid or a strong base.

Depending upon the definitions of $R^3$, $R^4$ and $R^5$, the compounds of formula I may exist in various isomeric forms. This invention is not related to any particular isomer but includes all possible individual isomers and racemates. Pharmaceutically acceptable salts can be prepared by reacting a compound of formula I with a strong base, such a sodium hydroxide, or a strong acid, such as hydrochloric acid, depending on the types of substituents present on the compound of formula I.

The following examples further illustrate the preparation of the compounds of this invention as well as the compounds used in the methods of this invention. The examples are illustrative only and are not intended to limit the scope of the invention in any way.

Example 1

5-{[3,5-bis(1,1-dimethylethyl)-4-hydroxyphenyl]-methylene}-2-thioxo-4-thiazolidinone (Compound A)

Under a nitrogen atmosphere, 117.2 g of 3,5-di-tert-butyl-4-hydroxybenzaldehyde, 66.6 g of rhodanine, and 143.5 g of fused sodium acetate were heated at reflux in 2500 ml of glacial acetic acid. After heating for 23 hours, the reaction mixture was cooled and poured into a mixture of 1 liter of ethanol and 1 liter of ice, with stirring. 500 ml of water were added and, after stirring for 30 minutes, the resulting precipitate was recovered by filtration. The solid was slurried with 500 ml of ethyl acetate and filtered. The precipitate was then dissolved in 3 liters of ethanol, heated to boiling, and water was added until the solution remained cloudy (approximately 450 ml of water). Upon cooling to room temperature, 99.6 g of the desired title product were recovered by filtration, m.p. approximately 260°C.

| Analysis for $C_{18}H_{23}NO_2S_2$ : | | | |
|---|---|---|---|
| Calculated: | C, 61.86; | H, 6.63; | N, 4.01; |
| Found: | C, 62.13; | H, 6.55; | N, 4.15. |

Examples 2-3

5-{[3,5-bis(1,1-dimethylethyl)-4-hydroxyphenyl]methylene)-4-thiazolidinone (Compound B) and 5-{[3,5-bis (1,1-dimethylethyl)-4-hydroxyphenyl]methyl}-4-thiazolidinone (Compound C)

A solution of 69.90 g of 5-{[3,5-bis(1,1-dimethylethyl}-4-hydroxyphenyl]methylene}-2-thioxo-4-thiazolidinone in 4 liters of ethanol was hydrogenated at 500 pounds per square inch (psi) in the presence of 200 g of 5% palladium on carbon overnight at 100°C. The reaction mixture was filtered and evaporated to dryness. In sections, the material was dissolved in 1 volume of hot ethyl acetate, diluted with 2 volumes of hexane, filtered, and loaded onto a silica gel chromatography column. Elution with 35% ethyl acetate in hexane provided various fractions which were combined according to the purities of the respective compounds. A total of 4.6 g of Compound B were isolated by chromatography. Fractions which were predominantly Compound B were crystallized from ethyl acetate/hexane providing a total yield of Compound B of 13.79 g. Rechromatography of fractions containing impure Compound C on silica eluting with 25% ethyl acetate in hexane provided 9.82 g of Compound C.

2. 5-{[3,5-bis(1,1-dimethylethyl)-4-hydroxyphenyl]methylene}-4-thiazolidinone, m.p. 209-213°C.

| Analysis for $C_{18}H_{25}NO_2S$: | | | |
|---|---|---|---|
| Calculated: | C, 67.67; | H, 7.89; | N, 4.38; |
| Found: | C, 67.44; | H, 8.11; | N, 4.65. |

3. 5-{[3,5-bis(1,1-dimethylethyl)-4-hydroxyphenyl]methyl}-4-thiazolidinone, m.p. 149-152°C.

| Analysis for $C_{18}H_{27}NO_2S$: | | | |
|---|---|---|---|
| Calculated: | C, 67.25; | H, 8.47; | N, 4.36; |

(continued)

| Analysis for $C_{18}H_{27}NO_2S$: | | | |
|---|---|---|---|
| Found: | C, 67.43; | H, 8.44; | N, 4.21. |

Example 4

5-{[3,5-bis(1,1-dimethylethyl)-4-hydroxyphenyl]methylene}-4-oxo-3-thiazolidinecarboxylic acid, methyl ester

Under a nitrogen atmosphere, 7.03 g of the compound of Example 2 were dissolved in 330 ml of THF to which was added 581 mg of sodium hydride. The mixture was stirred for 10 minutes after which about 2 g of methyl chloroformate were added and the resulting mixture stirred for an additional 50 minutes. Water (500 ml) and 7 ml of 1N hydrochloric acid (pH of solution about 3) were added. The resultant mixture was extracted twice with 200 ml portions of ethyl acetate. The organic extracts were combined, stripped to dryness, and crystallized from 15 ml of ethylacetate and 25 ml hexane to render the title compound, m.p. 165-167.5°C.

| Analysis for $C_{20}H_{27}NO_4S$: | | | |
|---|---|---|---|
| Calculated: | C, 63.63; | H, 7.21; | N, 3.71; |
| Found: | C, 63.76; | H, 7.33; | N, 3.68. |

Example 5

5-{[3,5-bis(1,1-dimethylethyl)-4-hydroxyphenyl]methylene}-4-oxo-3-thiazolidineacetamide

Under a nitrogen atmosphere, 7.03 g of the compound of Example 2 were dissolved in 330 ml of THF. To this was added 0.581 g of sodium hydride and the mixture was stirred for 10 minutes. Iodoacetamide (4.07 g) was added and the resultant mixture was heated at reflux temperature for one hour and then cooled. The solution was then poured into 500 ml of a mixture of rapidly stirred ice/water. The pH of the mixture was reduced to about pH 3 by the addition of 10 ml of 1N hydrochloric acid. The resultant mixture was extracted with three 200 ml portions of ethyl acetate. The extracts were combined, stripped, and crystallized from a mixture of 120 ml of ethyl acetate and 100 ml hexane to render 2.79 g of the title compound, m.p. 232-235.

| Analysis for $C_{20}H_{28}N_2O_3S$: | | | |
|---|---|---|---|
| Calculated: | C, 63.80; | H, 7.50; | N, 7.44; |
| Found: | C, 63.53; | H, 7.67; | N, 7.14. |

Example 6

5-{[3,5-bis(1,1-dimethylethyl)-4-hydroxyphenyl]methylene}-3-[2-(methylthio)ethyl]-4-thiazolidinone

5-{[3,5-Bis(1,1-dimethylethyl)-4-hydroxyphenyl]-methylene}-4-thiazolidinone (i.e., the compound of Example 2; 26.7 g) was dissolved in 418 ml of DMF to which was added 3.34 g of a 60% sodium hydride dispersion. The resultant mixture was stirred at 100°C under an argon atmosphere. To this were added 8.33 ml of methylthioethyl chloride and the resulting black solution was stirred at 100°C for 6 days. The material was allowed to cool to 30°C after which insoluble material was filtered off. The solid was washed with DMF until its color was gone leaving a white solid which was discarded. The pH of the filtrate and washings was adjusted to 1.5 by adding 1N hydrochloric acid with stirring. The mixture was diluted with 1000 ml of diethyl ether and 500 ml of IN hydrochloric acid and the resulting mixture was then shaken and separated. The organic layer was washed with two portions of water and one portion of brine and subsequently dried over sodium sulfate, filtered, evaporated and chased with chloroform to give a black foam/oil. This material was triturated with about 75 ml of chloroform and then filtered. The insoluble solid was washed with additional chloroform until its brown color was gone. The filtrate was then loaded onto a silica gel column which was eluted with 8000 ml of a gradient of 10-30% ethyl acetate in hexane. The various fractions containing the desired product were combined and again loaded onto a silica gel column and eluted with 8000 ml of a gradient of 10-35% acetone in hexane. The fractions containing the desired product were recrystalled from hexane/ethyl acetate to give 1.2 g of the title compound as a tan/orange solid, m.p. 165.5-168°C.

| Analysis for $C_{21}H_{31}NO_2S_2$ : | | | |
|---|---|---|---|
| Calculated: | C, 64.08; | H, 7.94; | N, 3.56; |
| Found: | C, 63.99; | H, 8.13; | N, 3.45. |

Example 7

5-{[3,5-bis(1,1-dimethylethyl)-4-hydroxyphenyl]methylene}-3-(2-methoxyethyl)-4-thiazolidinone

Under an argon atmosphere, 9.58 g of the compound of Example 2 were dissolved in THF with stirring. To this were added 1.2 g of a 60% sodium hydride dispersion and the reaction mixture was then heated to reflux. 2.82 ml of methoxyethylbromide were then added and the resultant mixture was allowed to stir at reflux for five days. After five days, 0.2 equivalents of potassium iodide were added and the reaction was allowed to continue at reflux temperature for an additional two days. The mixture was then allowed to cool and was diluted with diethyl ether and water. The pH of the mixture was adjusted to pH 2 by adding of 1N hydrochloric acid with stirring. Organic and aqueous layers formed and were separated. The organic layer was washed with saturated sodium bicarbonate, then brine, and subsequently dried over sodium sulfate, filtered, evaporated and then chased with chloroform. The resultant material was dissolved in 50 ml of chloroform and a precipitate formed. An additional 25 ml of chloroform were added and the mixture heated. The resultant solution was filtered, chromatographed on silica gel, and subsequently eluted with 8000 ml of a 10-30% gradient of ethyl acetate in hexane followed by elution with 4000 ml of a 30-40% gradient of ethyl acetate in hexane. The various fractions containing the desired product were combined, evaporated to dryness and then chased with chloroform to render an orange sticky solid. This material was dissolved in 15 ml of ethyl acetate while heating on a steam bath and subsequently diluted with 250 ml of hexane. The mixture was allowed to cool to room temperature, with precipitate forming, and allowed to stand for three days. The material was filtered and washed with hexane to yield 5.16 g of the title compound, m.p. 147-149°C.

| Analysis for $C_{21}H_{31}NO_3S$: | | | |
|---|---|---|---|
| Calculated: | C, 66.80; | H, 8.28; | N, 3.71; |
| Found: | C, 67.04; | H, 8.30; | N, 3.74. |

Example 8

5-{[3,5-bis(1,1-dimethylethyl)-4-hydroxyphenyl]methyl}-2-thioxo-4-thiazolidinone (Compound D)

Under a nitrogen atmosphere, 13.98 g of 5-{[3,5-bis(1,1-dimethylethyl)-4-hydroxyphenyl]methylene}-2-thioxo-4-thiazolidinone, 13.17 g of diethyl 2,6-dimethyl-1,4-dihydro-3,5-pyridinedicarboxylate and 600 ml of toluene were stirred to effect solution. Forty grams of silica gel 60 (finer than 230 mesh), previously dried in vacuo at 50°C for 7 hours, were added to the reaction. The reaction was heated at reflux for 18 hours and filtered hot. The filtrate was evaporated to dryness. The residue was dissolved in 500 ml of ethyl acetate, washed 5 times each with 400 ml of 1N hydrochloric acid, dried over sodium sulfate, filtered, and evaporated in vacuo to provide a yellow solid. Chromatography over silica gel eluting with 2.5% ethyl acetate in toluene provided 8.0 g of the desired title product, m.p. 178-179°C.

| Analysis for $C_{18}H_{25}NO_2S_2$ : | | | |
|---|---|---|---|
| Calculated: | C, 61.50; | H, 7.17; | N, 3.98; |
| Found: | C, 61.28; | H, 7.19; | N, 3.94. |

Example 9

5-{[3,5-bis(1,1-dimethylethyl)-4-hydroxyphenyl]methylene}-3-methyl-2-thioxo-4-thiazolidinone

The title compound was prepared in 76% yield from 3,5-di-tert-butyl-4-hydroxybenzaldehyde and N-methylrhodanine following the procedure of Example 1, m.p. >230°C.

| Analysis for $C_{19}H_{25}NO_2S_2$ : | | | | |
|---|---|---|---|---|
| Calculated: | C, 62.77; | H, 6.93; | N, 3.85; | S, 17.64; |
| Found: | C, 62.54; | H, 7.05; | N, 3.66; | S, 17.82. |

Example 10

5-{[3,5-bis(1,1-dimethylethyl)-4-hydroxyphenyl]methylene}-3-methyl-4-thiazolidinone

The title compound was prepared in 71% yield from 10.31 g of the thione of Example 9 upon heating with 38.15 ml of tri-n-butyl tin hydride and 1.16 g of azobisisobutyronitrile (AIBN) in 142 ml of toluene at reflux temperature for one hour. The product was isolated by adding water to the cooled reaction mixture, separating the layers, washing the organic layer with 1N hydrochloric acid and a saturated sodium chloride solution, drying over magnesium sulfate, concentrating in vacuo, and purifying the residue by chromatography over silica gel eluting with a 10-50% hexane in ethyl acetate gradient. The purified product had a melting point of 142-144°C.

| Analysis for $C_{19}H_{27}NO_2S$ : | | | |
|---|---|---|---|
| Calculated: | C, 68.43; | H, 8.16; | N, 4.20; |
| Found: | C, 68.68; | H, 8.00; | N, 3.97. |

Example 11

5-{[3,5-bis(1,1-dimethylethyl)-4-hydroxyphenyl]methyl}-3-methyl-4-thiazolidinone

To 100 ml of THF were added 6.43 g of the compound of Example 3. Sodium hydride (0.9 g) was added, resulting in the evolution of a gas. 1.25 ml (1.0 eq.) of iodomethane were added and the resultant mixture was stirred at room temperature for 23 hours after which the mixture was diluted with a volume of diethyl ether and 1N HCl. The organic layer was separated and dried over sodium sulfate, filtered and evaporated. The resultant solid was chased with chloroform to render an orange foam. A 5.93 g sample of this material was dissolved in 14 ml of a hot mixture of ethyl acetate, diluted with 225 ml of hexane and then allowed to cool to room temperature overnight. The solvent was evaporated and the resultant solid was dissolved in 40 ml of a hot mixture of diethyl ether diluted with about 400 ml of hexane. The mixture was allowed to cool to room temperature overnight and a precipitate formed which was collected by filtration, washed with hexane and dried in vacuo to render 3.98 g of the desired, title compound, m.p. 102°-105°C.

| Analysis for $C_{19}H_{29}NO_2S$ : | | | |
|---|---|---|---|
| Calculated: | C, 68.02; | H, 8.71; | N, 4.17; |
| Found: | C, 68.22; | H, 8.80; | N, 4.21. |

Example 12

5-{[3,5-bis(1,1-dimethylethyl)-4-hydroxyphenyl]methylene}-3-methyl-4-thiazolidinone, 1-oxide

Under a nitrogen atmosphere, 6.67 g of the compound of Example 10 were dissolved in 100 ml of chloroform, with stirring, and the resultant mixture was cooled to 4°C. Meta-chloroperbenzoic acid was added dropwise (with additional chloroform) after which the reaction mixture was poured into a separatory funnel and washed with a saturated sodium bicarbonate solution. The resultant layers were separated, and the organic layer was dried over sodium sulfate, filtered and then evaporated to give a white foam. This foam was dissolved in 70 ml of ethyl acetate while heating on a steam bath, then diluted with 125 ml of hexane while boiling. A precipitate formed and the reaction mixture was allowed to cool to room temperature overnight. The precipitate was then filtered, subsequently washed with hexane and dried under vacuum at room temperature for two hours to render 6.10 g of the title compound, m.p. 183-184°C.

| Analysis for $C_{19}H_{27}NO_3S$ : | | | |
|---|---|---|---|
| Calculated: | C, 65.30; | H, 7.79; | N, 4.01; |
| Found: | C, 65.46; | H, 7.68; | N, 4.01. |

Example 13

5-{[3,5-bis(1,1-dimethylethyl)-4-hydroxyphenyl]methylene}-3-methyl-4-thiazolidinone, 1,1-dioxide

Under a nitrogen atmosphere, 1 g of the compound of Example 10 was dissolved in 15 ml of chloroform, while cooled in an ice bath. To this solution was added, dropwise over a 15 minute period, 1.29 g of m-chloroperbenzoic acid and an additional 18 ml of chloroform. The mixture was removed from the ice bath, stirred at room temperature for 22 hours, transferred to a separatory funnel and then washed with a saturated sodium bicarbonate solution. The layers were separated and the organic layer was washed with brine, separated, dried over sodium sulfate, filtered and evaporated. The resultant residue was taken up in 12 ml of ethyl acetate and diluted with 50 ml hexane while boiling on a steam bath. The mixture was allowed to cool to room temperature overnight and the resultant precipitate was filtered, washed with hexane and dried in vacuo to yield 0.75 g of the desired titled compound, m.p. 217-221°C.

| Analysis for $C_{19}H_{27}NO_4S$ : | | | |
|---|---|---|---|
| Calculated: | C, 62.44; | H, 7.45; | N, 3.83; |
| Found: | C, 62.17; | H, 7.26; | N, 3.95. |

Example 14

5-{[3,5-Bis(1,1-dimethylethyl)-4-hydroxyphenyl]methylene}-3-ethyl-4-thiazolidinone

To a solution of 9.58 g of 5-{[3,5-bis(1,1-dimethylethyl)-4-hydroxyphenyl]methylene}-4-thiazolidinone in 150 ml of tetrahydrofuran were added 1.20 g of a 60% dispersion of sodium hydride in mineral oil. After gas evolution ceased, 2.4 ml of ethyl iodide were added and the reaction mixture was stirred for two days under an argon atmosphere. The mixture was heated at reflux for six hours, cooled, diluted with diethyl ether and water, and adjusted to pH 3 with 1N hydrochloric acid. The layers were separated, and the organic layer was washed with a saturated sodium bicarbonate solution followed by a saturated sodium chloride solution. Concentration of the dried organic solution and chromatography of the resulting residue over silica gel eluting with a 10-30% ethyl acetate in hexane gradient provided 3.65 g of the desired title product, m.p. 169-172.5°C.

| Analysis for $C_{20}H_{29}NO_2S$ : | | | |
|---|---|---|---|
| Calculated: | C, 69.12; | H, 8.41; | N, 4.03; |
| Found: | C, 69.39; | H, 8.52; | N, 4.30. |

Examples 15-16

The following compounds were prepared from the appropriate alkyl iodide according to the procedure of Example 14.

15. 5-{[3,5-bis(1,1-dimethylethyl)-4-hydroxyphenyl]methylene}-3-propyl-4-thiazolidinone, 60% yield, m.p. 145-146.5°C.

| Analysis for $C_{21}H_{31}NO_2S$ : | | | |
|---|---|---|---|
| Calculated: | C, 69.76; | H, 8.64; | N, 3.87; |
| Found: | C, 70.05; | H, 8.76; | N, 4.01. |

16. 5-{[3,5-bis(1,1-dimethylethyl)-4-hydroxyphenyl]methylene}-3-butyl-4-thiazolidinone, 60% yield, m.p. 168.5-169.5°C.

| Analysis for $C_{22}H_{33}NO_2S$ : | | | |
|---|---|---|---|
| Calculated: | C, 70.36; | H, 8.86; | N, 3.73; |
| Found: | C, 70.60; | H, 8.81; | N, 3.97. |

Example 17

4-{[3,5-bis(1,1-dimethylethyl)-4-hydroxyphenyl]methylene}-1,3-oxothiolan-5-one

A. Preparation of β-(3,5-di-t-butyl-4-hydroxyphenyl)-α-mercaptoacrylic acid.

A solution of 174.5 g of 5-{[3,5-bis(1,1-dimethylethyl)-4-hydroxyphenyl]methylene}-2-thioxo-4-thiazolidinone in 1250 ml of a 10% sodium hydroxide solution was heated on a steam bath for four hours. Decolorizing carbon was added and the mixture filtered through a high flow diatomaceous earth pad. The filtrate was chilled by adding ice and treated with 6N hydrochloric acid. The precipitated product was recovered by filtration, washed with water, and dried to provide 150 g of the desired subtitled intermediate.

B. Preparation of 4-{[3,5-bis(1,1-dimethylethyl)-4-hydroxyphenyl]methylene}-1,3-oxothiolan-5-one.

Following the procedure of Agr. Biol. Chem., 29(8), 728 (1965), six grams of the mercaptoacrylic acid from above were heated on a steam bath with 36 ml of acetic acid and 6 ml of formaldehyde (37% solution) for one hour. Evaporation of the mixture and chromatography of the residue over silica gel provided 1.7 g of the desired product, m.p. 127-129°C.

| Analysis for $C_{18}H_{24}O_3S$ : | | |
|---|---|---|
| Calculated: | C, 67.47; | H, 7.55; |
| Found: | C, 67.71; | H, 7.62. |

Examples 18-19

The following compounds were prepared according to the procedure of Example 1 employing the appropriate N-substituted rhodanine.

18. 5-{[3,5-bis(1,1-dimethylethyl)-4-hydroxyphenyl]methylene}-3-cyclopropyl-2-thioxo-4-thiazolidinone, 93% yield, m.p. 158-168°C.

19. 5-{[3,5-bis(1,1-dimethylethyl)-4-hydroxyphenyl]methylene}-3-dimethylamino-2-thioxo-4-thiazolidinone, 65% yield.

Examples 20-21

The thiones of Examples 18-19 were reduced using the procedure of Example 10 to provide the following compounds.

20. 5-{[3,5-bis(1,1-dimethylethyl)-4-hydroxyphenyl]methylene}-3-cyclopropyl-4-thiazolidinone, 46% yield, m.p. 162-164°C.

| Analysis for $C_{21}H_{29}NO_2S$ : | | | |
|---|---|---|---|
| Calculated: | C, 70.16; | H, 8.13; | N, 3.90; |
| Found: | C, 69.91; | H, 8.23; | N, 3.75. |

21. 5-{[3,5-bis(1,1-dimethylethyl)-4-hydroxyphenyl]methylene}-3-dimethylamino-4-thiazolidinone, 41% yield, m.p. 138-141°C.

| Analysis for $C_{20}H_{30}N_2O_2S$: | | | |
|---|---|---|---|
| Calculated: | C, 66.26; | H, 8.34; | N, 7.73; |
| Found: | C, 66.55; | H, 8.59; | N, 7.47. |

Example 22

5-{[3,5-bis(1,1-dimethylethyl)-4-hydroxyphenyl]methylene}-3-(dimethylamino)-4-thiazolidinone, 1-oxide

Under a nitrogen atmosphere, 9.06 g of the compound of Example 21 were dissolved in 125 ml of chloroform while cooled in an ice bath. To this were added (dropwise) 5.39 g of meta-chloroperbenzoic acid in 75 ml of chloroform over a period of 25 minutes at 0°c. After an additional 10 minutes, the reaction mixture was transferred to a separatory funnel, washed with saturated sodium bicarbonate and the layers separated. The aqueous layer was washed with chloroform. This wash was added to the original chloroform extract resulting in a slow breaking emulsion. The organic

layer was dried over sodium sulfate, filtered, washed and the solvent removed by evaporation. The resultant residue was subsequently taken up in about 225 ml of ethyl acetate with heating on a steam bath, and subsequently diluted with about 100 ml of hexane. A precipitate formed and the resultant mixture was allowed to cool to room temperature overnight. The precipitate was filtered, washed with hexane, allowed to air dry for one hour and subsequently dissolved in 100 ml of isopropyl alcohol on a steam bath. The resultant solution was allowed to cool to room temperature overnight resulting in a precipitate which was again washed with hexane and then dried under vacuum at 80°C for about four hours to yield 5.41 g of the title compound, m.p. 198-201°C.

| Analysis for $C_{20}H_{30}N_2O_3S$ : | | | |
|---|---|---|---|
| Calculated: | C, 63.46; | H, 7.97; | N, 7.40; |
| Found: | C, 63.68; | H, 7.78; | N, 7.56. |

Example 23

5-{[3,5-bis(1,1-dimethylethyl)-4-hydroxyphenyl]methylene}-4-thiazolidinone, 1-oxide

Utilizing procedures similar to those set forth in Example 22, 5.12 g of the title compound were prepared from 5-[[3,5-bis(1,1-dimethylethyl)-4-hydroxyphenyl]methylene]-3-dimethylamino-4-thiazolidinone (i.e., the compound of Example 21), m.p. 103-110°C.

| Analysis for $C_{18}H_{25}NO_3S$ : | | | |
|---|---|---|---|
| Calculated: | C, 63.77; | H, 8.41; | N, 3.54; |
| Found: | C, 64.11; | H, 8.26; | N, 3.55. |

Utilizing procedures set forth herein the following additional compounds were prepared.

Example 24

5-{[3,5-bis(1,1-dimethylethyl)-4-hydroxyphenyl]methylene}-3-(2-propenyl)-4-thiazolidinone, m.p. 154.5-156.5°C.

| Analysis for $C_{21}H_{29}NO_2S$ : | | | | |
|---|---|---|---|---|
| Calculated: | C, 70.16; | H, 8.13; | N, 3.90; | S, 8.92; |
| Found: | C, 70.27; | H, 8.21; | N, 4.01; | S, 9.09. |

Example 25

5-{[3,5-bis(1,1-dimethylethyl)-4-hydroxyphenyl]methylmethylene}-3-methyl-4-thiazolidinone, m.p., 152.5-153.5°C.

| Analysis for $C_{20}H_{29}NO_2S$ : | | | |
|---|---|---|---|
| Calculated: | C, 69.12; | H, 8.41; | N, 4.03; |
| Found: | C, 69.18; | H, 8.25; | N, 4.26. |

Example 26

5-{[3,5-bis(1,1-dimethylethyl)-4-hydroxyphenyl]-methylene}-3-[2-(acetyloxy)ethyl]-4-thiazolidinone

A. Preparation of N-(2-hydroxyethyl)rhodanine.

Sixty milliliters of carbon disulfide were added to 200 ml of diethyl ether. The solution was chilled to -5°C and slowly added to a solution of 138 ml of ethanolamine in 250 ml of ethanol. After holding the mixture at ambient temperature for 16 hours, the resulting top layer was decanted and the residual oil washed twice with 50 ml of diethyl ether. To the oil was added a 0°C solution of 71 g of chloroacetic acid in 150 ml of 5N sodium hydroxide. The reaction was then allowed to stand for 75 minutes. The mixture was poured into 400 ml of 6N hydrochloric acid and the resulting mixture heated to 91°C for 20 minutes. The heat was removed, and the solution allowed to stand for 5 hours at ambient

temperature. An oily organic layer was separated from the aqueous layer and the aqueous layer extracted twice with 250 ml of ethyl acetate. The organic layers were combined, washed twice with a saturated sodium chloride solution, dried and concentrated in vacuo to provide 113.4 g of the desired subtitled intermediate. This intermediate was used without further purification.

B. Preparation of 5-{[3,5-bis(1,1-dimethylethyl)-4-hydroxyphenyl]methylene}-3-[2-(acetyloxy)-ethyl]-2-thioxo-4-thiazolidinone.

A mixture of 124 g of 3,5-di-tert-butyl-4-hydroxybenzaldehyde, 103.1 g of the subtitle intermediate of Example 26A above, 346.9 g of sodium acetate, and 2.65 1 of glacial acetic acid was heated at reflux temperature for 7.5 hours under a nitrogen atmosphere. The heat was removed and the mixture allowed to cool overnight, with stirring. The resulting precipitate was removed by filtration and the filtrate concentrated in vacuo. Two liters of ethyl acetate were added to the residue, followed by 1.5 l of water. The layers were separated and the water layer extracted with 500 ml of ethyl acetate. The organic layers were combined, washed with water and a sodium bicarbonate solution, dried over sodium sulfate, and concentrated in vacuo. The residue was purified by chromatography over silica gel, eluting with a gradient of toluene to 7% ethyl acetate in toluene. The appropriate fractions were combined and concentrated in vacuo. The residue was crystallized from 75 ml of ethanol to provide 10.28 g of the desired subtitled intermediate, m.p. 140-143°C.

C. 5-{[3,5-bis(1,1-dimethylethyl)-4-hydroxyphenyl]methylene}-3-[2-(acetyloxy)ethyl]-4-thiazolidinone,

Under a nitrogen atmosphere, 82.2 g of 5-{[3,5-bis(1,1-dimethylethyl)-4-hydroxyphenyl]methylene}-3-[2-(acetyloxy)ethyl]-2-thioxo-4-thiazolidinone in 950 ml of toluene were heated to 65°C. Tri-n-butyl tin hydride (219.7 g) and AIBN (4.65 g) were added and the solution heated at reflux temperature for an additional 10 minutes. After cooling, the mixture was washed with 1.25 1 of 1N hydrochloric acid, followed by 500 ml of a saturated sodium chloride solution. The organic layer was stripped and allowed to stand overnight, during which time a precipitate separated. The liquid portion was decanted off, and the resulting residue was purified by chromatography over silica gel, eluting with a gradient of 25-50% of ethyl acetate in hexane. The appropriate fractions were combined and concentrated in vacuo to provide 45.7 g of the desired titled compound, m.p. = 152-155°C.

| Analysis for $C_{22}H_{31}NO_4S$ : | | | |
|---|---|---|---|
| Calculated: | C, 60.66; | H, 6.71; | N, 3.22; |
| Found: | C, 60.71; | H, 6.90; | N, 3.21. |

Example 27

5-{[3,5-bis(1,1-dimethylethyl)-4-hydroxyphenyl]methylene}-3-(2-aminoethyl)-4-thiazolidinone

A. Preparation of 5-{[3,5-bis(1,1-dimethylethyl)-4-hydroxyphenyl]methylene}-3-(2-hydroxyethyl)-4-thiazolidinone

A solution of 85.2 g of 5-{[3,5-bis(1,1-dimethylethyl)-4-hydroxyphenyl]methylene}-3-[2-(acetyloxy)ethyl]-4-thiazolidinone from Example 26 in 1.5 1 of acetonitrile was treated with 1.0 l of concentrated ammonium hydroxide. The reaction mixture was allowed to stand for approximately 90 hours at room temperature. The solution was concentrated in vacuo and 500 ml of ethyl acetate were added, with the pH adjusted to 3.0 with concentrated hydrochloric acid. The layers were separated and the aqueous layer extracted with 250 ml of ethyl acetate. The combined organic layers were washed with 250 ml of a saturated sodium chloride solution and concentrated in vacuo. The residue was crystallized from 95 ml of hexane and 70 ml of ethyl acetate to provide 35.68 g of the desired subtitled intermediate, m.p. 131-135°C.

| Analysis for $C_{20}H_{29}NO_3S$ : | | | |
|---|---|---|---|
| Calculated: | C, 66.08; | H, 8.04; | N, 3.85; |
| Found: | C, 65.91; | H, 8.21; | N, 3.96. |

B. Preparation of 5-{[3,5-bis(1,1-dimethylethyl)-4-hydroxyphenyl]methylene}-3-[2-(tosyloxy)ethyl]-4-thiazolidinone.

A solution of 30.2 g of the hydroxyethyl intermediate of Example 27A, above, in 415 ml of pyridine was cooled to

-3°C. p-Toluenesulfonyl chloride (39.6 g) was added with stirring. After stirring the mixture at 0°C for 4 hours, the solution was stored in a refrigerator overnight at -10°C. Approximately 1.0 l of ice water was added and the mixture extracted twice with 700 ml of diethyl ether. The combined organic layers were washed twice with 1.0 l of cold 1N hydrochloric acid, dried over sodium sulfate and concentrated in vacuo to provide 41.7 g of the desired tosyl intermediate. This intermediate was used without further purification.

C. Preparation of 5-{[3,5-bis(1,1-dimethylethyl)-4-hydroxyphenyl]methylene}-3-(2-aminoethyl)-4-thiazolidinone.

A mixture of 13 g of the tosyl intermediate of Example 27B, above, 250 ml of concentrated ammonium hydroxide, and 250 ml of acetonitrile was stirred for 2 days at room temperature. The mixture was concentrated in vacuo and diluted with 500 ml of ethyl acetate. The pH was adjusted to 9.0, and the layers separated. The organic layer was washed twice with water, dried, and concentrated in vacuo. The residue was purified by chromatography over silica gel, eluting with a gradient from methylene chloride to 90:10:1 methylene chloride/ethanol/ammonium hydroxide, respectively. The desired fractions were combined and concentrated in vacuo. The residue was triturated with hexane to provide 1.47 g of the desired title product, m.p. 176-178°C.

| Analysis for $C_{20}H_{30}N_2O_2S$ : | | | |
|---|---|---|---|
| Calculated: | C, 66.26; | H, 8.32; | N, 7.73; |
| Found: | C, 66.25; | H, 8.24; | N, 7.59. |

Examples 28-30

The following compounds were prepared by reacting the intermediate of Example 27B with the appropriate amine according to the procedures described herein.

28. 5-{[3,5-bis(1,1-dimethylethyl)-4-hydroxyphenyl]methylene}-3-[2-(methylamino)ethyl]-4-thiazolidinone, 28% yield, m.p. 137-140°C.

| Analysis for $C_{21}H_{32}N_2O_2S$ : | | | |
|---|---|---|---|
| Calculated: | C, 66.98; | H, 8.57; | N, 7.44; |
| Found: | C, 66.76; | H, 8.33; | N, 7.24. |

29. 5-{[3,5-bis(1,1-dimethylethyl)-4-hydroxyphenyl]methylene}-3-[2-(dimethylamino)ethyl]-4-thiazolidinone, 64% yield, m.p. 148-153°C.

| Analysis for $C_{22}H_{34}N_2O_2S$ : | | | |
|---|---|---|---|
| Calculated: | C, 67.65; | H, 8.77; | N, 7.17; |
| Found: | C, 67.43; | H, 8.55; | N, 6.98. |

30. 5-{[3,5-bis(1,1-dimethylethyl)-4-hydroxyphenyl]methylene}-3-[2-(2-hydroxyethylamino)ethyl]-4-thiazolidinone, 59% yield, m.p. 174-176°C.

Utilizing the procedures set forth herein the following additional compounds were prepared.

Example 31

5-{[3,5-bis(1,1-dimethylethyl)-4-hydroxyphenyl]methylene}-3-[2-(methyl-2-propynylamino)ethyl]-4-thiazolidinone, m. p. 116-118°C.

| Analysis for $C_{24}H_{34}N_2O_2S$ : | | | |
|---|---|---|---|
| Calculated: | C, 69.53; | H, 8.27; | N, 6.76; |
| Found: | C, 69.27; | H, 8.46; | N, 6.65. |

Example 32

5-{[3,5-bis(1,1-dimethylethyl)-4-hydroxyphenyl]methylene}-3-{2-[[2-(dimethylamino)ethyl]amino]-ethyl}-4-thiazolidinone, m.p. 245-249°C (dec.)

| Analysis for $C_{24}H_{39}N_3O_2S$ : | | | |
|---|---|---|---|
| Calculated: | C, 56.90; | H, 8.16; | N, 8.30; |
| Found: | C, 57.12; | H, 7.98; | N, 8.09. |

Example 33

5-{[3,5-bis(1,1-dimethylethyl)-4-hydroxyphenyl]methylene}-3-{2-[(phenylmethyl)amino]ethyl}-4-thiazolidinone hydrochloride, m.p. 254-259°C (dec.)

| Analysis for $C_{27}H_{36}N_2O_2S$ : | | | |
|---|---|---|---|
| Calculated: | C, 66.30; | H, 7.63; | N, 5.73; |
| Found: | C, 66.46; | H, 7.53; | N, 5.80. |

Example 34

5-{[3,5-bis(1,1-dimethylethyl)-4-hydroxyphenyl]methylene}-3-[3-(methylamino)propyl]-4-thiazolidinone, m.p. 177-180°C

| Analysis for $C_{22}H_{34}N_2O_2S$ : | | | |
|---|---|---|---|
| Calculated: | C, 67.65; | H, 8.77; | N, 7.17; |
| Found: | C, 67.72; | H, 8.94; | N, 7.00. |

Example 35

5-{[3,5-bis(1,1-dimethylethyl)-4-hydroxyphenyl]methylene}-3-(2-hydroxyethyl)-4-thiazolidinone, m.p. 131-135°C

| Analysis for $C_{20}H_{29}NO_3S$ : | | | |
|---|---|---|---|
| Calculated: | C, 66.08; | H, 8.04; | N, 3.85; |
| Found: | C, 66.36; | H, 8.13; | N, 3.87. |

Example 36

5-{[3,5-bis(1,1-dimethylethyl)-4-hydroxyphenyl]methylene}-3-[(4-methoxyphenyl)methyl]-4-thiazolidinone, m.p. 129-130°C

| Analysis for $C_{26}H_{33}NO_3S$ : | | | |
|---|---|---|---|
| Calculated: | C, 71.04; | H, 7.57; | N, 3.19; |
| Found: | C, 70.75; | H, 7.69; | N, 3.18. |

Example 37

5-{[3,5-bis(1,1-dimethylethyl)-4-hydroxyphenyl]methylene}-3-[2-(propylamino)ethyl]-4-thiazolidinone, m.p. 155-158°C

| Analysis for $C_{23}H_{36}N_2O_2S$ : | | | |
|---|---|---|---|
| Calculated: | C, 68.28; | H, 8.97; | N, 6.92; |
| Found: | C, 68.38; | H, 9.17; | N, 7.13. |

Example 38

5-{[3,5-bis(1,1-dimethylethyl)-4-hydroxyphenyl]methylene}-4-oxo-3-thiazolidineacetonitrile.

5-{[3,5-Bis(1,1-dimethylethyl)-4-hydroxyphenyl]methylene)-4-thiazolidinone (7.03 g) and 2.64 g of bromoacetonitrile were reacted in the presence of 0.97 g of 60% sodium hydride in mineral oil and 330 ml of tetrahydrofuran. Work-up of the reaction mixture provided 3.21 g of the desired title product, m.p. 186-188°C.

| Analysis for $C_{20}H_{26}N_2O_2S$ : | | | |
|---|---|---|---|
| Calculated: | C, 67.01; | H, 7.31; | N, 7.81; |
| Found: | C, 66.80; | H, 7.36; | N, 7.67. |

Example 39

5-{[3,5-bis(1,1-dimethylethyl)-4-hydroxyphenyl]methylene}-3-(1H-tetrazol-5-ylmethyl)-4-thiazolidinone

The title compound was prepared from the nitrile of Example 38 by treatment with tri-N-butyl azide in ethylene glycol dimethyl ether, melting point 260-263°C (dec.)

| Analysis for $C_{20}H_{27}N_5O_2S$: | | | |
|---|---|---|---|
| Calculated: | C, 59.83; | H, 6.78; | N, 17.44; |
| Found: | C, 59.93; | H, 6.82; | N, 17.32. |

Example 40

5-{[3,5-bis(1,1-dimethylethyl)-4-hydroxyphenyl]methylene}-3-(ethylmethylamino)-4-thiazolidinone

A. Preparation of nitrosomethylethylamine

70.15 g of methylethylamine were maintained at 10°C on an ice bath. To this was added 104 ml of concentrated hydrochloric acid (dropwise, with stirring). The addition of the hydrochloric acid was continued at a rate which maintained the reaction temperature at about 15°C. Upon completion of the acid addition, 90 g of sodium nitrite were added to the reaction in small portions. Upon dissolution of the sodium nitrite, a gas formed and the temperature of the reaction mixture dropped to about 0°C. The mixture was placed in an oil bath and heated to about 70°C during completion of the sodium nitrite addition. After about 90 minutes, gas evolution had ceased and an additional 10 ml of concentrated hydrochloric acid were added, generating additional gas evolution. Upon further stirring, an additional 5 ml of concentrated hydrochloric acid were added. The reaction mixture was allowed to stir overnight, with cooling, after which the resultant layers were separated. The upper layer was then extracted with 100 ml of diethyl ether, followed by a second extraction with an additional 50 ml of diethyl ether. The extracts were combined and evaporated on a steam bath to yield 26.8 g of the desired subtitled intermediate.

B. Preparation of N,N-methylethylhydrazine

To a stirred mixture of 46.75 g nitrosomethylethylamine, 588 ml of water and 133.9 g of zinc dust was added (dropwise) 159 ml of acetic acid. The addition was completed over approximately two hours, and the reaction mixture was maintained at 25-30°C. The reaction mixture was then heated to about 90°C, allowed to cool after about 30 minutes

to 60°C, allowed to cool to room temperature and then filtered. The aqueous filtrate was cooled in an ice bath and adjusted to pH 11 with 50% sodium hydroxide. A white precipitate formed which made additional stirring difficult. The white suspension was filtered and washed with two portions of water. The original filtrate and the first wash were combined for distillation. The mixture was heated and various fractions collected over a temperature range of about 67°C to 99°C, each of which contained the desired subtitled intermediate.

C. Preparation of S-carboxymethyl-N'-dithiocarboxy N-methyl-N-ethylhydrazine

N,N-Methylethylhydrazine (13.3 g) and 20 ml of ethanol were cooled in an ice/water bath. To this was added a mixture of 4.69 ml of carbon disulfide and 15.6 ml of diethyl ether, dropwise, with stirring over a period of about 13 minutes. The resultant yellow solution was stirred for an additional 15 minutes at 0°C and then removed from the ice bath. Additional diethyl ether was added to induce the formation of a precipitate. When the total volume reached 125 ml (due to addition of diethyl ether) two layers had formed. Within about 10 minutes the oily lower layer began to crystallize and the reaction mixture was allowed to stand at room temperature overnight. The reaction mixture was then maintained at 5°C for two hours prior to filtering. The mixture was filtered, washed with diethyl ether, dried under vacuum at room temperature for three hours and then added to a stirred, cooled (4°C) mixture of 5.66 g of chloroacetic acid in 12 ml of 5N sodium hydroxide. The reaction mixture was removed from the ice bath, allowed to warm to room temperature, with stirring, for 45 minutes, and then added, over a period of about 2 minutes, to 31.2 ml of 6N hydrochloric acid heated to 85°C. The mixture was warmed to 90°C over approximately 10 minutes and allowed to cool, while stirring, to room temperature overnight. A precipitate formed which was filtered, washed lightly with cold water and allowed to air dry for about 15 minutes. The precipitate was then dried under vacuum at 80°C for three days to yield 4.64 g of the desired subtitled intermediate. The filtrate was stirred at room temperature for 3 days and additional precipitate formed which was subsequently filtered, washed lightly with water and dried under vacuum at 80°C for 24 hours to yield an additional 1.76 g of the desired subtitled intermediate.

D. Preparation of 5-{[3,5-bis(1,1-dimethylethyl)-4-hydroxyphenyl]methylene}-3-(ethylmethylamino)-2-thioxo-4-thiazolidinone

Under nitrogen atmosphere, 6.40 g of the intermediate prepared in Example 40C, 154 ml of acetic acid and 8.82 g of sodium acetate were stirred for 10 minutes. 7.2 g of 3,5-bis(1,1-dimethylethyl)-4-hydroxybenzaldehyde were added and the resultant mixture was heated at reflux temperature for 23 hours, then poured into a 400 ml mixture of ice/water, with stirring. The resultant mixture was stirred for an additional 20 minutes, filtered and then washed with a volume of water to give the desired subtitled intermediate. This intermediate was dried under vacuum at 100°C for three days, after which it was dissolved in 45 ml of ethanol on a steam bath and diluted with water dropwise, while stirring, until cloudiness persisted. This mixture was then stirred for an additional five minutes, allowed to cool to room temperature overnight and dried under vacuum at 80°C for four hours to render 6.99 g of the desired subtitled intermediate.

E. Preparation of 5-{[3,5-bis(1,1-dimethylethyl)-4-hydroxyphenyl]methylene}-3-(ethylmethylamino)-4-thiazolidinone

7.02 g of 5-{[3,5-bis(1,1-dimethylethyl)-4-hydroxyphenyl]methylene}-3-(ethylmethylamino)-2-thioxo-4-thiazolidinone (from Example 40D) and 86.3 ml of toluene were stirred and heated to 60°C under a nitrogen atmosphere. To this were added 18.6 ml of tri-n-butyl tin hydride and 0.43 g of AIBN. The resultant mixture was heated to reflux temperature for 30 minutes. At that time an additional 0.43 g of AIBN were added. The resultant mixture was heated at reflux temperature for an additional 30 minutes, cooled and transferred to a separatory funnel. To this were added 100 ml of 1N hydrochloric acid and 100 ml of ethyl acetate. The resultant mixture was shaken and separated. The organic layer was washed with brine, dried over sodium sulfate, filtered, evaporated and subsequently chased with chloroform to give an orange/red oil which was taken up in 50 ml of chloroform and filtered. The filtrate was chromatographed on a silica gel column using an 8000 ml gradient of 10-40% ethyl acetate in hexane. Those fractions identified as containing product were evaporated and chased with chloroform. To these fractions were added 15 ml of hexane and the resultant solution was heated slightly. A precipitate formed which was diluted to about 25 ml with additional hexane. The resultant mixture was triturated for about 2 hours, filtered and then washed with hexane to yield 1.94 g of the desired product, m.p. 133.5-135°C.

| Analysis for $C_{21}H_{32}N_2O_2S$: | | | |
|---|---|---|---|
| Calculated: | C, 66.98; | H, 8.57; | N, 7.44; |
| Found: | C, 66.97; | H, 8.80; | N, 7.24. |

Utilizing the procedures substantially as described in Example 40 and described elsewhere herein, the following additional compounds were prepared.

Example 41

5-{[3,5-bis(1,1-dimethylethyl)-4-hydroxyphenyl]methylene}-3-(butylmethylamino)-4-thiazolidinone, m.p. 128.5-131°C

| Analysis for $C_{23}H_{36}N_2O_2S$: | | | |
|---|---|---|---|
| Calculated: | C, 68.28; | H, 8.97; | N, 6.92; |
| Found: | C, 68.45; | H, 9.00; | N, 6.70. |

Example 42

5-{[3,5-bis(1,1-dimethylethyl)-4-hydroxyphenyl]methylene}-3-[(2-phenylethyl)methylamino]-4-thiazolidinone, m.p. 93-97°C

| Analysis for $C_{27}H_{36}N_2O_2S$: | | | |
|---|---|---|---|
| Calculated: | C, 71.64; | H, 8.02; | N, 6.19; |
| Found: | C, 71.48; | H, 8.30; | N, 5.81. |

Example 43

5-{[3,5-bis(1,1-dimethylethyl)-4-hydroxyphenyl]methylene}-3-(4-methyl-1-piperazinyl)-4-thiazolidinone, m.p. 221-225°C

| Analysis for $C_{23}H_{34}N_2O_2S$: | | | |
|---|---|---|---|
| Calculated: | C, 66.15; | H, 8.45; | N, 10.06; |
| Found: | C, 66.10; | H, 8.36; | N, 9.81. |

Example 44

5-{[3,5-bis(1,1-dimethylethyl)-4-hydroxyphenyl]methylene}-3-(1-piperidinyl)-4-thiazolidinone, m.p. 213-215°C

| Analysis for $C_{23}H_{34}N_2O_2S$: | | | |
|---|---|---|---|
| Calculated: | C, 68.62; | H, 8.51; | N, 6.96; |
| Found: | C, 68.41; | H, 8.49; | N, 7.26. |

Example 45

5-{[3,5-bis(1,1-dimethylethyl)-4-hydroxyphenyl]methylene}-3-(4-morpholinyl)-4-thiazolidinone, m.p. 226-228°C

| Analysis for $C_{22}H_{32}N_2O_3S$: | | | |
|---|---|---|---|
| Calculated: | C, 65.31; | H, 7.97; | N, 6.92; |
| Found: | C, 65.59; | H, 7.94; | N, 7.20. |

Example 46

5-{1-[3,5-bis(1,1-dimethylethyl)-4-hydroxyphenyl]methylmethylene}-3-(dimethylamino)-4-thiazolidinone

A. Preparation of 1-[3,5-bis(1,1-dimethylethyl)-4-hydroxyphenyl]ethanone

Under a nitrogen atmosphere, 6.89 ml of acetylchloride and 14.75 ml of stannic chloride were dissolved in 200 ml of methylene chloride, then chilled to -4°C. To this were added 20 g of 2,6-di-t-butylphenol (in 100 ml of methylene chloride) over 10 minutes. The resultant mixture was stirred for 30 minutes at 0°C, then poured into a mixture of 400 ml of ice and 1N hydrochloric acid and stirred. The mixture separated into layers which were subsequently separated. The organic layer was washed with 100 ml of saturated sodium bicarbonate and 100 ml of brine. The organic layer was dried and the solvent evaporated to give 23.39 g of the desired subtitled intermediate.

B. Preparation of 5-{1-[3,5-bis(1,1-dimethylethyl)-4-hydroxyphenyl]methylmethylene}-3-(dimethylamino)-2-thioxo-4-thiazolidinone

To 675 ml of toluene were added 20.9 g of 1-[3,5-bis-(1,1-dimethylethyl)-4-hydroxyphenyl]ethanone, 13.3 g of N-dimethylaminorhodanine 6.5 g of ammonium acetate and about 20 ml of acetic acid. The mixture was heated at reflux temperature and any aqueous layer generated was collected in a Dean-Stark trap. Over the following 52 hours an additional 39 g of ammonium acetate and about 100 ml of acetic acid were added, in increments, and a total of 89.2 ml of aqueous phase was drawn off. Following workup by conventional techniques, 17.1 g of the desired subtitled intermediate was recovered.

C. Preparation of 5-{1-[3,5-bis(1,1-dimethylethyl)-4-hydroxyphenyl]methylmethylene}-3-(dimethylamino)-4-thiazolidinone

Utilizing the procedure set forth in Example 40E, reduction of the thione was effected utilizing tri-n-butyl tin hydride and AIBN in toluene to render the desired title compound, m.p. 181-186°C

| Analysis for $C_{21}H_{32}N_2O_2S$: | | | |
|---|---|---|---|
| Calculated: | C, 66.98; | H, 8.57; | N, 7.44; |
| Found: | C, 66.84; | H, 8.48; | N, 7.39. |

Example 47

5-{[3,5-bis(1,1-dimethylethyl)-4-hydroxyphenyl]methylene}-3-(methylamino)-4-thiazolidinone

A. Preparation of benzaldehydemethylhydrazone

Benzaldehyde (50.8 ml, 500 mmol) and 26.5 ml (500 mmol) of methylhydrazine were dissolved in 1.0 1 of methanol. The mixture was stirred together at room temperature for 75 minutes and then stripped of solvent to give 67.8 g of the desired subtitled intermediate.

B. Preparation of benzaldehyde N-methyl, N-2-propenylhydrazone

67.8 g of benzaldehydemethylhydrazone (as prepared in Example 47A, above), 60.5 g of allyl bromide and 50.5 g of triethylamine were dissolved in 1.0 l of acetonitrile and the mixture was heated at reflux temperature for 16 hours, then cooled. An additional 45 g of allyl bromide and 38 g of triethylamine were added and the mixture was again heated at reflux for an additional 7 hours, cooled and then stripped of solvent to yield 268 g of a residue. To this residue were added 500 ml of THF and the resultant mixture was shaken, filtered and washed with an additional 125 ml of THF. The filtrate was stripped of solvent to yield 67 g of the desired subtitled intermediate.

C. Preparation of N-methyl, N-2-propenylhydrazine

59.9 g of benzaldehyde N-methyl, N-2-propenylhydrazone (prepared as described in Example 47B, above), 44 g of hydrazine and 137 ml of ethanol were heated at reflux temperature for 21.5 hours and allowed to cool. The reflux condenser was replaced with a distillation head and the mixture was distilled at atmospheric pressure. The first three distillates were collected, combined and 100 ml of 1N HCl were added. An additional 100 ml of concentrated HCl were added, with ice, and the resultant mixture separated and washed with a small amount of ethyl acetate. The resultant

layers were separated and the water distilled off until solids clogged the stir bar. The solids were filtered off and the filtrate was stripped and added to 125 ml of chilled 50% NaOH. The resulting solid was filtered off and discarded. The filtrate contained two layers which were separated. The top layer contained the desired subtitled intermediate and the bottom, aqueous layer was extracted with diethyl ether which, upon stripping, gave additional product.

D. Preparation of N-Methyl, N-3-propenyl-5-carboxymethyl-dithiocarbamate

To 12.67 g of N-methyl, N-2-propenylhydrazine (prepared as described in Example 47C) in 23 ml of cold (0°C) ethanol was added a solution of 11.18 g of carbon disulfide in 26 ml of diethyl ether. The resultant mixture was removed from the ice bath and allowed to stand at room temperature for about 15.5 hours, after which the solvent was stripped to yield a residue of approximately 36.5 g. To this residue were added 13.9 g of chloroacetic acid dissolved in 29.5 ml of 5N NaOH (chilled in an ice bath). The resultant solution was allowed to stand for 3 hours at room temperature. The pH of the solution was lowered to about 3 by adding 8 ml of concentrated hydrochloric acid. To this were added 50 ml of diethyl ether, resulting in a three phase separation. The aqueous phases were pooled and extracted with 50 ml of chloroform, then stripped of solvent to yield approximately 40.4 g of the desired subtitled intermediate.

E. Preparation of 5-{[3,5-bis(1,1-dimethylethyl)-4-hydroxyphenyl]methylene}-2-thioxo-3-(methyl-2-propenylamino)-4-thiazolidinone

29.3 g of 3,5-di-tert-butyl-4-hydroxybenzaldehyde, 38.8 g of the intermediate prepared as described in Example 47D, above, and 40.34 g of sodium acetate were mixed in 810 ml of acetic acid and the resultant solution was heated at reflux temperature for 24 hours. The solution was allowed to cool and stirred for an additional 60 hours at room temperature. The solution was then poured into 2 l of ice water, separated and washed with an additional volume of water to yield about 44 g of the desired subtitled intermediate.

F. Preparation of 5-{[3,5-bis(1,1-dimethylethyl)-4-hydroxyphenyl]methylene}-3-(methyl-2-propenylamino)-4-thiazolidinone

Utilizing the procedure described in Example 40E, and elsewhere herein, 42.8 g of the thione of Example 47E, above, were reduced to the desired subtitled intermediate (8.34 g).

G. Preparation of 5-{[3,5-bis(1,1-dimethylethyl)-4-hydroxyphenyl]methylene}-3-(methylamino)-4-thiazolidinone

6.11 g of the subtitled intermediate of Example 47F were dissolved in a mixture of 135 ml ethanol and 15.3 ml of water and the mixture heated to 70°C. 50 mg of tris-(triphenylphosphine)rhodium (I) chloride were added and the mixture heated at reflux temperature for 50 minutes, after which an additional 550 mg of the catalyst were added followed by heating at reflux temperature for an additional 2.5 hours. The mixture was cooled, stirred at room temperature overnight and stripped of solvent to give 2.05 g of the desired product after further workup, m.p. 151-153.5°C.

| Analysis for $C_{19}H_{28}N_2O_2S$: | | | |
|---|---|---|---|
| Calculated: | C, 65.86; | H, 7.56; | N, 8.09; |
| Found: | C, 65.67; | H, 7.81; | N, 8.34. |

Utilizing the procedures set forth herein, the following additional compounds were prepared.

Example 48

5-{1-[3,5-bis(1,1-dimethylethyl)-4-hydroxyphenyl]methylmethylene}-4-thiazolidinone, m.p. >230°C

| Analysis for $C_{19}H_{27}N_1O_2S$: | | | |
|---|---|---|---|
| Calculated: | C, 68.43; | H, 8.16; | N, 4.20; |
| Found: | C, 68.60; | H, 8.28; | N, 4.17. |

Example 49

5-{[3,5-bis(1,1-dimethylethyl)-4-hydroxyphenyl]methylene}-3-[2-(4-morpholinyl)ethyl]amino-4-thiazolidinone, m.p. 218-222°C (dec.)

| Analysis for $C_{24}H_{36}N_2O_3S$: | | | |
|---|---|---|---|
| Calculated: | C, 66.83; | H, 8.39; | N, 6.48; |
| Found: | C, 66.58; | H, 8.15; | N, 6.67. |

Example 50

3-amino-5-{[3,5-bis(1,1-dimethylethyl)-4-hydroxyphenyl]methylene}-4-thiazolidinone, m.p. 162-164°C

| Analysis for $C_{18}H_{26}N_2O_2S$: | | | |
|---|---|---|---|
| Calculated: | C, 64.64; | H, 7.84; | N, 8.38; |
| Found: | C, 64.85; | H, 7.92; | N, 8.19. |

Example 51

5-{[3,5-bis(1,1-dimethylethyl)-4-hydroxyphenyl]methylene}-3-(propylamino)-4-thiazolidinone, m.p. 131-136°C

| Analysis for $C_{21}H_{32}N_2O_2S$: | | | |
|---|---|---|---|
| Calculated: | C, 66.98; | H, 8.57; | N, 7.44; |
| Found: | C, 67.22; | H, 8.70; | N, 7.37. |

Example 52

5-{[3,5-bis(1,1-dimethylethyl)-4-hydroxyphenyl]methylene}-3-(ethylamino)-4-thiazolidinone, m.p. 125-127°C

| Analysis for $C_{20}H_{30}N_2O_2S$: | | | |
|---|---|---|---|
| Calculated: | C, 66.26; | H, 8.34; | N, 7.73; |
| Found: | C, 66.46; | H, 8.35; | N, 7.95. |

Example 53

5-{[3,5-bis(1,1-dimethylethyl)-4-hydroxyphenyl]methylene}-3-(dimethylamino)-2-thioxo-4-thiazolidinone, m.p. 158-160°C

| Analysis for $C_{20}H_{28}N_2O_2S_2$: | | | |
|---|---|---|---|
| Calculated: | C, 61.19; | H, 7.19; | N, 7.14; |
| Found: | C, 61.33; | H, 7.23; | N, 7.43. |

Example 54

5-{[3,5-bis(1,1-dimethylethyl)-4-hydroxyphenyl]methylene}-3-[2-(propylamino)ethyl]-4-thiazolidinone, m.p. 155-158°C

| Analysis for $C_{23}H_{36}N_2O_2S$: | | | |
|---|---|---|---|
| Calculated: | C, 68.28; | H, 8.97; | N, 6.92; |
| Found: | C, 68.38; | H, 9.17; | N, 7.13. |

Example 55

5-{[3,5-bis(1,1-dimethylethyl)-4-hydroxyphenyl]methylene}-3-[(2-hydroxyethyl)amino]-4-thiazolidinone, m.p. 128-132°C

| Analysis for $C_{20}H_{30}N_2O_3S$: | | | |
|---|---|---|---|
| Calculated: | C, 63.46; | H, 7.99; | N, 7.40; |
| Found: | C, 63.57; | H, 7.92; | N, 7.45. |

Example 56

5-[(4-hydroxy-3,5-dipropylphenyl)methylene]-3-methyl-4-thiazolidinone, m.p. 162-165°C

| Analysis for $C_{17}H_{23}NO_2S$: | | | |
|---|---|---|---|
| Calculated: | C, 66.85; | H, 7.59; | N, 4.59; |
| Found: | C, 67.12; | H, 7.37; | N, 4.52. |

Example 57

5-[(4-hydroxy-3,5-dipropylphenyl)methylene]-4-thiazolidinone, m.p. 202-205°C

| Analysis for $C_{16}H_{23}NO_2S$: | | | |
|---|---|---|---|
| Calculated: | C, 65.95; | H, 7.26; | N, 4.81; |
| Found: | C, 66.16; | H, 7.49; | N, 4.79. |

Example 58

5-{[3-(1,1-dimethylethyl)-4-hydroxy-5-methylphenyl]methylene}-4-thiazolidinone

A. Preparation of 3-(1,1-dimethylethyl)-4-hydroxy-5-methylbenzaldehyde

Under a nitrogen atmosphere, 76.65 g of 2-(1,1-dimethylethyl)-6-methylphenol (Aldrich), 65.42 g of hexamethylenetetramine and 700 ml of trifluoroacetic acid were stirred at reflux temperature for about 24 hours, then allowed to cool and evaporated. The residue from the evaporation was taken up in 1500 ml of water and 1000 ml of chloroform and neutralized to pH 7 with solid sodium carbonate. The resultant layers were separated and the aqueous layer was washed with chloroform. The organic layer was dried over sodium sulfate overnight, washed with a volume of chloroform and evaporated. The resultant residue was taken up in 375 ml of toluene, heated on a steam bath and then allowed to cool to room temperature overnight. Subsequent workup gave 28.3 g of the desired subtitled intermediate.

B. Preparation of 5-{[3-(1,1-dimethylethyl)-4-hydroxy-5-methylphenyl]methylene}-2-thioxo-4-thiazolidinone

28.3 g of the intermediate prepared in Example 58A, 24 g of N-aminorhodanine, 48.3 g of sodium acetate in 735

ml of acetic acid were heated to reflux temperature for about 7 hours and then allowed to cool to room temperature with continual stirring overnight. The resultant mixture was poured into 1500 ml of ice water, with stirring, and then filtered. The wet filter cake was transferred to a beaker and dissolved in a mixture of ethyl acetate and water and then separated. The organic layer was dried over sodium sulfate, filtered and then washed with ethyl acetate. Further workup, followed by trituration in hot chloroform and subsequent drying under vacuum, rendered about 18 g of the desired subtitled intermediate, m.p. 210-216°C.

C. Preparation of 5-{[3-(1,1-dimethylethyl)-4-hydroxy-5-methylphenyl]methylene}-4-thiazolidinone

Reduction of the thione of Example 58B as described herein was effected which, following workup, rendered 1.56 g of the titled product, m.p. 162-165°C.

| Analysis for $C_{15}H_{19}NO_2S$: | | | |
|---|---|---|---|
| Calculated: | C, 64.95; | H, 6.90; | N, 5.05; |
| Found: | C, 65.12; | H, 7.05; | N, 4.99. |

Utilizing the procedures set forth in Example 58 and elsewhere herein, the following additional compounds were prepared.

Example 59

3-amino-5-{[3-(1,1-dimethylethyl)-4-hydroxy-5-methylphenyl]methylene}-4-thiazolidinone, m.p. 110°C (dec.)

| Analysis for $C_{15}H_{20}N_2O_2S$: | | | |
|---|---|---|---|
| Calculated: | C, 61.81; | H, 7.29; | N, 9.01; |
| Found: | C, 61.90; | H, 7.47; | N, 8.78. |

Example 60

5-{[3-(1,1-dimethylethyl)-4-hydroxy-5-methylphenyl]methylene}-3-dimethylamino-2-thioxo-4-thiazolidinone, m.p. 189-190°C

| Analysis for $C_{17}H_{22}N_2O_2S$: | | | |
|---|---|---|---|
| Calculated: | C, 58.26; | H, 6.33; | N, 7.99; |
| Found: | C, 58.55; | H, 6.08; | N, 8.28. |

Example 61

5-{[3-(1,1-dimethylethyl)-4-hydroxy-5-methylphenyl]methylene}-3-methyl-4-thiazolidinone, m.p. 192-195°C

| Analysis for $C_{16}H_{21}NO_2S$: | | | |
|---|---|---|---|
| Calculated: | C, 65.95; | H, 7.26; | N, 4.81; |
| Found: | C, 66.24; | H, 7.17; | N, 5.02. |

Example 62

5-{[3-(1,1-dimethylethyl)-4-hydroxy-5-methylphenyl]methylene}-3-dimethylamino-4-thiazolidinone, m.p. 182-192°C

| Analysis for $C_{17}H_{24}N_2O_2S$: | | | |
|---|---|---|---|
| Calculated: | C, 63.72; | H, 7.55; | N, 8.74; |
| Found: | C, 63.45; | H, 7.58; | N, 8.93. |

Example 63

5-{[3,5-bis[3-(acetyloxy)propyl]-4-hydroxyphenyl]methylene}-3-methyl-4-thiazolidinone

A. Preparation of 3,5-di(3-trifluoroacetyloxypropyl)-4-hydroxybenzaldehyde

200 g of 3-(2-hydroxyphenyl)propene, 226 g of potassium carbonate and 180 g of allyl bromide were stirred in 490 ml of acetone at reflux temperature for 23 hours and then cooled. One liter of water was added and the resultant layers were separated. Subsequent distillation of the organic phase gave 256 g of 3-(2-propenyloxyphenyl)propene which was then heated with about 136 ml of diethylaniline for 45 minutes at 215-220°C. The mixture was cooled and 250 ml of ethyl acetate were added. The mixture was washed with two 450 ml portions of 1N HCl which, followed by subsequent workup, yielded about 232 g of 2,6-dipropenylphenol. 52.2 g of this phenolic intermediate were dissolved in 500 ml of THF and chilled to -5°C. 300 ml of one molar borane were added over 15 minutes (maximum temperature not exceeding 18°C), after which the mixture was stirred for 36 hours and chilled to 0°C. 80 ml of water were added over a 5 minute period, after which 120 ml of 5N sodium hydroxide were added all at once. When the temperature of the reaction mixture reached 1°C, 81 ml of 30% hydrogen peroxide were added over a 25 minute period and the mixture stirred for one hour, then concentrated. An additional 500 ml of water and 250 ml of ethyl acetate were added which, following workup, gave about 54 g of 2,6-di(3-hydroxypropyl)phenol, m.p. 176-187°C.

30.48 g of said 2,6-di(3-hydroxypropyl)phenol, 20.33 g of hexamethylenetetramine and 220 g of trifluoroacetic acid were heated at reflux temperature for 17 hours after which the mixture was cooled and concentrated. A volume of acetonitrile was added and then stripped and subsequently repeated to provide a residue. The residue was dissolved in 500 ml of ethyl acetate which was then washed with 250 ml of water and four 250 ml portions of a saturated sodium bicarbonate solution. Following workup, about 56 g of the desired subtitled intermediate was obtained.

B. Preparation of 5-{[3,5-bis[3-(acetyloxy)-propyl]-4-hydroxyphenyl]methylene}-3-methyl-2-thioxo-4-thiazolidinone

25 g of the intermediate prepared in Example 63A, 11.2 g of N-methylrhodanine and 19 g of sodium acetate were heated at reflux temperature in 300 ml of acetic acid for 16.5 hours. The mixture, allowed to cool to room temperature for 6 hours, was filtered and then washed with acetic acid. Further workup rendered the subtitled intermediate, m.p. 151-155°C.

C. Preparation of 5-{[3,5-bis[3-(acetyloxy)-propyl]-4-hydroxyphenyl]methylene}-3-methyl-4-thiazolidinone

Utilizing procedures set forth herein, the thione intermediate prepared in Example 63B was reduced using tri-n-butyl tin hydride and AIBN to yield the desired title product, m.p. 112-116°C.

| Analysis for $C_{21}H_{27}NO_6S$: | | | |
|---|---|---|---|
| Calculated: | C, 59.84; | H, 6.46; | N, 3.32; |
| Found: | C, 60.05; | H, 6.58; | N, 3.30. |

Utilizing the procedures set forth in Example 63, and elsewhere herein, the following compounds were prepared.

Example 64

5-{[3,5-bis[3-(acetyloxy)propyl]-4-hydroxyphenyl]methylene}-3-(dimethylamino)-4-thiazolidinone, m.p. 108-110°C

| Analysis for $C_{22}H_{30}N_2O_6S$: | | | |
|---|---|---|---|
| Calculated: | C, 58.65; | H, 6.71; | N, 6.22; |
| Found: | C, 58.80; | H, 6.76; | N, 6.17. |

Example 65

5-{[3-(1,1-dimethylethyl)-4-hydroxy-5-propylphenyl]methylene}-3-methyl-4-thiazolidinone, m.p. 189.5-191.5°C

| Analysis for $C_{18}H_{25}NO_2S$: | | | |
|---|---|---|---|
| Calculated: | C, 67.68; | H, 7.89; | N, 4.38; |
| Found: | C, 67.97; | H, 8.16; | N, 4.40. |

Example 66

5-{[3-(1,1-dimethylethyl)-4-hydroxyphenyl]-methylene}-3-methyl-4-thiazolidinone.

A. Preparation of 3-(1,1-dimethylethyl)-4-hydroxybenzaldehyde

Into 101.5 g of N-methylformanilide were added dropwise over a period of 15 minutes, with cooling, 107 g of phosphoryl chloride. The mixture was allowed to warm to room temperature and stirred for 70 minutes. 67.5 g of ortho-t-butylphenol were added and stirred for about 45 minutes, after which the mixture was heated to about 50-60°C and allowed to stir for 4.5 hours. The reaction mixture was poured into a volume of crushed ice and extracted with chloroform. The aqueous layer was separated and washed with chloroform. The chloroform layers were combined and extracted with 2000 ml of a 5% potassium hydroxide solution. The aqueous potassium hydroxide layer was separated and added to 1000 ml of chloroform. The pH of the resulting two-phase mixture was adjusted to 3 with concentrated hydrochloric acid, while stirring. The resultant layers were then separated The aqueous layer was again extracted with chloroform and dried over sodium sulfate overnight to give 18.1 g of the desired subtitled intermediate.

B. Preparation of 5-{[3-(1,1-dimethylethyl)-4-hydroxyphenyl]methylene}-3-methyl-2-thioxo-4-thiazolidinone

The benzaldehyde intermediate from Example 66A (17.5 g) was dissolved in 490 ml of acetic acid, which solution was then added to a mixture of 14.45 g of N-methylrhodanine and 28.18 g of sodium acetate. The resultant suspension was heated, stirred at reflux temperature for 24 hours (at which time a yellow precipitate had formed), filtered and washed with acetic acid and diethyl ether. The precipitate was triturated with 300 ml of diethyl ether, filtered, washed again with diethyl ether and triturated yet a second time with 600 ml of water. Drying the resultant solid in vacuo yielded the desired subtitled intermediate, m.p. >230°C.

C. Preparation of 5-{[3-(1,1-dimethylethyl)-4-hydroxyphenyl]methylene}-3-methyl-4-thiazolidinone

The thione prepared in Example 66B was reduced as described above utilizing tri-n-butyl tin hydride and AIBN to yield the desired title product, m.p. >230°C.

| Analysis for $C_{15}H_{19}NO_2S$: | | | |
|---|---|---|---|
| Calculated: | C, 64.95; | H, 6.90; | N, 5.05; |
| Found: | C, 65.07; | H, 7.02; | N, 5.28. |

Utilizing the procedures set forth herein, the following additional compounds were prepared.

Example 67

5-{[3,5-bis(1,1-dimethylethyl)-4-hydroxyphenyl]methylene}-2,4-thiazolidinedione, m.p. 234-238°C

| Analysis for $C_{18}H_{23}NO_3S$: | | | |
|---|---|---|---|
| Calculated: | C, 64.83; | H, 6.95; | N, 4.20; |
| Found: | C, 64.77; | H, 6.73; | N, 3.93. |

Example 68

5-[(4-hydroxy-3,5-dimethylphenyl)methylene]-3-methyl-4-thiazolidinone, m.p. 207-212°C (dec.)

| Analysis for $C_{13}H_{15}NO_2S$: | | | |
|---|---|---|---|
| Calculated: | C, 62.62; | H, 6.06; | N, 5.62; |
| Found: | C, 62.58; | H, 6.05; | N, 5.65. |

Example 69

3-{[3,5-bis(1,1-dimethylethyl)-4-hydroxyphenyl]methylene}-2-pyrrolidinone

Under a nitrogen atmosphere, 1.52 ml of 2-pyrrolidinone were added to a solution of 32 ml of 2M magnesium methyl carbonate in DMF and 5.86 g of 3,5-di-tert-butyl-4-hydroxybenzaldehyde. The resultant mixture was stirred at reflux temperature for six days and then allowed to cool. The cooled reaction mixture was poured into 40 g of ice containing 10 ml concentrated hydrochloric acid and then extracted with chloroform. The chloroform layer was collected, filtered and the filtrate was concentrated. The resulting residue was purified by silica gel chromatography and recrystallized from ethyl acetate to give the desired titled product (21% yield), m.p. 216-218°C.

| Analysis for $C_{19}H_{27}NO_2$ : | | | |
|---|---|---|---|
| Calculated: | C, 75.71; | H, 9.03; | N, 6.05; |
| Found: | C, 75.95; | H, 9.08; | N, 4.66. |

Example 70

3-{[3,5-bis(1,1-dimethylethyl)-4-hydroxyphenyl]-methylene}-1-methyl-2-pyrrolidinone

A. Preparation of 3,5-bis(1,1-dimethylethyl)-oxypivaloylquinone methide

Under a nitrogen atmosphere, 8.2 g of 3,5-di-tert-butyl-4-hydroxybenzaldehyde were dissolved in 350 ml of methylene chloride, with stirring. Separately, to a dropping funnel were added an additional 35 ml of methylene chloride followed by 4.74 ml of pivaloyl chloride. To the aldehyde solution were added 3.37 ml of triethylamine, after which dropwise addition of the solution from the dropping funnel was immediately started. The dropwise addition was completed in 5 minutes. The resultant mixture was stirred an additional 10 minutes at room temperature, poured into 350 ml of water, shaken and then separated. The methylene chloride layer was dried with sodium sulfate, filtered, washed with a volume of distilled methylene chloride and evaporated to render the desired subtitled intermediate as a yellow solid.

B. Preparation of 3-{[3,5-bis(1,1-dimethylethyl)-4-hydroxypheny]methylene}-1-methyl-2-pyrrolidinone

39.55 ml of 1.77M n-butyllithium in hexane were added to a cold (0°C) solution of 9.81 ml of diisopropylamine in 210 ml of THF. After stirring for 15 minutes at 0°C, 6.72 ml of N-methyl pyrrolidinone were added. Stirring was continued for 5 minutes at 0°C and the solution was then cooled to -78°C, at which time a solution of the intermediate of Example 70A in 175 ml of THF was added over a 10 minute period. After stirring the solution for one hour at -70°C, the reaction was quenched with 1N hydrochloric acid. The reaction mixture was diluted with additional 1N hydrochloric acid and

ethyl acetate, shaken and then separated. The organic layer was extracted with a saturated sodium bicarbonate solution and then brine. The organic layer was dried with sodium sulfate, filtered and then evaporated to give 18 g of a red/orange solid. 7.99 g of p-toluene sulfonic acid monohydrate were added to a solution of 18 g of the red/orange solid in 350 ml of toluene and the resultant solution was stirred at room temperature for 24 hours. The reaction was filtered and evaporated to give 9.1 g of crude product which was chromatographed on silica gel using a gradient of 10-35% acetone in hexane. Those fractions containing purified product were combined and evaporated to give 3.9 g of the desired titled product, m.p. 187-188.5°C.

| Analysis for $C_{20}H_{29}NO_2$: | | | |
|---|---|---|---|
| Calculated: | C, 76.15; | H, 9.27; | N, 4.44; |
| Found: | C, 76.36; | H, 9.20; | N, 4.47. |

Example 71

5-{[3,5-bis(1,1-dimethylethyl)-4-hydroxyphenyl]methylene}-2-hydroxy-3-methyl-4-thiazolidinone

12.56 g of 5-{[3,5-bis(1,1-dimethylethyl)-4-hydroxyphenyl]methylene}-3-methyl-4-thiazolidinone, 1-oxide (the compound of Example 12) were dissolved in 216 ml of methylene chloride and the resultant solution cooled to -78°C. Separately, 6.1 ml of trifluoroacetic anhydride and 72 ml of methylene chloride were placed in a dropping funnel and the solution added dropwise, over a 40-minute period (temperature maintained at or below -70°C), to the previously prepared solution of the compound of Example 12 in methylene chloride. The resulting reaction mixture was stirred at -75°C for 1 hour, then warmed to 0°C over 45 minutes, diluted with a volume of methylene chloride, washed with two volumes of water, dried over sodium sulfate and subsequently filtered and evaporated to give 15.8 g of the desired titled product and trace impurities. This product was dissolved in 25 ml of warm ethyl acetate and then added to 450 ml of hexane. As the solution cooled, it became milky and an additional 5 ml of ethyl acetate were added. The solution was allowed to cool to room temperature overnight while precipitate formed. The precipitate was collected by filtration, washed with hexane and dried at room temperature in vacuo overnight. The resultant product was further worked up by adding it to 30 ml of hot ethyl acetate, to which was added an additional 150 ml of hexane. A precipitate began to form. The mixture was allowed to cool to room temperature and stand for 6 hours, after which it was filtered, washed with a volume of hexane and dried in vacuo at 50°C overnight to render 8.83 g of the desired titled product, m.p. 165-170°C.

| Analysis for $C_{19}H_{27}NO_3S$: | | | |
|---|---|---|---|
| Calculated: | C, 65.30; | H, 7.79; | N, 4.01; |
| Found: | C, 65.50; | H, 7.80; | N, 4.02. |

As noted previously, the compounds of the present invention are physiologically active as demonstrated in the following test systems.

Carrageenin Assay

The compounds were evaluated for antiinflammatory activity in the test method described by C.A. Winter, Proc. Soc. Exp. Biol. Med., 111, 544 (1962). In this test, inflammation is created by injecting carrageenin into the hind paws of rats. Test compounds are administered prior to injection to determine percent inhibition of the subsequent inflammation in comparison with control animals. The results are reported in Table I.

Table I

| Antiinflammatory Activity in the Carrageenin Assay | | |
|---|---|---|
| Compound of Example No. | Dose mg/kg* | Percent Inhibition |
| 1 | 30 | 67% |
| 2 | 50 | 46% |
| 3 | 50 | 12% |
| 4 | 50 | 65% |

* orally by gavage

Table I (continued)

| Antiinflammatory Activity in the Carrageenin Assay | | |
|---|---|---|
| Compound of Example No. | Dose mg/kg* | Percent Inhibition |
| 14 | 50 | 43% |
| 15 | 50 | 42% |
| 16 | 50 | 40% |
| 17 | 50 | 18% |
| 18 | 50 | 22% |

* orally by gavage

Collagen-Induced Arthritis Assay

Type II collagen was isolated from bovine articular cartilage by the method of Strawich and Nimni [Biochemistry, 10, 3905 (1971)]. The collagen was dissolved in 0.1 M acetic acid and stored at -20°C. Type II collagen solution was diluted to 2 mg/ml concentration and emulsified thoroughly with an equal volume of incomplete Freund's adjuvant (ICFA). The emulsion containing approximately 0.5 mg of collagen was injected intradermally on day 0 to groups of 6 inbred Lewis male rats (Charles River Breeders; 170-200 g) at various sites in the dorsal area. The hindpaw volumes of each rat were measured and recorded three times a week throughout the test period to assess the inflammatory reaction. The test group animals received compounds under test as suspensions in carboxymethylcellulose vehicle, by oral gavage, 5 days per week (Monday-Friday), beginning on day 1. Control animals received vehicle without a test compound. At the end of the test (day 28 or 30), the blood of these animals was drawn by cardiac puncture and the serum anti-type II collagen antibody levels were estimated by passive hemaglutination technique, using glutaraldehyde treated sheep red cells, to which type II collagen is conjugated [Avrameas et al., Immunochemistry, 6, 67 (1969); Andriopoulos et al., Arth. Rheum., 19, 613 (1976)]. The cellular response or delayed-type hypersensitivity response to type II collagen was measured by the radiometric ear index assay [Kostiala, Immunology, 33, 561 (1977)]. In certain experiments, the bone damage occurring because of immunization with type II collagen and the effects of drugs were determined from the radiographs of the hind-paws of two or three representative animals from each group. Injections of ICFA without collagen II were employed in some rats as a negative control; these rats received only carboxymethycellulose vehicle during the test.

The results of testing the compounds of the present invention in the collagen-induced arthritis system are summarized in Table II. The % inhibition was calculated according to the following formula:

$$\% \text{ inhibition} = [1-\frac{Vt - Vv}{Vc - Vv}] \text{ X } 100$$

where Vt is the hindpaw volume of a compound-treated animal (test group), Vc is the hindpaw volume of a non-compound-treated animal (carboxymethylcellulose vehicle only-the control group), and Vv is the hindpaw volume of a vehicle (carboxymethylcellulose) treated animal which received ICFA with no collagen II (negative control group).

Table II

| Inhibition of Collagen-Induced Arthritis | | |
|---|---|---|
| Compond of Example No. | Dose mg/kg* | % inhibition* |
| 1 | 50 | 100% |
| | 50 | 53% |
| 2 | 30 | 91% |
| | 50 | 100% |
| | 30 | 50% |
| 3 | 50 | 79% |
| 4 | 50 | 5% |

* See text for experimental method.

Developing Adjuvant-Induced Arthritis Test in Rats

Compounds were tested for their ability to alter hind paw swelling and bone damage resulting from adjuvant-induced edema in rats. In order to quantitate the inhibition of hind paw swelling resulting from adjuvant-induced arthritis, two phases of inflammation have been defined: (1) the primary and secondary injected hind paw, and (2) the secondary

uninjected hind paw, which generally begins developing about eleven days from the induction of inflammation in the injected paw. Reduction of the latter type of inflammation is an indication of immunosuppressive activity. Cf. Chang, Arth. Rheum., 20, 1135-1141 (1977).

Adjuvant arthritis was induced in male Lewis-Wistar rats (200-210 grams) by a single subplantar injection into the right hind paw of 0.1 ml of a 0.5% suspension of heat-killed, lyophilized Mycobacterium tuberculosis (Calbiochem-Perrigen-C) in mineral oil (a modification of a method reported by Winter et al., Arth. Rheum., 9, 394-397 (1966)). One group of 10 rats ("TB control") received only this treatment. Another group of 5 rats received no treatment (normal control). Each compound to be tested was suspended in carboxymethylcellulose (1%) and administered p.o. to rats (groups of 5 each) in daily doses of 50 mg/kg beginning on day one and continuing through the 28th day after the adjuvant injection (29 doses). Paw volumes were measured by mercury displacement using a Statham pressure transducer and digital voltmeter. Volumes of both the injected and the uninjected hind paws were measured on days 16, 18, 21, 23, 25, 28, and 30. X-ray photos were taken on day 30, after the animals were sacrificed. The paw volume measurements on the uninjected paw beginning with day 16 through day 30 were computer plotted for the TB controls, the normal controls, and the drug-treated animals, and the areas under the curves [(TB controls minus normal controls) and (treated animals minus normal controls)] were determined. The results are summarized in Table III.

## Table III

### Inhibition of Uninjected Paw Volume Inflammation

### Days 16 through 30

| Compound of Example No. | Dose mg/kg P.O. x 29 | % Inhibition* |
|---|---|---|
| 1 | 50 | 41 |
| 2 | 50 | 81 |
| 3 | 50 | 10 |
| 4 | 50 | 23 |
| 5 | 50 | 7 |
| 6 | 50 | -- |
| 7 | 50 | 30 |
| 8 | 50 | 4 |
| 9 | 50 | -- |
| 10 | 50 | 57 |
| 11 | 50 | +19.7 |
| 12 | 50 | +4.8 |
| 13 | 50 | +8.7 |
| 14 | 50 | 6 |
| 15 | 50 | 40 |
| 16 | 50 | 18 |
| 17 | 50 | 61 |
| 18 | 50 | -- |
| 19 | 50 | +20.7 |
| 20 | 50 | 36 |
| 21 | 50 | 81 |
| 22 | 50 | 1 |
| 23 | 50 | 28 |
| 24 | 50 | 0 |
| 25 | 50 | +8.5 |
| 26 | 50 | 30.4 |
| 27 | 50 | 40 |
| 28 | 50 | 72 |
| 29 | 50 | 49.0 |
| 30 | 50 | 44.1 |
| 31 | 50 | 28.9 |
| 32 | 50 | 48.6 |
| 33 | 50 | 30.1 |
| 34 | 50 | +1.6 |
| 35 | 75 | 86 |

## Table III (continued)

| Compound of Example No. | Dose mg/kg P.O. x 29 | % Inhibition* |
|---|---|---|
| 36 | 50 | -- |
| 37 | 50 | 3~~1~~7.6 |
| 38 | 50 | 1.8 |
| 39 | 50 | 2 |
| 40 | 50 | 23 |
| 41 | 50 | 27 |
| 42 | 50 | -- |
| 43 | 50 | 36 |
| 44 | 50 | 15 |
| 45 | 50 | 36 |
| 46 | 50 | 9 |
| 47 | 50 | 69.3 |
| 47 | 25 | 57.7 |
| 49 | 50 | 36 |
| 50 | 50 | 62 |
| 51 | 25 | 39 |
| 52 | 25 | 45 |
| 54 | 50 | 37.6 |
| 55 | 50 | 96.2 |
| 56 | 50 | 38.2 |
| 57 | 50 | 44.1 |
| 58 | 50 | 16.9 |
| 59 | 50 | 19.8 |
| 60 | 50 | 40.6 |
| 61 | 50 | 5.3 |
| 62 | 50 | 11.2 |
| 63 | 50 | 9.8 |
| 64 | 50 | 5.5 |
| 66 | 50 | 26.1 |
| 68 | 50 | 6.5 |
| 71 | 50 | 48.2 |

*% inhibition is the difference of the areas under the curves (AUC) of the mean uninjected paw volumes plotted for days 16, 18, 21, 23, 25, 28 and 30 according to the following formula:

$$\%\ \text{inhibition} = \left[1 - \frac{(\text{Drug treated AUC})-(\text{normal control AUC})}{(\text{TB control AUC})-(\text{normal control AUC})}\right] \times 100$$

Compounds of Formula II have also been shown to prevent ischemia-induced neuronal cell damage as demonstrated in the following test system.

Stroke model in rats

Strokes were produced in rats by occluding the four arteries that supply blood to the brain according to the following procedure. Male Wistar rats were anesthetized with Metofane and placed into a stereotaxic instrument. A longitudinal incision was made on the dorsal surface of the neck. The neck muscles were reflected to expose the dorsal surface

of the spinal column. The two vertebral arteries were exposed where they pass through the first cervical vertebra. Both arteries were permanently occluded by the application of electrocautery. After coagulation of the vertebral arteries, the rat was removed from the stereotaxic instrument and the surgical wound was sutured. Two longitudinal incisions were then made on the ventral surface of the neck. The two common carotid arteries were exposed and dissected free from surrounding nerves and connective tissue. An atraumatic clasp, fabricated mainly from silicon rubber tubing, was placed around each carotid artery in a manner such that the vessel was not traumatized or occluded. The surgical wounds were then closed. The atraumatic clasps were designed in such a manner that they could be tightened to occlude the carotid arteries by pulling on a small silastic thread that was allowed to protrude from the wound. Circulation to the brain through the carotids could be restored by relieving the tension on the silastic threads. After the surgery, the rats were allowed to recover for 24 hours.

On the day of testing, compounds were suspended in 2% acacia and were administered orally at various times before stroke induction. Strokes (cerebral ischemia) were induced by tightening the clasps around the carotids for a period of 30 minutes. During this time, rats in which strokes had successfully been produced lost the righting reflex and became unresponsive to stimuli. After 30 minutes of ischemia, tension on the clasps was removed and blood flow to the brain restored. Rats were again treated with compounds on the morning after the stroke. On the third day after the stroke the animals received an overdose of barbituate anesthetic, and the brain was perfused in situ with 10% neutral, buffered formalin. After perfusion with amounts of formalin adequate to fix the brain, the brains were removed and stored in 10% formalin until histological sections could be prepared.

One of the areas of the brain that is most susceptible to ischemia induced damage both in the rat and the human is the $CA_1$ pyramidal cell layer of the hippocampus. In animals that remain unresponsive for the 30 minute period of ischemia, the $CA_1$ pyramidal cell layer is completely destroyed. This layer of cells was examined microscopically in histological sections prepared from the hippocampus. Brain damage was rated according to the following scale:

0 = no damage, completely intact cell layer
1 = mild damage, one-third of $CA_1$ layer dead
2 = moderate damage, two-thirds of $CA_1$ layer dead
3 = severe damage, complete destruction of $CA_1$ layer

Damage in 10-12 sections from each brain was assessed in order to obtain an accurate estimate of damage. An average damage score was calculated for each treatment group. Scores from treated groups were compared statistically with scores from control groups which received only the vehicle (2% acacia) that was used to suspend the compounds. The level of significance was determined using Student's "t-test". Results are summarized in Table IV.

## Table IV

### Prevention of ischemia-induced brain damage in the hippocampal $CA_1$ region in rats

| Treatment | Dose* | No. of Rats | Damage Score** |
|---|---|---|---|
| Vehicle control | - | 6 | 2.5 ± 0.2 |
| Example 2 | 50 | 10 | 1.2 ± 0.2 ($p<0.02$) |
| Vehicle control | - | 4 | 2.3 ± 0.8 |
| Example 2 | 200 | 6 | 0.2 ± 0.2 ($p<0.02$) |
| Vehicle control | - | 10 | 2.5 ± 0.2 |
| Example 2 | 500 | 7 | 0.07 ± 0.007 ($p<0.001$) |
| Vehicle control | - | 3 | 2.8 ± 0.2 |
| Example 2 | 500 | 4 | 0.0 ($p<0.001$) |
| Vehicle control | - | 6 | 2.8 ± 0.2 |
| Example 5 | 100 | 8 | 1.8 ± 0.4 ($p=0.05$) |
| Vehicle control | - | 8 | 2.8 ± 0.1 |
| Example 6 | 100 | 11 | 2.5 ± 0.2 |
| Vehicle control | - | 10 | 2.5 ± 0.3 |
| Example 7 | 100 | 9 | 2.5 ± 0.2 |
| Vehicle control | - | 4 | 2.5 ± 0.26 |
| Example 10*** | 100 | 4 | 0.75 ± 0.26 ($p<0.02$) |
| Vehicle control | - | 8 | 2.7 ± 0.1 |
| Example 11 | 100 | 5 | 2.1 ± 0.3 |
| Vehicle control | - | 8 | 2.2 ± 0.3 |
| Example 12 | 100 | 8 | 1.8 ± 0.4 |
| Vehicle control | - | 12 | 2.2 ± 0.3 |
| Example 13 | 100 | 12 | 2.1 ± 0.3 |
| Vehicle control | - | 10 | 2.3 ± 0.4 |
| Example 21 | 50 | 11 | 1.9 ± 0.3 |
| Vehicle control | - | 10 | 2.7 ± 0.2 |
| Example 22 | 100 | 10 | 2.3 ± 0.3 |

Table IV (continued)

| Treatment | Dose* | No. of Rats | •Damage Score** |
|---|---|---|---|
| Vehicle control | - | 10 | 2.7 ± 0.2 |
| Example 23 | 100 | 9 | 2.2 ± 0.4 |
| Vehicle control | - | 8 | 2.7 ± 0.1 |
| Example 26 | 100 | 8 | 1.9 ± 0.4 |
| Vehicle control | - | 9 | 2.4 ± 0.3 |
| Example 28 | 50 | 9 | 1.0 ± 0.4 (p=0.009) |
| Vehicle control | - | 8 | 2.2 ± 0.3 |
| Example 35 | 100 | 9 | 0.9 ± 0.4 (p<0.001) |
| Vehicle control | - | 11 | 2.3 ± 0.3 |
| Example 36 | 100 | 7 | 2.3 ± 0.4 |
| Vehicle control | - | 8 | 2.7 ± 0.4 |
| Example 38 | 200 | 11 | 1.6 ± 0.04 |
| Vehicle control | - | 10 | 2.6 ± 0.2 |
| Example 61 | 50 | 11 | 1.7 ± 0.3 (p=0.03) |
| Vehicle control | - | 9 | 2.4 ± 0.3 |
| Example 62 | 100 | 10 | 1.2 ± 0.3 (p<0.05) |
| Vehicle control | - | 10 | 2.5 ± 0.3 |
| Example 67 | 100 | 10 | 1.4 ± 0.3 (p<0.05) |
| Vehicle control | - | 10 | 2.28 ± 0.2 |
| Example 69 | 50 | 8 | 0.98 ± 0.42 (p=0.037) |
| Vehicle control | - | 8 | 2.2 ± 0.4 |
| Example 70 | 100 | 6 | 0.7 ± 0.4 (p=0.039) |

*mg/kg given orally as a suspension in 2% acacia

**mean ± standard error

***Three rats showed no damage, one rat died and was revived; p<0.02.

Compounds of Formula III have also been shown to prolong the lifespan of dystrophic mammals as demonstrated in the following test system.

Muscular Dystrophy Animal Model

Dystrophic mice (dy/dy) were obtained from Jackson Laboratories after weaning (approximately 21 days) and treatment with the compounds shown in Table V was begun at the first sign of dystrophy. The compounds that were tested were administered in the diet and lifespan was measured during the course of treatment. The food and water sources were located in different parts of the cage requiring the animals to walk from the food source to the water

source to survive. The results are shown in Table V.

Table V

Life Span Measurements of Dystrophic Mice

| Treatment | No. of Mice | Concentration[a] | Average Life Span[b] |
|---|---|---|---|
| Control | 6 | | 86 |
| Example 2 | 6 | 0.3 | 74 |
| Control | 6 | | 46 |
| Example 2 | 6 | 0.1 | 108 |
| Control | 5 | | 38 |
| Example 2 | 5 | 0.08 | 73 |
| Control | 5 | | 56 |
| Example 2 | 5 | 0.08 | 116 |
| Control | 6 | | 55 |
| Example 2 | 6 | 0.03 | 57 |

Table V (continued)

| Treatment | No. of Mice | Concentration[a] | Average Life Span[b] |
|---|---|---|---|
| Control | 6 | | 86 |
| Example 10 | 6 | 0.3 | 83 |
| Control | 8 | | 54 |
| Example 10 | 8 | 0.08 | 45 |
| Control | 7 | | 62 |
| Example 10 | 7 | 0.08 | 67 |
| Control | 6 | | 55 |
| Example 10 | 6 | 0.03 | 110 |
| Control | 6 | | 58 |
| Example 10 | 11 | 0.03 | 93 |
| Control | 4 | | 83 |
| Example 10 | 4 | 0.03 | 112 |
| Control | 8 | | 54 |
| Example 10 | 8 | 0.03 | 90 |
| Control | 6 | | 43 |
| Example 10 | 6 | 0.03 | 80 |
| Control | 7 | | 83 |
| Example 17 | 8 | 0.03 | 112 |
| Control | 6 | | 94 |
| Example 21 | 6 | 0.03 | 151 |
| Control | 7 | | 73 |
| Example 35 | 7 | 0.05 | 107 |

[a]Concentration (percent by weight) of compound tested in diet

[b]Expressed in days

As noted above, the compounds of the present invention are physiologically active thereby lending themselves to valuable therapeutic methods as claimed herein. The methods include administering to a mammal in need thereof an effective amount of one or more compounds of the present invention sufficient for the therapeutic or prophylactic intervention desired. Such administration is accomplished by means of pharmaceutical compositions which are prepared by techniques well known in the pharmaceutical sciences. Accordingly, the present invention is also directed to pharmaceutical compositions which include at least one compound of formula I in association with one or more pharmaceutically acceptable diluents, excipients or carriers.

In making the pharmaceutical compositions of the present invention, one or more active ingredients will usually be mixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the compositions can be in the form of

tablets, pills, powders, lozenges, sachets, cachets, elixirs, suspensions, emulsions, solutions, syrups, aerosols (as a solid or in a liquid medium), ointments containing for example up to 10% by weight of the active compound, soft and hard gelatin capsules, suppositories, sterile injectable solutions and sterile packaged powders.

Some examples of suitable carriers, excipients, and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, methylcellulose, methyl - and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide rapid, sustained or delayed release of the active ingredient after administration to the patient by employing procedures well known in the art.

The compositions are formulated, preferably in a unit dosage form, such that each dosage contains from about 5 to about 500 mg, more usually about 25 to about 300 mg, of the active ingredient. The term "unit dosage form" refers to physically discrete units suitable as unitary dosages for human subjects and other mammals, each unit containing a predetermined quantity of active material calculated to produce the desired therapeutic effect, in association with one or more suitable pharmaceutical diluents, excipients or carriers.

The compounds of the present invention are effective over a wide dosage range for the indications for which they are administered. Thus, as used herein, the term "effective amount" refers to a dosage range of from about 0.5 to about 200 mg/kg of body weight per day. In the treatment of adult humans, the range of about 1 to about 50 mg/kg, in single or divided doses, is preferred. However, it will be understood that the amount of the compound actually administered will be determined by a physician, in the light of the relevant circumstances including the condition to be treated, the choice of compound to be administered, the chosen route of administration, the age, weight, and response of the individual patient, and the severity of the patient's symptoms, and therefore the above dosage ranges are not intended to limit the scope of the invention in any way.

The following formulation examples may employ as active ingredients any of the compounds of formula I. The examples are illustrative only and are not intended to limit the scope of the invention in any way.

Example 72

Hard gelatin capsules are prepared using the following ingredients:

| | Quantity (mg/capsule) |
|---|---|
| Compound of Example 55 | 250 |
| Starch dried | 200 |
| Magnesium stearate | 10 |

The above ingredients are mixed and filled into hard gelatin capsules in 460 mg quantities.

Example 73

A tablet formula is prepared using the ingredients below:

| | Quantity (mg/tablet) |
|---|---|
| Compound of Example 21 | 250 |
| Cellulose, microcrystalline | 400 |
| Silicon dioxide, fumed | 10 |
| Stearic acid | 5 |

The components are blended and compressed to form tablets each weighing 665 mg.

Example 74

An aerosol solution is prepared containing the following components:

| | Weight % |
|---|---|
| Compound of Example 50 | 0.25 |

(continued)

| | Weight % |
|---|---|
| Ethanol | 29.75 |
| Propellant 22 (Chlorodifluoromethane) | 70.00 |

The active compound is mixed with ethanol and the mixture added to a portion of the propellant 22, cooled to -30°C and transferred to a filling device. The required amount is then fed to a stainless steel container and diluted with the remainder of the propellant. The valve units are then fitted to the container.

Example 75

Tablets each containing 60 mg of active ingredient are made up as follows:

| | |
|---|---|
| Compound of Example 28 | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50-60°C and passed through a No. 18 mesh U.S. sieve. The sodium carboxymethyl starch, magnesium stearate and talc, previously passed through a No. 60 mesh U.S. sieve, are then added to the granules which, after mixing, are compressed by a tablet machine to yield tablets each weighing 150 mg.

Example 76

Capsules each containing 80 mg of medicament are made as follows:

| | |
|---|---|
| Compound of Example 62 | 80 mg |
| Starch | 59 mg |
| Microcrystalline cellulose | 59 mg |
| Magnesium stearate | 2 mg |
| Total | 200 mg |

The active ingredient, cellulose, starch and magnesium stearate are blended, passed through a No. 45 mesh U. S. sieve, and filled into hard gelatin capsules in 200 mg quantities.

Example 77

Suppositories each containing 225 mg of active ingredient are made as follows:

| | |
|---|---|
| Compound of Example 35 | 225 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

Example 78

Suspensions each containing 50 mg of medicament per 5 ml dose are made as follows:

| | |
|---|---|
| Compound of Example 62 | 50 mg |
| Sodium carboxymethylcellulose | 50 mg |
| Syrup | 1.25 ml |
| Benzoic acid solution | 0.10 ml |
| Flavor | q.v. |
| Color | q.v. |
| Purified water to | 5 ml |

The medicament is passed through a No. 45 mesh U.S. sieve and mixed with the sodium carboxymethyl cellulose and syrup to form a smooth paste. The benzoic acid solution, flavor and color are diluted with some of the water and added, with stirring. Sufficient water is then added to produce the required volume.

Example 79

Capsules each containing 150 mg of medicament are made as follows:

| | |
|---|---|
| Compound of Example 47 | 150 mg |
| Starch | 164 mg |
| Microcrystalline cellulose | 164 mg |
| Magnesium stearate | 22 mg |
| Total | 500 mg |

The active ingredient, cellulose, starch and magnesium stearate are blended, passed through a No. 45 mesh U. S. sieve, and filled into hard gelatin capsules in 500 mg quantities.

## Claims

**Claims for the following Contracting States : AT, BE, CH, DE, DK, FR, GB, IT, LI, NL, SE**

**1.** A compound, and the pharmaceutically acceptable salts thereof, of the formula (I):

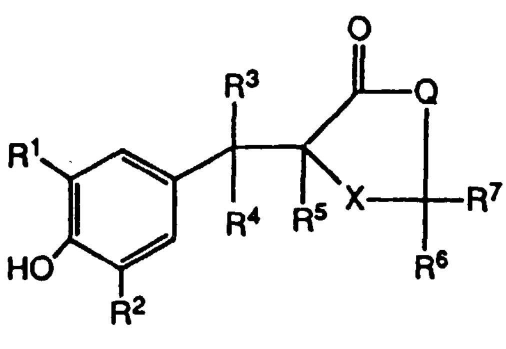

(I)

wherein:

$R^1$ and $R^2$ are each independently hydrogen, $C_1$-$C_6$ alkyl, $C_1$-$C_6$ alkoxy or

$$-C_1-C_4\ alkyl-O-\overset{O}{\overset{\|}{C}}-(C_1-C_4\ alkyl);$$

$R^3$ is is hydrogen or $C_1$-$C_6$ alkyl;

$R^4$ and $R^5$ are each hydrogen, or when taken together form a bond;
$R^6$ and $R^7$ are each hydrogen or when taken together are =S, or when one of $R^6$ or $R^7$ is hydrogen, the other is -OH or $-SCH_3$;
X is

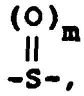

where m is 0, 1 or 2; and
Q is $-CH_2-$, -O- or $NR^8$ where $R^8$ is is hydrogen, $C_1$-$C_6$ alkyl, $C_3$-$C_8$ cycloalkyl, $C_2$-$C_6$ alkenyl, $-SO_2CH_3$ or $-(CH_2)_n$-Y where n is an integer from 0 to 3, both inclusive, and Y is cyano, $OR^9$,

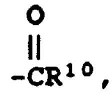

tetrazolyl, $-NR^{11}R^{12}$, -SH, -S($C_1$-$C_4$ alkyl) or

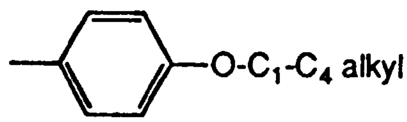

where $R^9$ is hydrogen, $C_1$-$C_4$ alkyl, tosyl or

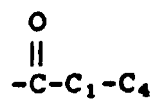

alkyl; $R^{10}$ is $C_1$-$C_4$ alkyl, $C_1$-$C_4$ alkoxy or $-NH_2$; $R^{11}$ and $R^{12}$ are each independently hydrogen, $C_1$-$C_6$ alkyl, $C_2$-$C_6$ alkenyl, $C_2$-$C_6$ alkynyl, $-(CH_2)_qOH$, $-(CH_2)_q$-N($C_1$-$C_4$ alkyl)$_2$, $-(CH_2)_q$-S($C_1$-$C_4$ alkyl) or

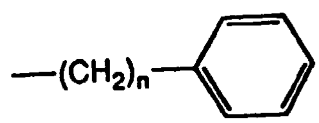

where q is an integer from 1 to 6, both inclusive, and n is as defined above; or $R^{11}$ and $R^{12}$ taken together form a morpholinyl, piperidinyl, piperazinyl or an N-methylpiperazinyl ring;
with the proviso that when Q is -O- or $NR^8$ ($R^8$ is hydrogen or $C_1$-$C_4$ alkyl), $R^3$ is hydrogen, $R^4$ and $R^5$ are each hydrogen or when taken together form a bond, $R^6$ and $R^7$ are each hydrogen or when taken together are =S and X is

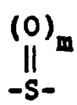

(where m is 0), $R^1$ and $R^2$ cannot both be a t-butyl group;
and when Q is $NR^8$ ($R^8$ is hydrogen), $R^3$ is hydrogen, $R^6$ and $R^7$ taken together are =S and X is $-S(O)_m-$ (where m is 0), $R^4$ and $R^5$ must both be hydrogen;
and when Q is $NR^8$ [$R^8$ is hydrogen or $-(CH_2)_n$-Y, where n is 0 and Y is CO($C_1$-$C_4$ alkyl)], $R^3$ is hydrogen, $R^4$ and $R^5$ taken together form a bond, $R^6$ and $R^7$ are each hydrogen and X is $-S(O)_m-$ (where m is 0), $R^1$ and $R^2$ cannot both be an isopropyl group.

**2.** A compound of Claim 1 wherein $R^1$ and $R^2$ are each $C_1$-$C_6$ alkyl; $R^3$ is hydrogen; $R^4$ and $R^5$ taken together form a bond; $R^6$ and $R^7$ are each hydrogen; X is

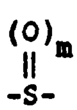

where m is 0; and Q is -O- or $NR^8$, where $R^8$ is as defined in Claim 1.

3. A compound of Claim 2 wherein $R^1$ and $R^2$ are each $C_1$-$C_4$ alkyl; $R^3$ is hydrogen; $R^4$ and $R^5$ taken together form a bond; $R^6$ and $R^7$ are each hydrogen; X is

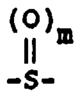

where m is 0; and Q is $NR^8$, where $R^8$ is hydrogen, $C_1$-$C_6$ alkyl or $-(CH_2)_n$-Y, where n and Y are as defined in Claim 1.

4. A compound of the formula I as defined in any one of Claims 1, 2 or 3 for use in treating inflammation and arthritis in mammals.

5. A pharmaceutical composition comprising as an active ingredient a compound of the formula (I) as claimed in any one of Claims 1, 2 or 3 associated with one or more pharmaceutically acceptable diluents, excipients or carriers therefor.

6. Use of a compound of the formula (II)

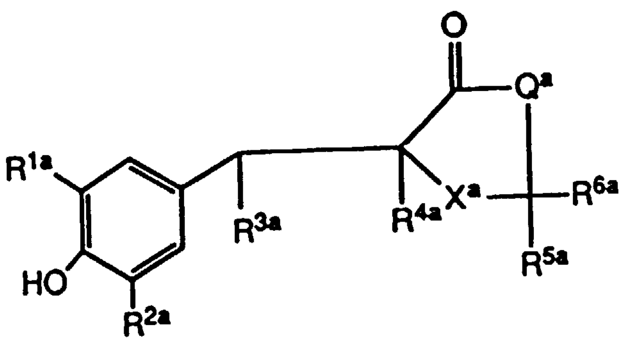

(II)

wherein:

$R^{1a}$ and $R^{2a}$ are each independently $C_1$-$C_6$ alkyl;
$R^{3a}$ and $R^{4a}$ are each hydrogen or when taken together form a bond;
$R^{5a}$ and $R^{6a}$ are each hydrogen or when taken together are =O; and
$X^a$ is $-CH_2$- or

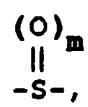

where m is 0, 1 or 2;
$Q^a$ is $NR^{7a}$;
$R^{7a}$ is hydrogen, $C_1$-$C_6$, alkyl, or $-(CH_2)_n$-$Y^a$, where n is an integer from 0 to 3, both inclusive, and $Y^a$ is cyano, $OR^{8a}$,

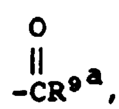

-SH, -S($C_1$-$C_4$ alkyl), tetrazolyl, $-NR^{10a}R^{11a}$ or

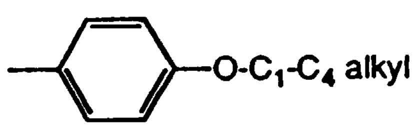

where $R^{8a}$ is hydrogen, $C_1$-$C_4$ alkyl, or

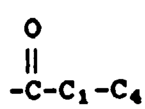

alkyl; $R^{9a}$ is $-NH_2$ or -OH; and $R^{10a}$ and $R^{11a}$ are each independently hydrogen, $C_1$-$C_6$ alkyl, $C_2$-$C_6$ alkenyl or

$C_2$-$C_6$ alkynyl; with the proviso that when $R^{3a}$ and $R^{4a}$ taken together form a bond, $R^{5a}$ and $R^{6a}$ are each hydrogen, $X^a$ is

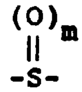

(where m is 0) and $R^{7a}$ is hydrogen or $C_1$-$C_4$ alkyl, $R^{1a}$ and $R^{2a}$ cannot both be a t-butyl group, for the preparation of a medicament for preventing ischemia-induced cell damage in mammals.

**7.** A compound of the formula (II)

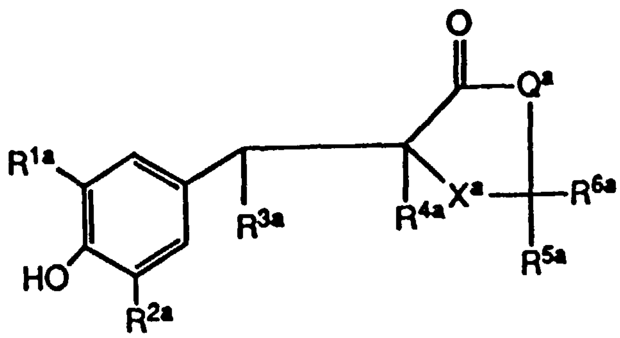

(II)

wherein:

$R^{1a}$ and $R^{2a}$ are each independently $C_1$-$C_6$ alkyl;
$R^{3a}$ and $R^{4a}$ are each hydrogen or when taken together form a bond;
$R^{5a}$ and $R^{6a}$ are each hydrogen or when taken together are =O; and
$X^a$ is $-CH_2-$ or

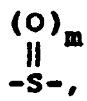

where m is 0, 1 or 2;
$Q^a$ is $NR^{7a}$;
$R^{7a}$ is hydrogen, $C_1$-$C_6$ alkyl, or $-(CH_2)_n-Y^a$, where n is an integer from 0 to 3, both inclusive, and $Y^a$ is cyano, $OR^{8a}$,

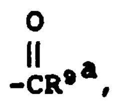

-SH, -S($C_1$-$C_4$ alkyl), tetrazolyl, $-NR^{10a}R^{11a}$ or

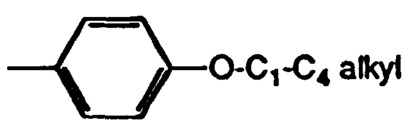

where $R^{8a}$ is hydrogen, $C_1$-$C_4$ alkyl, or

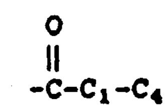

alkyl; $R^{9a}$ is $-NH_2$ or -OH; and $R^{10a}$ and $R^{11a}$ are each independently hydrogen, $C_1$-$C_6$ alkyl, $C_2$-$C_6$, alkenyl or $C_2$-$C_6$ alkynyl; with the proviso that when $R^{3a}$ and $R^{4a}$ taken together form a bond, $R^{5a}$ and $R^{6a}$ are each hydrogen or when taken together are =O, $X^a$ is

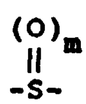

(where m is 0) and $R^{7a}$ is hydrogen or $C_1$-$C_4$ alkyl, $R^{1a}$ and $R^{2a}$ cannot both be an isopropyl or a t-butyl group, for use in preventing ischemia-induced cell damage in mammals.

**8.** A compound of the formula (III)

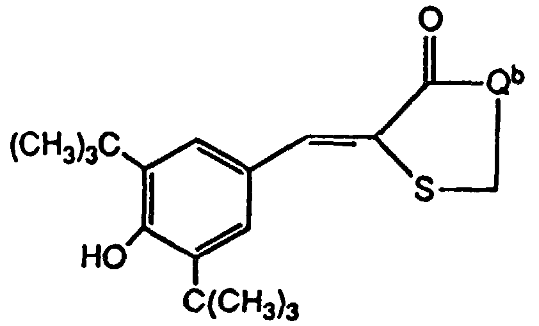

(III)

wherein:

$Q^b$ is -O- or $NR^{7b}$, where $R^{7b}$ is hydrogen, $C_1$-$C_6$ alkyl, $NR^{8b}R^{9b}$ or $-(CH_2)_n$-OH, where $R^{8b}$ and $R^{9b}$ are each independently hydrogen or $C_1$-$C_4$ alkyl and n is an integer from 0 to 3, both inclusive for use in treating a dystrophic mammal.

**9.** A process for preparing a compound of formula (I) as claimed in any of Claims 1, 2 or 3 which comprises:

(A) reacting a compound of the formula

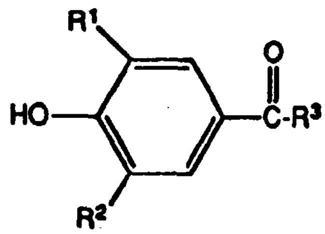

with a compound of the formula

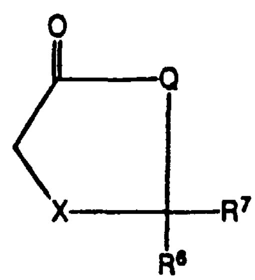

wherein $R^1$, $R^2$, $R^3$ and X are as in formula I, Q is $-CH_2$- or $NR^8$ (where $R^8$ is as defined in formula I) and $R^6$ and $R^7$ taken together are =S, so as to provide a compound of the formula

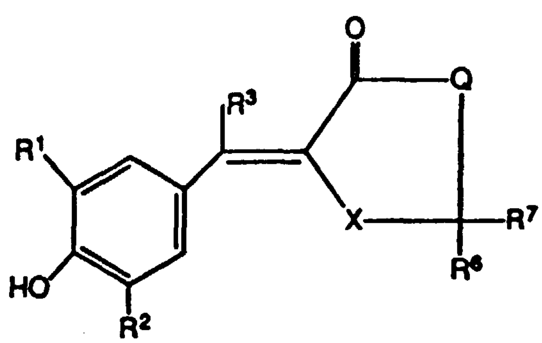

wherein $R^1$, $R^2$, $R^3$, $R^6$, $R^7$, X and Q are as set forth above;
(B) reducing a compound of formula I wherein $R^6$ and $R^7$ taken together are =S so as to prepare a compound of formula I in which $R^6$ and $R^7$ are hydrogen;
(C) reducing a compound of formula I in which $R^4$ and $R^5$ taken together form a bond so as to prepare a compound of formula I in which $R^4$ and $R^5$ are hydrogen;
(D) reducing a compound of formula I in which $R^4$ and $R^5$ taken together form a bond and $R^6$ and $R^7$ taken together are =S so as to prepare a compound of formula I in which $R^4$, $R^5$, $R^6$ and $R^7$ are all hydrogen;
(E) alkylating a compound of formula I in which $R^8$ is hydrogen so as to prepare a compound of formula I in which $R^8$ is $C_1$-$C_6$ alkyl, $C_2$-$C_6$ alkenyl, $C_3$-$C_8$ cycloalkyl or $-(CH_2)_n$-Y (where n is an integer from 0 to 3, both inclusive, and Y is cyano, $OR^9$, -SH, -S($C_1$-$C_4$ alkyl), $-NR^{11}R^{12}$ or

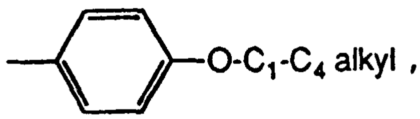

where $R^9$, $R^{11}$ and $R^{12}$ are as defined in formula I);
(F) acylating a compound of formula I in which $R^8$ is hydrogen so as to prepare a compound of formula I in which $R^8$ is $-(CH_2)_n$-Y where n is an integer from 0 to 3, both inclusive, and Y is

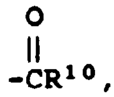

where $R^{10}$ is as defined in formula I;
(G) oxidizing a compound of formula I wherein X is

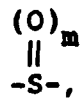

where m is 0, so as to prepare a compound of formula I wherein X is

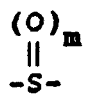

and m is 1;
(H) oxidizing a compound of formula I wherein X is

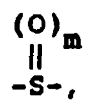

where m is 0, so as to prepare a compound of formula I wherein X is

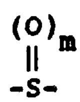

and m is 2;
(I) oxidizing a compound of formula I wherein X is

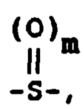

where m is 1, so as to prepare a compound of formula I wherein X is

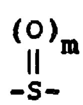

and m is 2;
(J) reacting a compound of the formula

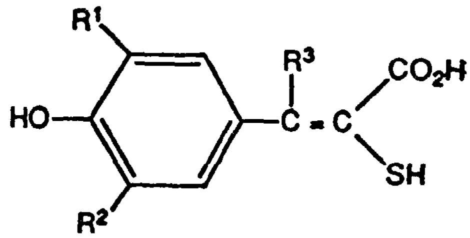

with

i) formic acid, so as to provide a compound of formula I wherein Q is O, $R^4$ and $R^5$ taken together form a bond and $R^6$ and $R^7$ are hydrogen; or

ii) carbon disulfide, so as to provide a compound of formula I wherein Q is O, $R^3$ and $R^4$ taken together form a bond and $R^6$ and $R^7$ taken together are =S;

(K) reacting a compound of the formula

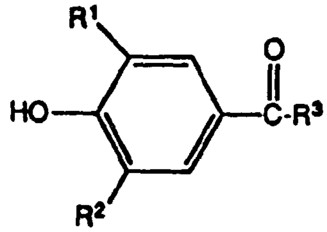

with a compound of the formula

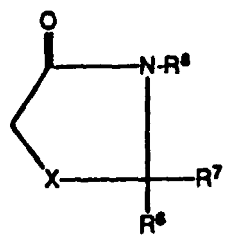

wherein $R^1$, $R^2$, $R^3$ and X are as defined in formula I, $R^6$ and $R^7$ taken together are =S, and $R^8$ is $-(CH_2)_n-Y$ (where n is an integer from 0 to 3, both inclusive, and Y is $OR^9$, where $R^9$ is hydrogen) so as to provide a compound of the formula

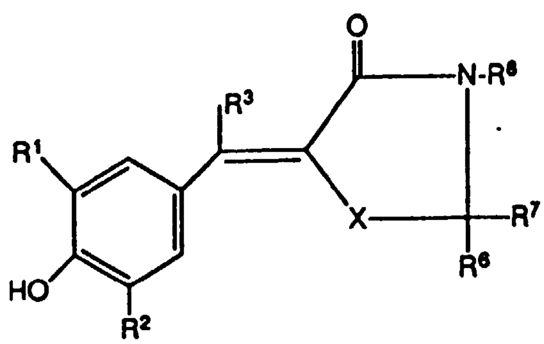

wherein $R^1$, $R^2$, $R^3$, $R^6$, $R^7$ and X are as set forth above and $R^8$ is $-(CH_2)_n-Y$ (where n is an integer from 0 to 3, both inclusive, and Y is $OR^9$, where $R^9$ is

$$-\overset{\overset{O}{\|}}{C}-CH_3 \;);$$

(L) reducing a compound of formula I in which $R^8$ is $-(CH_2)_n-Y$ wherein n is 0 to 3, both inclusive, and Y is $-OR^9$, where $R^9$ is

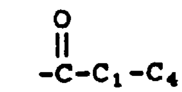

alkyl, so as to prepare a compound of formula I in which $R^8$ is $-(CH_2)_n$-Y, wherein n is 0 to 3, both inclusive, and Y is $OR^9$, where $R^9$ is hydrogen;

(M) reacting a compound of formula I in which $R^8$ is $-(CH_2)_n$-Y wherein n is 0 to 3, both inclusive, and Y is $-OR^9$, where $R^9$ is hydrogen, with a tosyl-halide so as to prepare a compound of formula I in which $R^8$ is $-(CH_2)_n$-Y wherein n is 0 to 3, both inclusive, and Y is $OR^9$, where $R^9$ is tosyl;

(N) reacting a compound of formula I in which $R^8$ is $-(CH_2)_n$-Y, wherein n is 0 to 3, both inclusive, and Y is $-OR^9$, where $R^9$ is tosyl, with an amine of the formula $HNR^{11}R^{12}$ (where $R^{11}$ and $R^{12}$ are as defined for formula I) so as to prepare a compound of formula I in which $R^8$ is $-(CH_2)_n$-Y, wherein n is 0 to 3, both inclusive, and Y is $-NR^{11}R^{12}$;

(O) treating a compound of formula I in which $R^8$ is $-(CH_2)_n$-Y, wherein n is 0 to 3, both inclusive, and Y is cyano with tri-n-butyl tin azide so as to prepare a compound of formula I in which $R^8$ is $-(CH_2)_n$-Y wherein n is 0 to 3, both inclusive, and Y is tetrazolyl;

(P) reacting a compound of the formula

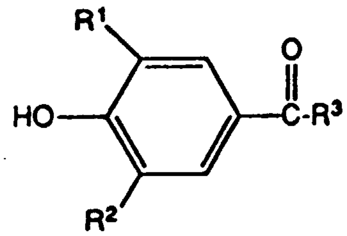

with a compound of the formula

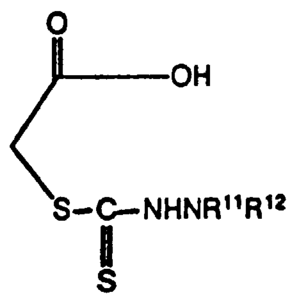

wherein $R^1$, $R^2$, $R^3$, $R^{11}$ and $R^{12}$ are as in formula I, so as to provide a compound of the formula

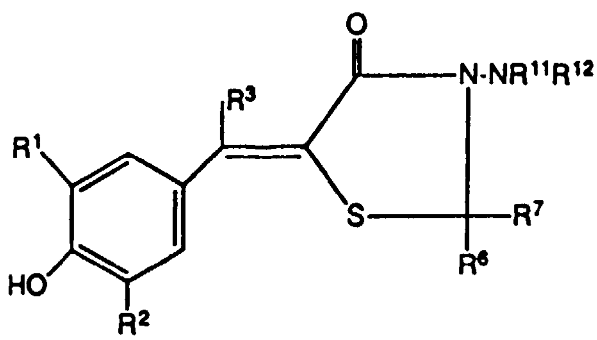

wherein $R^6$ and $R^7$ taken together are =S and $R^1$, $R^2$, $R^3$, $R^{11}$ and $R^{12}$ are as defined in formula I;

(Q) heating a compound of formula I in which $R^8$ is $-(CH_2)_n$-Y and Y is $-NR^{11}R^{12}$ (niether of $R^{11}$ or $R^{12}$ being hydrogen) in an ethanol/water mixture in the presence of a catalyst so as to prepare a compound of formula I in which $R^8$ is $-(CH_2)_n$-Y and Y is $-NR^{11}R^{12}$ (where one of $R^{11}$ or $R^{12}$ is hydrogen and the other is not hydrogen);

(R) reacting a compound of formula I in which $R^6$ and $R^7$ are both hydrogen with trifluoroacetic anhydride so as to prepare a compound of formula I in which one of $R^6$ and $R^7$ is hydrogen and the other is -OH;

(S) salifying a compound of formula I by reacting the non-salt form of the compound with either a strong acid

or a strong base.

**10.** A compound of formula (I) whenever prepared by a process according to Claim 9.

**Claims for the following Contracting States : ES, GR**

**1.** A process for preparing a compound, and the pharmaceutically acceptable salts thereof, of the formula (I):

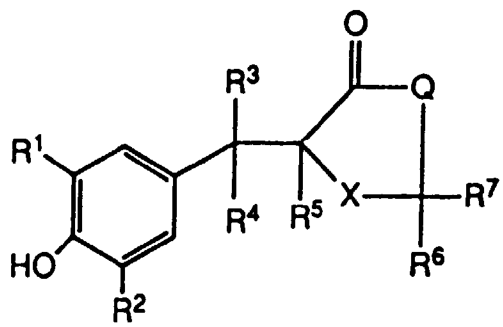

(I)

wherein:

$R^1$ and $R^2$ are each independently hydrogen, $C_1$-$C_6$ alkyl, $C_1$-$C_6$ alkoxy or

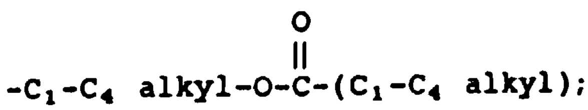

$R^3$ is hydrogen or $C_1$-$C_6$ alkyl;
$R^4$ and $R^5$ are each hydrogen, or when taken together form a bond;
$R^6$ and $R^7$ are each hydrogen or when taken together are =S or =O, or when one of $R^6$ or $R^7$ is hydrogen, the other is -OH or $-SCH_3$;
X is

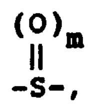

where m is 0, 1 or 2; and
Q is $-CH_2$-, -O- or $NR^8$ where $R^8$ is hydrogen, $C_1$-$C_6$ alkyl, $C_3$-$C_8$ cycloalkyl, $C_2$-$C_6$ alkenyl, $-SO_2CH_3$ or $-(CH_2)_n$-Y, where n is an integer from 0 to 3, both inclusive, and Y is cyano, $OR^9$,

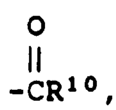

tetrazolyl, $-NR^{11}R^{12}$, -SH, -S($C_1$-$C_4$ alkyl) or

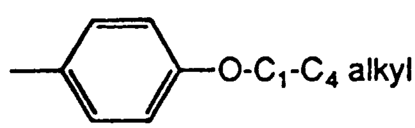

where $R^9$ is hydrogen, $C_1$-$C_4$ alkyl, tosyl or

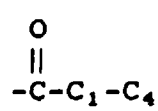

alkyl; $R^{10}$ is -OH, $C_1$-$C_4$ alkyl, $C_1$-$C_4$ alkoxy or $-NH_2$; $R^{11}$ and $R^{12}$ are each independently hydrogen, $C_1$-$C_6$ alkyl, $C_2$-$C_6$ alkenyl, $C_2$-$C_6$ alkenyl, $-(CH_2)_qOH$, $-(CH_2)_q$-N($C_1$-$C_4$ alkyl)$_2$, $-(CH_2)_q$-S($C_1$-$C_4$ alkyl) or

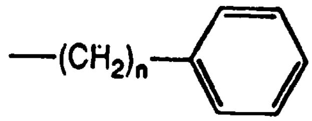

where q is an integer from 1 to 6, both inclusive, and n is as defined above; or $R^{11}$ and $R^{12}$ taken together form a morpholinyl, piperidinyl, piperazinyl or an N-methylpiperazinyl ring;
with the proviso that when Q is -O- or $NR^8$ ($R^8$ is hydrogen or $C_1$-$C_4$ alkyl), $R^3$ is hydrogen, $R^4$ and $R^5$ are each hydrogen or when taken together form a bond, $R^6$ and $R^7$ are each hydrogen or when taken together are =S and X is

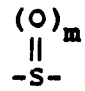

(where m is 0), $R^1$ and $R^2$ cannot both be a t-butyl group
and when Q is $NR^8$ ($R^8$ is hydrogen), $R^3$ is hydrogen, $R^6$ and $R^7$ taken together are =S and X is $-S(O)_m-$ (where m is 0), $R^4$ and $R^5$ must both be hydrogen;
and when Q is $NR^8$ [$R^8$ is hydrogen or $-(CH_2)_n$-Y, where n is 0 and Y is CO($C_1$-$C_4$ alkyl)], $R^3$ is hydrogen, $R^4$ and $R^5$ taken together form a bond, $R^6$ and $R^7$ are each hydrogen and X is $-S(O)_m-$ (where m is 0), $R^1$ and $R^2$ cannot both be an isopropyl group

which comprises:

(A) reacting a compound of the formula

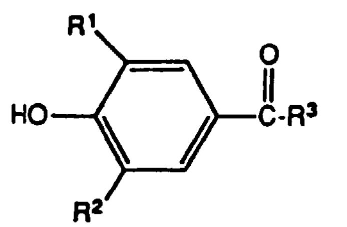

with a compound of the formula

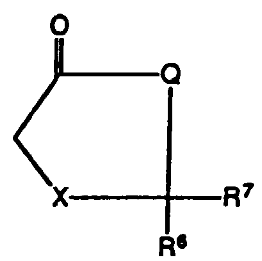

wherein $R^1$, $R^2$, $R^3$ and X are as in formula I, Q is $-CH_2-$ or $NR^8$ (where $R^8$ is as defined in formula I) and $R^6$ and $R^7$ taken together are =S, so as to provide a compound of the formula

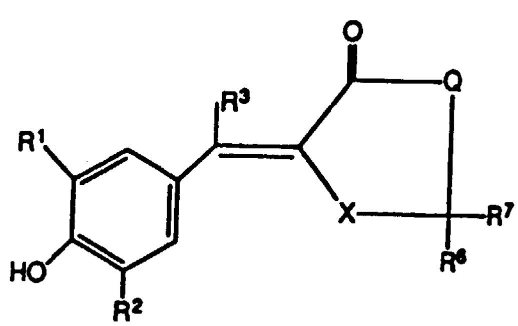

wherein $R^1$, $R^2$, $R^3$, $R^6$, $R^7$, X and Q are as set forth above;
(B) reducing a compound of formula I wherein $R^6$ and $R^7$ taken together are =S so as to prepare a compound of formula I in which $R^6$ and $R^7$ are hydrogen;

(C) reducing a compound of formula I in which $R^4$ and $R^5$ taken together form a bond so as to prepare a compound of formula I in which $R^4$ and $R^5$ are hydrogen;
(D) reducing a compound of formula I in which $R^4$ and $R^5$ taken together form a bond and $R^6$ and $R^7$ taken together are =S so as to prepare a compound of formula I in which $R^4$, $R^5$, $R^6$ and $R^7$ are all hydrogen;
(E) alkylating a compound of formula I in which $R^8$ is hydrogen so as to prepare a compound of formula I in which $R^8$ is $C_1$-$C_6$ alkyl, $C_2$-$C_6$ alkenyl, $C_3$-$C_8$ cycloalkyl or $-(CH_2)_n$-Y (where n is an integer from 0 to 3, both inclusive, and Y is cyano, $OR^9$, -SH, -S($C_1$-$C_4$ alkyl), $-NR^{11}R^{12}$ or

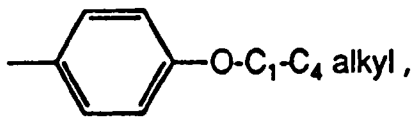

where $R^9$, $R^{11}$ and $R^{12}$ are as defined in formula I);
(F) acylating a compound of formula I in which $R^8$ is hydrogen so as to prepare a compound of formula I in which $R^8$ is $-(CH_2)_n$-Y where n is an integer from 0 to 3, both inclusive, and Y is

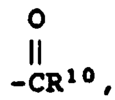

where $R^{10}$ is as defined in formula I;
(G) oxidizing a compound of formula I wherein X is

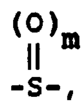

where m is 0, so as to prepare a compound of formula I wherein X is

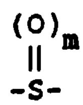

and m is 1;
(H) oxidizing a compound of formula I wherein X is

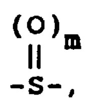

where m is 0, so as to prepare a compound of formula I wherein X is

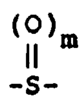

and m is 2;
(I) oxidizing a compound of formula I wherein X is

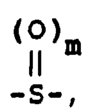

where m is 1, so as to prepare a compound of formula I wherein X is

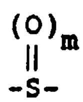

and m is 2;
(J) reacting a compound of the formula

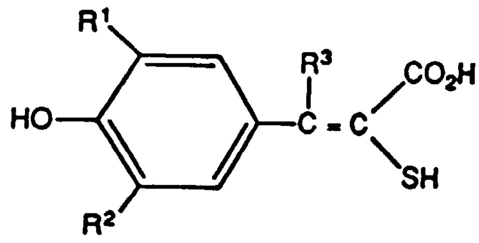

with

i) formic acid, so as to provide a compound of formula I wherein Q is O, $R^4$ and $R^5$ taken together form a bond and $R^6$ and $R^7$ are hydrogen; or
ii) carbon disulfide, so as to provide a compound of formula I wherein Q is O, $R^3$ and $R^4$ taken together form a bond and $R^6$ and $R^7$ taken together are =S;

(K) reacting a compound of the formula

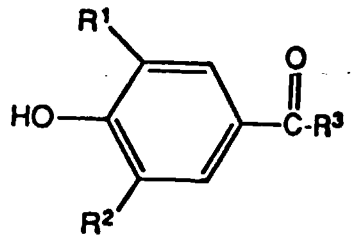

with a compound of the formula

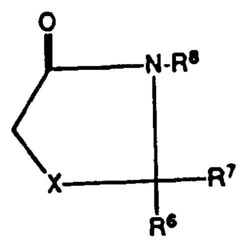

wherein $R^1$, $R^2$, $R^3$ and X are as defined in formula I, $R^6$ and $R^7$ taken together are =S, and $R^8$ is $-(CH_2)_n-Y$ (where n is an integer from 0 to 3, both inclusive, and Y is $OR^9$, where $R^9$ is hydrogen) so as to provide a compound of the formula

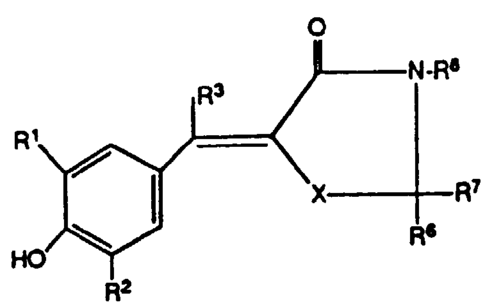

wherein $R^1$, $R^2$, $R^3$, $R^6$, $R^7$ and X are as set forth above and $R^8$ is $-(CH_2)_n-Y$ (where n is an integer from 0 to 3, both inclusive, and Y is $OR^9$, where $R^9$ is

$$-\overset{\overset{\displaystyle O}{\|}}{C}-CH_3\ );$$

(L) reducing a compound of formula I in which $R^8$ is $-(CH_2)_n-Y$, wherein n is 0 to 3, both inclusive, and Y is $-OR^9$, where $R^9$ is

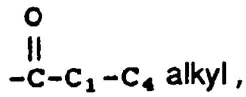

so as to prepare a compound of formula I in which $R^8$ is $-(CH_2)_n$-Y, wherein n is 0 to 3, both inclusive, and Y is $OR^9$, where $R^9$ is hydrogen;

(M) reacting a compound of formula I in which $R^8$ is $-(CH_2)_n$-Y, wherein n is 0 to 3, both inclusive, and Y is $-OR^9$, where $R^9$ is hydrogen, with a tosyl-halide so as to prepare a compound of formula I in which $R^8$ is $-(CH_2)_n$-Y, wherein n is 0 to 3, both inclusive, and Y is $OR^9$, where $R^9$ is tosyl;

(N) reacting a compound of formula I in which $R^8$ is $-(CH_2)_n$-Y, wherein n is 0 to 3, both inclusive, and Y is $-OR^9$, where $R^9$ is tosyl, with an amine of the formula $HNR^{11}R^{12}$ (where $R^{11}$ and $R^{12}$ are as defined for formula I) so as to prepare a compound of formula I in which $R^8$ is $-(CH_2)_n$-Y, wherein n is 0 to 3, both inclusive, and Y is $-NR^{11}R^{12}$;

(O) treating a compound of formula I in which $R^8$ is $-(CH_2)_n$-Y, wherein n is 0 to 3, both inclusive, and Y is cyano with tri-n-butyl tin azide so as to prepare a compound of formula I in which $R^8$ is $-(CH_2)_n$-Y wherein n is 0 to 3, both inclusive, and Y is tetrazolyl;

(P) reacting a compound of the formula

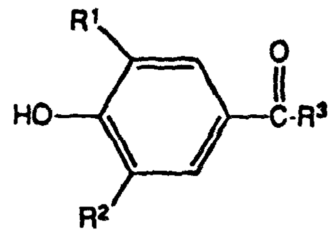

with a compound of the formula

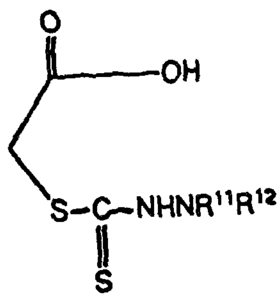

wherein $R^1$, $R^2$, $R^3$, $R^{11}$ and $R^{12}$ are as in formula I, so as to provide a compound of the formula

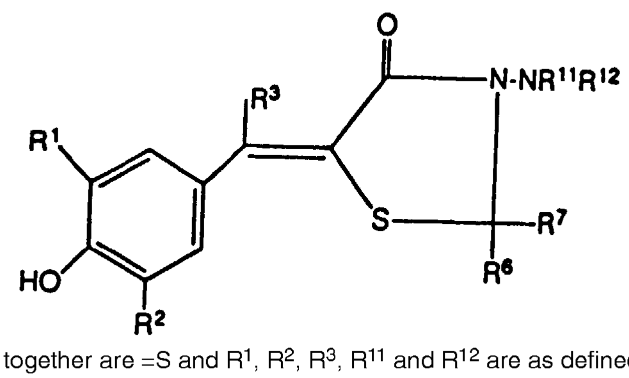

wherein $R^6$ and $R^7$ taken together are =S and $R^1$, $R^2$, $R^3$, $R^{11}$ and $R^{12}$ are as defined in formula I;

(Q) heating a compound of formula I in which $R^8$ is $-(CH_2)_n$-Y and Y is $-NR^{11}R^{12}$ (niether of $R^{11}$ or $R^{12}$ being hydrogen) in an ethanol/water mixture in the presence of a catalyst so as to prepare a compound of formula I in which $R^8$ is $-(CH_2)_n$-Y and Y is $-NR^{11}R^{12}$ (where one of $R^{11}$ or $R^{12}$ is hydrogen and the other is not hydrogen);

(R) reacting a compound of formula I in which $R^6$ and $R^7$ are both hydrogen with trifluoroacetic anhydride so as to prepare a compound of formula I in which one of $R^6$ and $R^7$ is hydrogen and the other is -OH;

(S) salifying a compound of formula I by reacting the non-salt form of the compound with either a strong acid or a strong base.

**2.** A pharmaceutical composition comprising as an active ingredient a compound of the formula (I), as defined in Claim 1, associated with one or more pharmaceutically acceptable diluents, excipients or carriers therefor.

**3.** A process for preparing a medicament of the formula (I), as defined in Claim 1, for use in treating inflammation and arthritis in mammals.

**4.** A process for preparing a medicament of the formula (II)

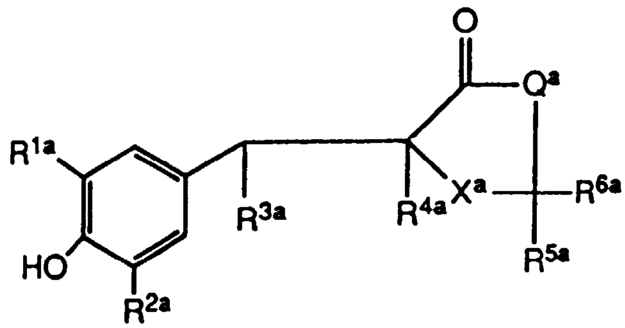

(II)

wherein:

$R^{1a}$ and $R^{2a}$ are each independently $C_1$-$C_6$ alkyl;
$R^{3a}$ and $R^{4a}$are each hydrogen or when taken together form a bond;
$R^{5a}$ and $R^{6a}$ are each hydrogen or when taken together are =O; and
$X^a$ is $-CH_2-$ or

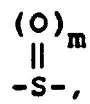

where m is 0, 1 or 2;
$Q^a$ is $NR^{7a}$;
$R^{7a}$ is hydrogen, $C_1$-$C_6$ alkyl, or $-(CH_2)_n-Y^a$, where n is an integer from 0 to 3, both inclusive, and $Y^a$ is cyano, $OR^{8a}$,

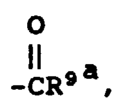

-SH, -S($C_1$-$C_4$ alkyl), tetrazolyl, $-NR^{10a}R^{11a}$ or

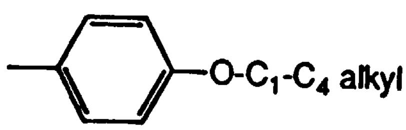

where $R^{8a}$ is hydrogen, $C_1$-$C_4$ alkyl, or

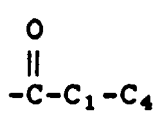

alkyl; $R^{9a}$ is $-NH_2$ or -OH; and $R^{10a}$ and $R^{11a}$ are each independently hydrogen, $C_1$-$C_6$ alkyl, $C_2$-$C_6$ alkenyl or $C_2$-$C_6$ alkynyl; with the proviso that when $R^{3a}$ and $R^{4a}$ taken together form a bond, $R^{5a}$ and $R^{6a}$ are each hydrogen, $X^a$ is

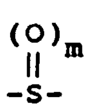

(where m is 0) and $R^{7a}$ is hydrogen or $C_1$-$C_4$ alkyl, $R^{1a}$ and $R^{2a}$ cannot both be a t-butyl group, for use in preventing ischemia-induced cell damage in mammals.

**5.** A process for preparing a medicament of the formula (III)

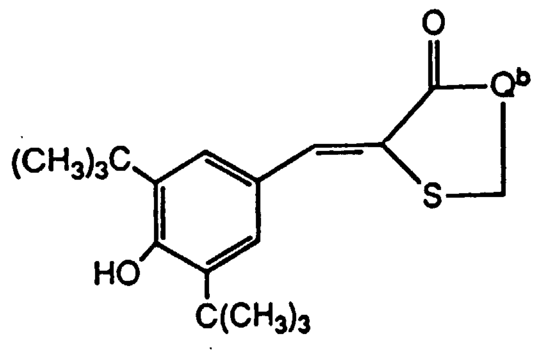

(III)

wherein:

$Q^b$ is -O- or $NR^{7b}$, where $R^{7b}$ is hydrogen, $C_1$-$C_6$ alkyl, $NR^{8b}R^{9b}$ or $-(CH_2)_n$-OH where $R^{8b}$ and $R^{9b}$ are each independently hydrogen or $C_1$-$C_4$ alkyl and n is an integer from 0 to 3, both inclusive for use in treating a dystrophic mammal.

## Patentansprüche

### Patentansprüche für folgende Vertragsstaaten : AT, BE, CH, DE, DK, FR, GB, IT, LI, NL, SE

**1.** Verbindung und pharmazeutisch annehmbare Salze davon mit der Formel (I):

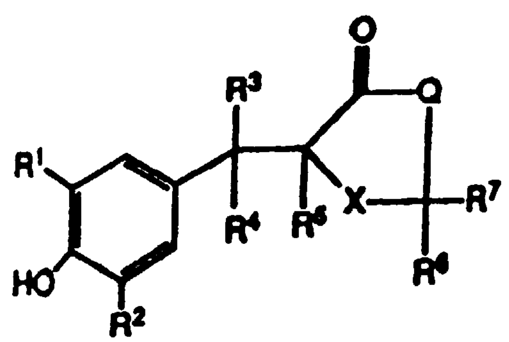

(I)

worin

$R^1$ und $R^2$ unabhängig Wasserstoff, $C_1$-$C_6$-Alkyl-, $C_1C_6$- Alkoxy- oder

$-C_1-C_4\text{-Alkyl-O-}\overset{O}{\overset{\|}{C}}\text{-}(C_1-C_4\text{-alkyl})$-Reste sind;

$R^3$ Wasserstoff oder ein $C_1$-$C_6$-Alkylrest ist;

$R^4$ und $R^5$ jeweils Wasserstoff sind oder zusammen eine Bindung bilden;

$R^6$ und $R^7$ jeweils Wasserstoff sind oder zusammen =S bilden oder wenn einer der Reste $R^6$ oder $R^7$ Wasserstoff ist, der andere -OH oder $-SCH_3$ ist;

$$X\ \overset{(O)_m}{\overset{|}{-S-}}$$

ist, worin m 0, 1 oder 2 ist und

Q $-CH_2$-, -O- oder $NR^8$ ist, worin $R^8$ Wasserstoff, ein $C_1$-$C_6$-Alkyl-, $C_3$-$C_8$-Cycloalkyl, $C_2$-$C_6$-Alkenylrest, $-SO_2CH_3$ oder $-(CH_2)_n$-Y ist, worin n eine ganze Zahl von 0 bis einschließlich 3 ist und

Y ein Cyanorest, $OR^9$,

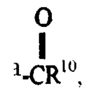

ein Tetrazolylrest, $-NR^{11}R^{12}$, -SH, $-S(C_1-C_4\text{-Alkyl})$ oder

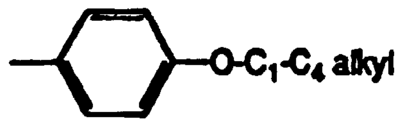

ist, worin

$R^9$ Wasserstoff, ein $C_1$-$C_4$-Alkyl-, Tosyl- oder

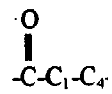

Alkylrest ist;

$R^{10}$ ein $C_1C_4$-Alkyl-, $C_1$-$C_4$-Alkylrest oder $-NH_2$ ist;

$R^{11}$ und R12 jeweils unabhängig Wasserstoff, $C_1$-$C_6$-Alkyl-, $C_2$-$C_6$-Alkenyl-, $C_2$-$C_6$-Alkinylreste, $-(CH_2)_qOH$. $-(CH_2)_q-N(C_1-C_4\text{-Alkyl})_z$, $-(CH_2)_q-S(C_1-C_4\text{-Alkyl})$ oder

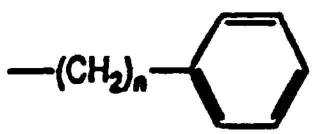

bedeuten worin q eine ganze Zahl von 1 bis einschließlich 6 ist und n wie oben definiert ist oder $R^{11}$ und $R^{12}$ zusammen einen Morpholinyl-, Piperidinyl-, Piperazinyl- oder N-Methylpiperazinylring bilden; mit dem Vorbehalt, daß dann, wenn Q -O- oder $NR^8$ ist ($R^8$ ist Wasserstoff oder ein $C_1$-$C_4$-Alkylrest), $R^3$ Wasserstoff ist, $R^4$ und $R^5$ jeweils Wasserstoff sind oder zusammen eine Bindung bilden, $R^6$ und $R^7$ jeweils Wasserstoff sind oder zusammen =S bilden und

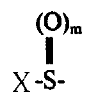

(worin m 0 ist) ist, $R^1$ und $R^2$ nicht beide eine t-Butylgruppe sein können und wenn Q $NR^8$ ist ($R^8$ ist Wasserstoff), $R^3$ Wasserstoff ist, $R^6$ und $R^7$ zusammen =S sind und X $-S(O)_m-$ ist, (worin m 0 ist), $R^4$ und $R^5$ beide Wasserstoff sein müssen und wenn Q $NR^8$ ist [$R^8$ ist Wasserstoff oder $-(CH_2)_n-Y$, worin n 0 ist und Y $CO(C_1$-$C_4$-Alkyl)ist], $R^3$ Wasserstoff ist, $R^4$ und $R^5$ zusammen eine Bindung bilden, $R^6$ und $R^7$ jeweils Wasserstoff sind und X $-S(O)_m-$ ist (worin m 0 ist), $R^1$ und $R^2$ nicht beide eine Isopropylgruppe sein können.

**2.** Verbindung nach Anspruch 1, worin $R^1$ und $R^2$ jeweils $C_1$-$C_6$-Alkylreste sind; $R^3$ Wasserstoff ist, $R^4$ und $R^5$ zusammen eine Bindung bilden; $R^6$ und $R^7$ jeweils Wasserstoff sind;

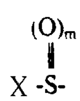

ist, worin m 0 ist und Q -O- oder $NR^8$ ist, worin $R^8$ wie in Anspruch 1 definiert ist.

**3.** Verbindung nach Anspruch 2, worin $R^1$ und $R^2$ jeweils $C_1$-$C_4$-Alkylreste sind; $R^3$ Wasserstoff ist, $R^4$ und $R^5$ zusammen eine Bindung bilden; $R^6$ und $R^7$ jeweils Wasserstoff sind;

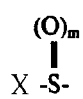

ist, worin m 0 ist und Q $NR^8$ ist, worin $R^8$ Wasserstoff, ein $C_1$-$C_6$-Alkylrest oder $-(CH_2)_n-Y$ ist, worin n und Y wie in Anspruch 1 definiert sind.

4. Verbindung der Formel I, wie in einem der Ansprüche 1, 2 oder 3 definiert, zur Verwendung zur Behandlung von Entzündungen oder Arthritis bei Säugetieren.

5. Pharmazeutische Zusammensetzung umfassend als aktiven Inhaltsstoff eine Verbindung der Formel (I) nach einem der Ansprüche 1, 2 oder 3 zusammen mit einem oder mehreren pharmazeutisch annehmbaren Verdünnungsmitteln, Hilfsstoffen oder Trägern dafür.

6. Verwendung einer Verbindung der Formel (II)

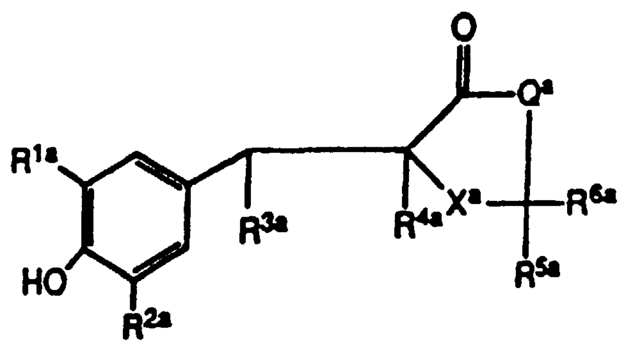

(II)

worin

$R^{1a}$ und $R^{2a}$ jeweils unabhängig $C_1$-$C_6$-Alkylreste sind;
$R^{3a}$ und $R^{4a}$ jeweils Wasserstoff sind oder zusammen eine Bindung bilden;
$R^{5a}$ und $R^{6a}$ jeweils Wasserstoff sind oder zusammen =O bilden und
$X^a$ $-CH_2-$ oder

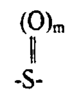

ist, worin m 0, 1 oder 2 ist;
$Q^a$ $NR^{7a}$ ist;
$R^{7a}$ Wasserstoff, ein $C_1$-$C_6$-Alkylrest oder $-(CH_2)_n-Y^a$ ist, worin n eine ganze Zahl von 0 bis einschließlich 3 ist und
$Y^a$ ein Cyanorest, $OR^{8a}$,

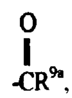

-SH, $-S(C_1$-$C_4$-Alkyl), ein Tetrazolylrest, $-NR^{10a}R^{11a}$ oder

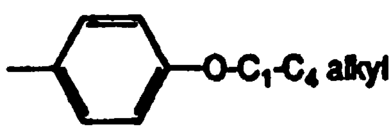

ist, worin
$R^{8a}$ Wasserstoff, ein $C_1$-$C_4$-Alkylrest oder

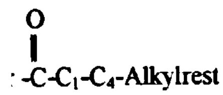

ist;
$R^{9a}$ $-NH_2$ oder -OH ist und
$R^{10a}$ und $R^{11a}$ jeweils unabhängig Wasserstoff, $C_1$-$C_6$-Alkyl-, $C_2$-$C_6$-Alkenyl- oder $C_2$-$C_6$-Alkinylreste sind, mit dem Vorbehalt, daß dann, wenn $R^{3a}$ und $R^{4a}$ zusammen eine Bindung bilden, $R^{5a}$ und $R^{6a}$ jeweils Wasserstoff sind,

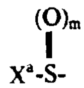

ist (worin m 0 ist) und $R^{7a}$ Wasserstoff oder ein $C_1$-$C_4$-Alkylrest ist, $R^{1a}$ und $R^{2a}$ nicht beide eine t-Butylgruppe sein können, zur Herstellung eines Arzneimittels zur Verhütung einer durch Ischämie induzierten Zellschädigung bei Säugetieren.

**7.** Verbindung der Formel (II)

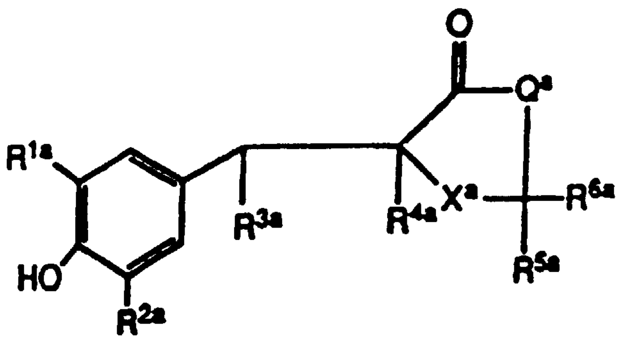

(II)

worin

$R^{1a}$ und $R^{2a}$ jeweils unabhängig $C_1$-$C_6$-Alkylreste sind;
$R^{3a}$ und $R^{4a}$ jeweils Wasserstoff sind oder zusammen eine Bindung bilden;
$R^{5a}$ und $R^{6a}$ jeweils Wasserstoff sind oder zusammen =O bilden und
$X^a$ $-CH_2-$ oder

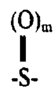

ist, worin m 0,1 oder 2 ist;
$Q^a$ $NR^{7a}$ ist;
$R^{7a}$ Wasserstoff, ein $C_1$-$C_6$-Alkylrest oder $-(CH_2)_n-Y^a$ ist, worin n eine ganze Zahl von 0 bis einschließlich 3 ist und
$Y^a$ ein Cyanorest, $OR^{8a}$,

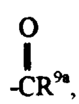

-SH, -S($C_1$-$C_4$-Alkyl), ein Tetrazolylrest, $-NR^{10a}R^{11a}$ oder

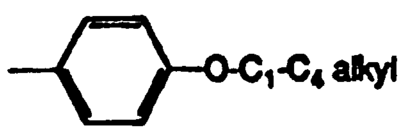

ist, worin
$R^{8a}$ Wasserstoff, ein $C_1$-$C_4$-Alkylrest oder ein

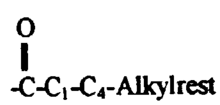

ist;
$R^{9a}$ $-NH_2$ oder -OH ist und
$R^{10a}$ und $R^{11a}$ unabhängig Wasserstoff, $C_1$-$C_6$-Alkyl-, $C_2$-$C_6$-Alkenyl- oder $C_2$-$C_6$-Alkinylreste sind; mit dem Vorbehalt, daß dann, wenn $R^{3a}$ und $R^{4a}$ zusammen eine Bindung bilden, $R^{5a}$ und $R^{6a}$ jeweils Wasserstoff sind oder zusammen =O bilden,

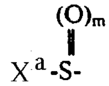

ist (worin m 0 ist) und $R^{7a}$ Wasserstoff oder eine $C_1$-$C_4$-Alkylgruppe ist, $R^{1a}$ und $R^{2a}$ nicht beide eine Isopropyl- oder t-Butylgruppe sein können, zur Verwendung zur Verhütung einer durch Ischämie induzierten Zellschädigung bei Säugetieren.

**8.** Verbindung der Formel (III)

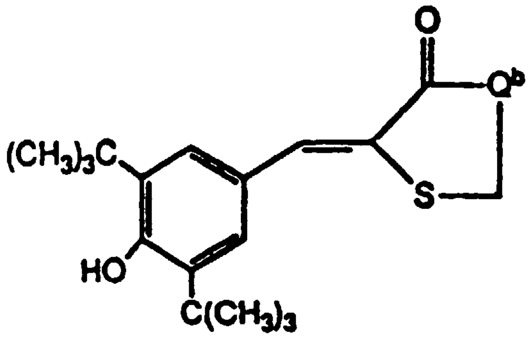

(III)

worin

$Q^b$ -O- oder $NR^{7b}$ ist, worin $R^{7b}$ Wasserstoff, ein $C_1$-$C_6$-Alkylrest, $NR^{8b}R^{9b}$ oder $-(CH_2)_n$-OH ist, worin $R^{8b}$ und $R^{9b}$ jeweils unabhängig Wasserstoff oder $C_1$-$C_4$-Alkylreste sind und n eine ganze Zahl von 0 bis einschließlich 3 ist, zur Verwendung zur Behandlung eines dystrophen Säugetieres.

**9.** Verfahren zur Herstellung einer Verbindung der Formel (I) nach einem der Ansprüche 1, 2 oder 3 umfassend, daß man

(A) eine Verbindung der Formel

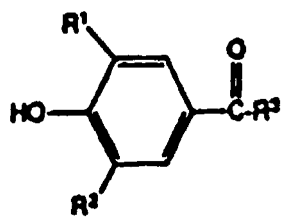

mit einer Verbindung der Formel

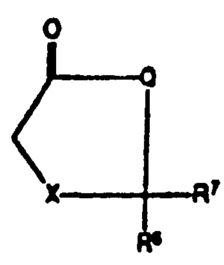

umsetzt, worin $R^1$, $R^2$, $R^3$ und X wie in Formel I definiert sind, Q $-CH_2-$ oder $NR^8$ ist (worin $R^8$ wie in Formel I definiert ist) und $R^6$ und $R^7$ zusammen =S sind, um eine Verbindung der Formel

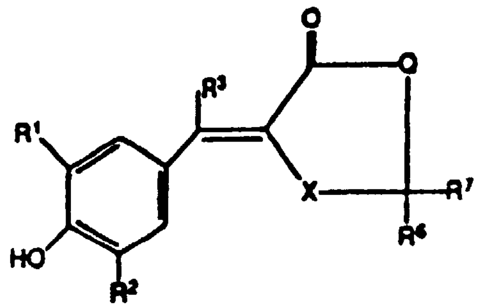

zu liefern, worin $R^1$, $R^2$, $R^3$, $R^6$, $R^7$, X und Q wie oben angegeben sind;
(B) eine Verbindung der Formel I, worin $R^6$ und $R^7$ zusammen =S sind, reduziert, um eine Verbindung der Formel I herzustellen, worin $R^6$ und $R^7$ Wasserstoff sind;
(C) eine Verbindung der Formel I, worin $R^4$ und $R^5$ zusammen eine Bindung bilden, reduziert, um eine Verbindung der Formel I, worin $R^4$ und $R^5$ Wasserstoff sind, herzustellen;
(D) eine Verbindung der Formel I, worin $R^4$ und $R^5$ zusammen eine Bindung bilden und $R^6$ und $R^7$ zusammen =S sind, reduziert, um eine Verbindung der Formel I herzustellen, worin $R^4$, $R^5$, $R^6$ und $R^7$ alle Wasserstoff sind;
(E) eine Verbindung der Formel I, worin $R^8$ Wasserstoff ist, alkyliert, um eine Verbindung der Formel I herzustellen, worin $R^8$ ein $C_1$-$C_6$-Alkyl-, $C_2$-$C_6$-Alkenyl-, $C_3$-$C_8$-Cycloalkylrest oder $-(CH_2)_n$-Y ist (worin n eine ganze Zahl von 0 bis einschließlich 3 ist) und Y ein Cyanorest, $-OR^9$, -SH, -S($C_1$-$C_4$-Alkyl), $-NR^{11}R^{12}$ oder

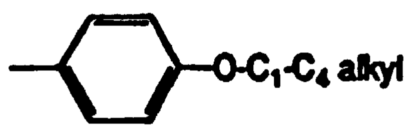

ist, worin $R^9$, $R^{11}$ und $R^{12}$ wie in Formel I definiert sind;
(F) eine Verbindung der Formel I, worin $R^8$ Wasserstoff ist, acyliert, um eine Verbindung der Formel I herzustellen, worin $R^8$-$(CH_2)_n$-Y ist, worin n eine ganze Zahl von 0 bis einschließlich 3 ist und

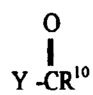

ist, worin $R^{10}$ wie in Formel I definiert ist;
(G) eine Verbindung der Formel I, worin

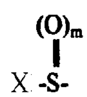

ist, worin m 0 ist, oxidiert, um eine Verbindung der Formel I herzustellen, worin

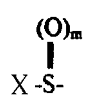

ist und m 1 ist;
(H) eine Verbindung der Formel I, worin

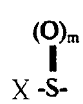

ist, worin m 0 ist, oxidiert, um eine Verbindung der Formel I
herzustellen, worin

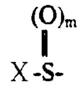

ist und m 2 ist;
(I) eine Verbindung der Formel I, worin

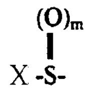

ist, worin m 1 ist, oxidiert, um eine Verbindung der Formel I herzustellen, worin

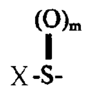

ist und m 2 ist;
(J) eine Verbindung der Formel

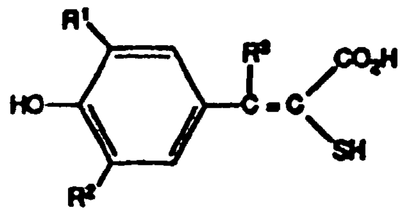

mit

i) Ameisensäure umsetzt, was eine Verbindung der Formel I liefert, worin Q O ist $R^4$ und $R^5$ zusammen eine Bindung bilden und $R^6$ und $R^7$ Wasserstoff sind oder
ii) mit Schwefelkohlenstoff umsetzt, was eine Verbindung der Formel I liefert, worin Q O ist, $R^3$ und $R^4$ zusammen eine Bindung bilden und $R^6$ und $R^7$ zusammen =S sind;

(K) eine Verbindung der Formel

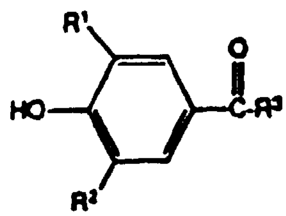

mit einer Verbindung der Formel

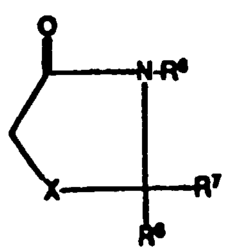

umsetzt, worin $R^1$, $R^2$, $R^3$ und X wie für Formel I definiert sind, $R^6$ und $R^7$ zusammen =S bilden und $R^8$ $-(CH_2)_n-$Y ist (worin n eine ganze Zahl von 0 bis einschließlich 3 ist und Y $OR^9$ ist, worin $R^9$ Wasserstoff ist), um eine Verbindung der Formel

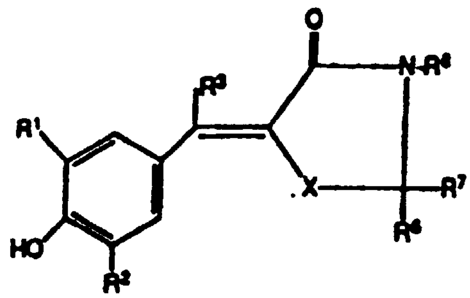

zu liefern, worin $R^1$, $R^2$, $R^3$, $R^6$, $R^7$ und X wie oben angegeben sind und $R^8$ $-(CH_2)_n$-Y ist (worin n eine ganze Zahl von 0 bis einschließlich 3 ist und Y $OR^9$ ist, worin

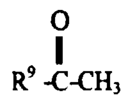

ist);

(L) eine Verbindung der Formel I, $R^8$ $-(CH_2)_n$-Y ist, worin n 0 bis einschließlich 3 ist und Y $-OR^9$ ist, worin

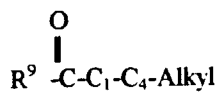

ist, reduziert, um eine Verbindung der Formel I herzustellen, worin $R^8$ $-(CH_2)_n$-Y ist, worin n 0 bis einschließlich 3 ist und Y $OR^9$ ist, worin $R^9$ Wasserstoff ist;

(M) eine Verbindung der Formel I, worin $R^8$ $-(CH_2)_n$-Y ist, worin n 0 bis einschließlich 3 ist und Y - $OR^9$ ist, worin $R^9$ Wasserstoff ist, mit einem Tosylhalogenid umsetzt, um eine Verbindung der Formel I herzustellen, worin $R^8$-$(CH_2)_n$-Y ist, worin n 0 bis einschließlich 3 ist und Y $OR^9$ ist, worin $R^9$ ein Tosylrest ist;

(N) eine Verbindung der Formel I, worin $R^8$ $-(CH_2)_n$-Y ist, worin n 0 bis einschließlich 3 ist und Y $-OR^9$ ist, worin $R^9$ ein Tosylrest ist, mit einem Amin der Formel $HNR^{11}R^{12}$ umsetzt (worin $R^{11}$ und $R^{12}$ wie für Formel I definiert sind), um eine Verbindung der Formel I herzustellen, worin $R^8$ $-(CH_2)_n$-Y ist, worin n 0 bis einschließlich 3 ist und Y $-NR^{11}R^{12}$ ist;

(O) eine Verbindung der Formel I, worin $R^8$ $-(CH_2)_n$-Y ist, worin n 0 bis einschließlich 3 ist und Y ein Cyanorest ist, mit Tri-n-butylzinnazid versetzt, um eine Verbindung der Formel I herzustellen, worin $R^8$ $-(CH_2)_n$-Y ist, worin n 0 bis einschließlich 3 ist und Y ein Tetrazolylrest ist;

(P) eine Verbindung der Formel

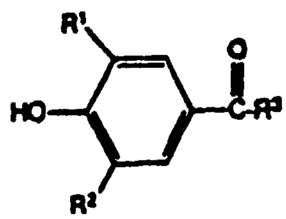

mit einer Verbindung der Formel

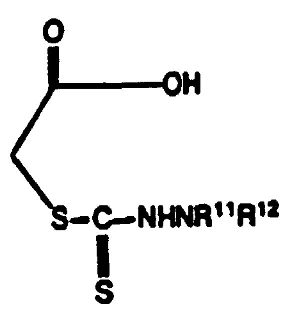

umsetz, worin $R^1$, $R^2$, $R^3$, $R^{11}$ und $R^{12}$ wie in Formel I definiert sind, um eine Verbindung der Formel

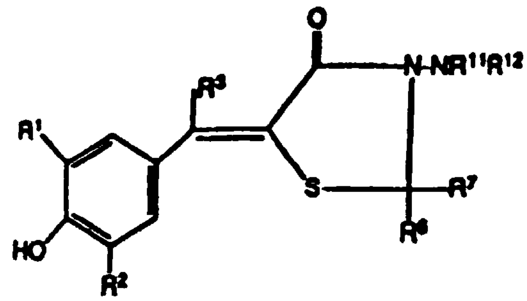

zu liefern, worin $R^6$ und $R^7$ zusammen =S sind und $R^1$, $R^2$, $R^3$, $R^{11}$ und $R^{12}$ wie in Formel I definiert sind;
(Q) eine Verbindung der Formel I, worin $R^8$-$(CH_2)_n$-Y ist und Y-$NR^{11}R^{12}$ ist (wobei weder $R^{11}$ noch $R^{12}$ Wasserstoff sind) in einer Ethanol/Wasser-Mischung in Gegenwart eines Katalysators erhitzt, um eine Verbindung der Formel I herzustellen, worin $R^8$ -$(CH_2)_n$-Y ist und Y-$NR^{11}R^{12}$ ist (worin einer der Reste $R^{11}$ oder $R^{12}$ Wasserstoff ist und der andere kein Wasserstoff ist);
(R) eine Verbindung der Formel I, worin $R^6$ und $R^7$ beide Wasserstoff sind, mit Trifluoressigsäureanhydrid umsetzt, um eine Verbindung der Formel I herzustellen, worin einer der Reste $R^6$ und $R^7$ Wasserstoff ist und der andere -OH ist;
(S) ein Salz einer Verbindung der Formel I bildet, in dem man die Nichtsalzform der Verbindung entweder mit einer starken Säure oder einer starken Base umsetzt.

**10.** Verbindung der Formel (I) hergestellt mit einem Verfahren nach Anspruch 9.

**Patentansprüche für folgende Vertragsstaaten : ES, GR**

**1.** Verfahren zur Herstellung einer Verbindung und der pharmazeutisch annehmbaren Salze davon mit der Formel (I):

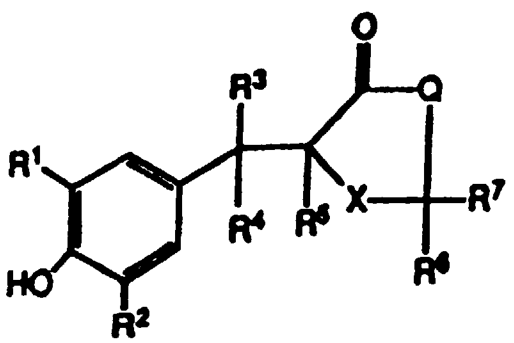

(I)

worin

$R^1$ und $R^2$ unabhängig Wasserstoff, $C_1$-$C_6$-Alkyl-, $C_1$-$C_6$-Alkoxy- oder

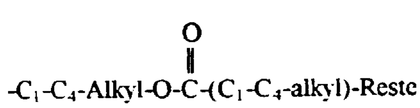

sind;
$R^3$ Wasserstoff oder ein $C_1$-$C_6$-Alkylrest ist;
$R^4$ und $R^5$ jeweils Wasserstoff sind oder zusammen eine Bindung bilden;
$R^6$ und $R^7$ jeweils Wasserstoff sind oder zusammen =S bilden oder wenn einer der Reste $R^6$ oder $R^7$ Wasserstoff ist, der andere -OH oder -$SCH_3$ ist;

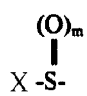

ist, worin m 0, 1 oder 2 ist und

Q $-CH_2-$, -O- oder $NR^8$ ist, worin $R^8$ Wasserstoff, ein $C_1$-$C_6$-Alkyl-, $C_3$-$C_8$-Cycloalkyl-, $C_2$-$C_6$-Alkenylrest, $-SO_2CH_3$ oder $-(CH_2)_n$-Y ist, worin n eine ganze Zahl von 0 bis einschließlich 3 ist und

Y ein Cyanorest, $OR^9$,

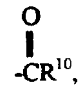

ein Tetrazolylrest, $-NR^{11}R^{12}$, -SH, -S($C_1$-$C_4$-Alkyl) oder

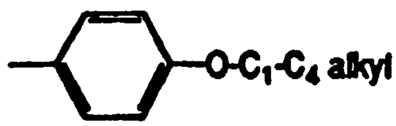

ist, worin

$R^9$ Wasserstoff, ein $C_1$-$C_4$-Alkyl-, Tosyl- oder

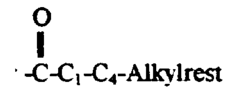

ist;

$R^{10}$ ein $C_1$-$C_4$-Alkyl-, $C_1$-$C_4$-Alkoxyrest oder $-NH_2$ ist;

$R^{11}$ und $R^{12}$ jeweils unabhängig Wasserstoff, $C_1$-$C_6$-Alkyl-, $C_2$-$C_6$-Alkenyl-, $C_2$-$C_6$-Alkinylreste, $-(CH_2)_qOH$, $-(CH_2)_q$-N($C_1$-$C_4$-Alkyl$)_2$, $-(CH_2)_q$-S($C_1$-$C_4$-Alkyl) oder

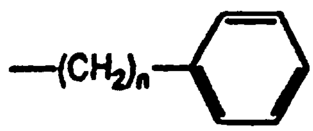

bedeuten, worin q eine ganze Zahl von 1 bis einschließlich 6 ist und n wie oben definiert ist oder $R^{11}$ und $R^{12}$ zusammen einen Morpholinyl-. Piperidinyl-, Piperazinyl- oder N-Methylpiperazinylring bilden; mit dem Vorbehalt, daß dann, wenn Q -O- oder $NR^8$ ist ($R^8$ ist Wasserstoff oder ein $C_1$-$C_4$-Alkylrest), $R^3$ Wasserstoff ist, $R^4$ und $R^5$ jeweils Wasserstoff sind oder zusammen eine Bindung bilden, $R^6$ und $R^7$ jeweils Wasserstoff sind oder zusammen =S bilden und

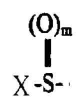

(worin m 0 ist) ist, $R^1$ und $R^2$ nicht beide eine t-Butylgruppe sein können und wenn

Q $NR^8$ ist ($R^8$ ist Wasserstoff), $R^3$ Wasserstoff ist, $R^6$ und $R^7$ zusammen =S sind und X $-S(O)_m$- ist, (worin m 0 ist),

$R^4$ und $R^5$ beide Wasserstoff sein müssen und wenn Q $NR^8$ ist [$R^8$ ist Wasserstoff oder $-(CH_2)_n$-Y, worin n 0 ist und

Y CO($C_1$-$C_4$-Alkyl) ist], $R^3$ Wasserstoff ist, $R^4$ und $R^5$ zusammen eine Bindung bilden, $R^6$ und $R^7$ jeweils Wasserstoff sind und X $-S(O)_m$- ist (worin m 0 ist), $R^1$ und $R^2$ nicht beide eine Isopropylgruppe sein können, umfassend, daß man

(A) eine Verbindung der Formel

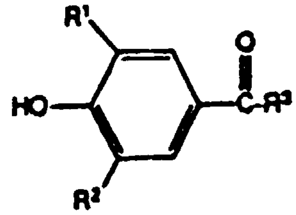

mit einer Verbindung der Formel

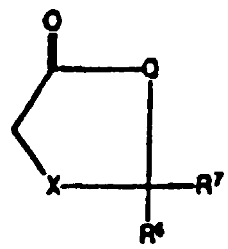

umsetzt, worin $R^1$, $R^2$, $R^3$ und X wie in Formel I definiert sind, Q $-CH_2-$ oder $NR^8$ ist (worin $R^8$ wie in Formel I definiert ist) und $R^6$ und $R^7$ zusammen =S sind, um eine Verbindung der Formel

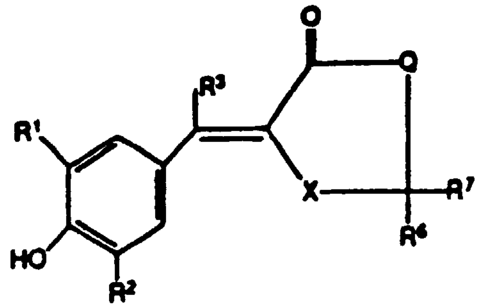

zu liefern, worin $R^1$, $R^2$, $R^3$, $R^6$, $R^7$, X und Q wie oben angegeben sind;

(B) eine Verbindung der Formel I, worin $R^6$ und $R^7$ zusammen =S sind, reduziert, um eine Verbindung der Formel I herzustellen, worin $R^6$ und $R^7$ Wasserstoff sind;

(C) eine Verbindung der Formel I, worin $R^4$ und $R^5$ zusammen eine Bindung bilden, reduziert, um eine Verbindung der Formel I, worin $R^4$ und $R^5$ Wasserstoff sind, herzustellen;

(D) eine Verbindung der Formel I, worin $R^4$ und $R^5$ zusammen eine Bindung bilden und $R^6$ und $R^7$ zusammen =S sind, reduziert, um eine Verbindung der Formel I herzustellen, worin $R^4$, $R^5$, $R^6$ und $R^7$ alle Wasserstoff sind;

(E) eine Verbindung der Formel I, worin $R^8$ Wasserstoff ist, alkyliert, um eine Verbindung der Formel I herzustellen, worin $R^8$ ein $C_1$-$C_6$-Alkyl-, $C_2$-$C_6$-Alkenyl-, $C_3$-$C_8$-Cycloalkylrest oder $-(CH_2)_n-Y$ ist (worin n eine ganze Zahl von 0 bis einschließlich 3 ist und Y ein Cyanorest, $OR^9$, -SH, -S($C_1$-$C_4$-Alkyl), $-NR^{11}R^{12}$ oder

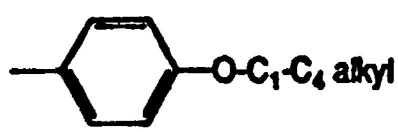

ist, worin $R^9$, $R^{11}$ und $R^{12}$ wie in Formel I definiert sind;

(F) eine Verbindung der Formel I, worin $R^8$ Wasserstoff ist, acyliert, um eine Verbindung der Formel I herzustellen, worin $R^8$ $-(CH_2)_n-Y$ ist, worin n eine ganze Zahl von 0 bis einschließlich 3 ist und

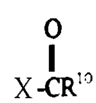

ist, worin $R^{10}$ wie in Formel I definiert ist;

(G) eine Verbindung der Formel I, worin

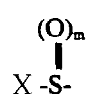

ist, worin m 0 ist, oxidiert, um eine Verbindung der Formel I herzustellen, worin

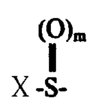

ist und m 1 ist;

(H) eine Verbindung der Formel I, worin

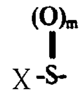

ist, worin m 0 ist, oxidiert, um eine Verbindung der Formel I herzustellen, worin

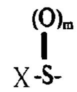

ist und m 2 ist;
(I) eine Verbindung der Formel I, worin

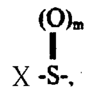

worin m 1 ist, oxidiert, um eine Verbindung der Formel I herzustellen, worin

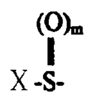

ist und m 2 ist;
(J) eine Verbindung der Formel

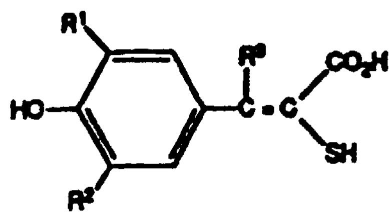

mit

i) Ameisensäure umsetzt, was eine Verbindung der Formel I liefert, worin Q O ist, $R^4$ und $R^5$ zusammen eine Bindung bilden und $R^6$ und $R^7$ Wasserstoff sind oder
ii) mit Schwefelkohlenstoff umsetzt, was eine Verbindung der Formel I liefert, worin Q O ist, $R^3$ und $R^4$ zusammen eine Bindung bilden und $R^6$ und $R^7$ zusammen =S sind;

(K) eine Verbindung der Formel

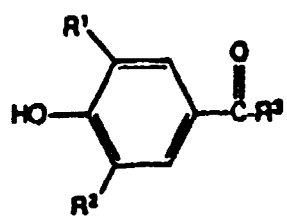

mit einer Verbindung der Formel

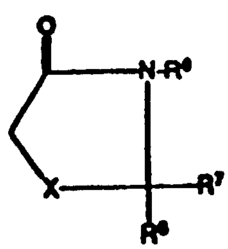

umsetzt, worin $R^1$, $R^2$, $R^3$ und X wie für Formel I definiert sind, $R^6$ und $R^7$ zusammen =S bilden und $R^8$ $-(CH_2)_n$-Y ist (worin n eine ganze Zahl von 0 bis einschließlich 3 ist und Y $OR^9$ ist, worin $R^9$ Wasserstoff ist), um eine Verbindung der Formel

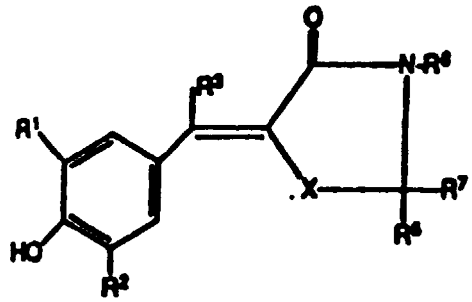

zu liefern, worin $R^1$, $R^2$, $R^3$, $R^6$, $R^7$ und X wie oben angegeben sind und $R^8$-$(CH_2)_n$-Y ist (worin n eine ganze Zahl von 0 bis einschließlich 3 ist und Y $OR^9$ ist, worin

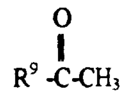

ist)

(L) eine Verbindung der Formel I, worin $R^8$-$(CH_2)_n$-Y ist, worin n 0 bis einschließlich 3 ist und Y $-OR^9$ ist, worin

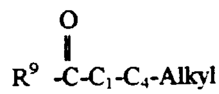

ist, reduziert, um eine Verbindung der Formel I herzustellen, worin $R^8$ $-(CH_2)_n$-Y ist worin n 0 bis einschließlich 3 ist und Y $OR^9$ ist, worin $R^9$ Wasserstoff ist;

(M) eine Verbindung der Formel I, worin $R^8$ $-(CH_2)_n$-Y ist, worin n 0 bis einschließlich 3 ist und Y $-OR^9$ ist, worin $R^9$ Wasserstoff ist, mit einem Tosylhalogenid umsetzt, um eine Verbindung der Formel I herzustellen, worin $R^8$-$(CH_2)_n$-Y ist, worin n 0 bis einschließlich 3 ist und Y $OR^9$ ist, worin $R^9$ ein Tosylrest ist;

(N) eine Verbindung der Formel I, worin $R^8$-$(CH_2)_n$-Y ist worin n 0 bis einschließlich 3 ist und Y $-OR^9$ ist, worin $R^9$ ein Tosylrest ist, mit einem Amin der Formel $HNR^{11}R^{12}$ umsetzt (worin $R^{11}$ und $R^{12}$ wie für Formel I definiert sind), um eine Verbindung der Formel I herzustellen, worin $R^8$ $-(CH_2)_n$-Y ist, worin n 0 bis einschließlich 3 ist und Y $-NR^{11}R^{12}$ ist;

(O) eine Verbindung der Formel I, worin $R^8$ $-(CH_2)_n$-Y ist, worin n 0 bis einschließlich 3 ist und Y ein Cyanorest ist, mit Tri-n-butylzinnazid versetzt, um eine Verbindung der Formel I herzustellen, worin $R^8$ $-(CH_2)_n$-Y ist, worin n 0 bis einschließlich 3 ist und Y ein Tetrazolylrest ist;

(P) eine Verbindung der Formel

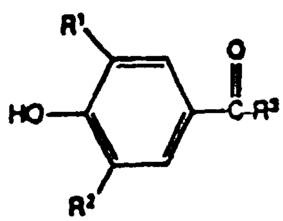

mit einer Verbindung der Formel

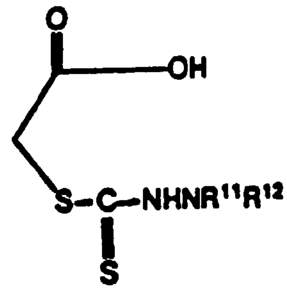

umsetzt, worin $R^1$, $R^2$, $R^3$, $R^{11}$ und $R^{12}$ wie in Formel I definiert sind, um eine Verbindung der Formel

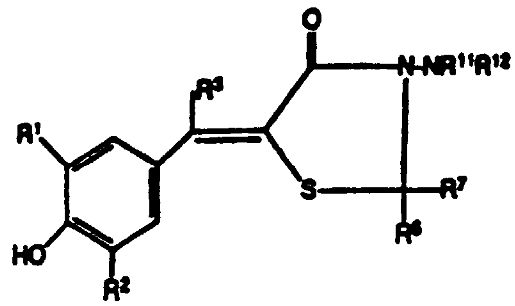

zu liefern, worin $R^6$ und $R^7$ zusammen =S sind und $R^1$, $R^2$, $R^3$, $R^{11}$ und $R^{12}$ wie in Formel I definiert sind;
(Q) eine Verbindung der Formel I, worin $R^8$ $-(CH_2)_n$-Y ist und Y $-NR^{11}R^{12}$ ist (wobei weder $R^{11}$ noch $R^{12}$ Wasserstoff sind) in einer Ethanol/Wasser-Mischung in Gegenwart eines Katalysators erhitzt, um eine Verbindung der Formel I herzustellen, worin $R^8$ $-(CH_2)_n$-Y ist und Y-$NR^{11}R^{12}$ ist (worin einer der Reste $R^{11}$ oder $R^{12}$ Wasserstoff ist und der andere kein Wasserstoff ist);
(R) eine Verbindung der Formel I, worin $R^6$ und $R^7$ beide Wasserstoff sind, mit Trifluoressigsäureanhydrid umsetzt, um eine Verbindung der Formel I herzustellen, worin einer der Reste $R^6$ und $R^7$ Wasserstoff ist und der andere -OH ist;
(S) ein Salz einer Verbindung der Formel I bildet, indem man die Nichtsalzform der Verbindung entweder mit einer starken Säure oder einer starken Base umsetzt.

2. Pharmazeutische Zusammensetzung umfassend als aktiven Inhaltsstoff eine Verbindung der Formel (I), wie in Anspruch 1 definiert, zusammen mit einem oder mehreren pharmazeutisch annehmbaren Verdünnungsmitteln, Hilfsstoffen oder Trägern dafür.

3. Verfahren zur Herstellung eines Arzneimittels der Formel (I), wie in Anspruch 1 definiert, zur Behandlung von Entzündungen und Anthritis bei Säugetieren.

4. Verfahren zur Herstellung eines Arzneimittels der Formel (II)

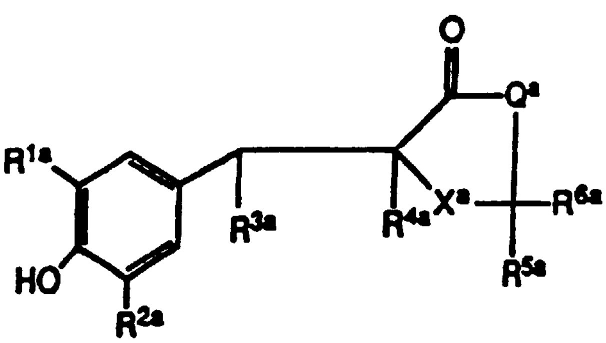

(II)

worin

$R^{1a}$ und $R^{2a}$ unabhängig $C_1$-$C_6$-Alkylreste sind;

$R^{3a}$ und $R^{4a}$ jeweils Wasserstoff sind oder zusammen eine Bindung bilden;
$R^{5a}$ und $R^{6a}$ jeweils Wasserstoff sind oder zusammen =O bilden und
$X^a$ $-CH_2-$ oder

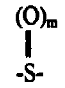

ist, worin m 0, 1 oder 2 ist;
$Q^aNR^{7a}$ ist;
$R^{7a}$ Wasserstoff, ein $C_1$-$C_6$-Alkylrest oder $-(CH_2)_n-Y^a$ ist, worin n eine ganze Zahl von 0 bis einschließlich 3 ist und
$Y^a$ ein Cyanorest, $OR^{8a}$,

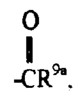

-SH, -S($C_1$-$C_4$-Alkyl), ein Tetrazolylrest, $-NR^{10a}R^{11a}$ oder

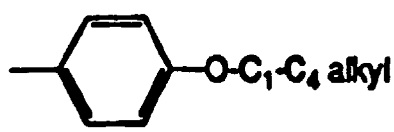

ist, worin
$R^{8a}$ Wasserstoff, ein $C_1$-$C_4$-Alkylrest oder ein

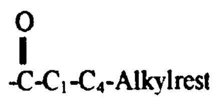

ist;
$R^{9a}$ $-NH_2$ oder -OH ist und
$R^{10a}$ und $R^{11a}$ jeweils unabhängig Wasserstoff, $C_1$-$C_6$-Alkyl-, $C_2$-$C_6$-Alkenyl- oder $C_2$-$C_6$-Alkinylreste sind; mit dem Vorbehalt, daß dann, wenn $R^{3a}$ und $R^{4a}$ zusammen eine Bindung bilden, $R^{5a}$ und $R^{6a}$ jeweils Wasserstoff sind,

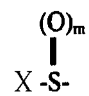

(worin m 0 ist) ist und $R^{7a}$ Wasserstoff oder ein $C_1$-$C_4$-Alkylrest ist, $R^{1a}$ und $R^{2a}$ nicht beide eine t-Butylgruppe sein können, zur Verwendung zur Verhütung einer durch Ischämie induzierten Zellschädigung bei Säugetieren.

**5.** Verfahren zur Herstellung eines Arzneimittels der Formel (III)

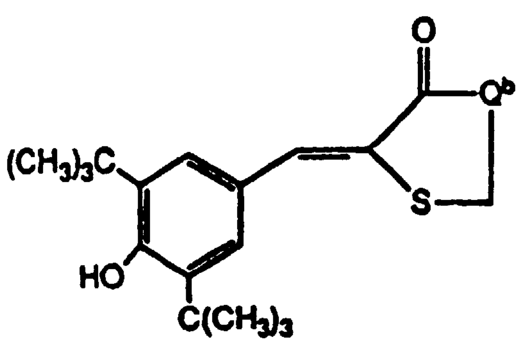

(III)

worin

$Q^b$ -O- oder $NR^{7b}$ ist, worin $R^{7b}$ Wasserstoff, ein $C_1$-$C_6$-Alkylrest, $NR^{8b}R^{9b}$ oder $-(CH_2)_n$-OH ist, worin $R^{8b}$ und

$R^{9b}$ jeweils unabhängig Wasserstoff oder $C_1$-$C_4$-Alkylreste sind und n eine ganze Zahl von 0 bis einschließlich 3 ist, zur Verwendung zur Behandlung eines dystrophen Säugetiers.

## Revendications

**Revendications pour les Etats contractants suivants : AT, BE, CH, DE, DK, FR, GB, IT, LI, NL, SE**

**1.** Composé, ainsi que ses sels pharmaceutiquement acceptables, répondant à la formule (I):

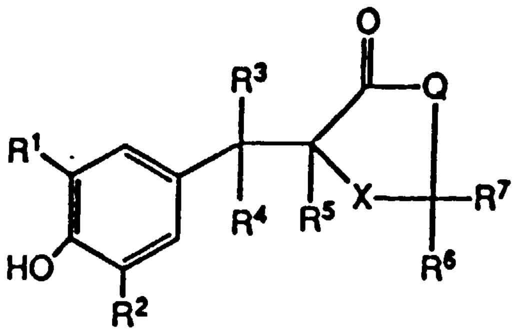

(I)

dans laquelle

$R^1$ et $R^2$ représentent, chacun indépendamment l'un de l'autre, un atome d'hydrogène, un groupe alkyle en $C_1$-$C_6$, un groupe alcoxy en $C_1$-$C_6$ ou un groupe

$$\text{alkyl(en } C_1\text{-}C_4\text{)-O-}\overset{\overset{O}{\|}}{C}\text{-alkyle}$$

en $C_1$-$C_4$;

$R^3$ représente un atome d'hydrogène ou un groupe alkyle en $C_1$-$C_6$;

$R^4$ et $R^5$ représentent, chacun indépendamment l'un de l'autre, un atome d'hydrogène ou, lorsqu'ils sont pris ensemble, forment une liaison;

$R^6$ et $R^7$ représentent, chacun indépendamment l'un de l'autre, un atome d'hydrogène ou, lorsqu'ils sont pris ensemble, =S, ou encore lorsqu'un des radicaux $R^6$ ou $R^7$ représente un atome d'hydrogène, l'autre représente -OH ou $-SCH_3$;

X représente

$$\overset{\overset{(O)_m}{\|}}{\text{-S-}}$$

où m est égal à 0, 1 ou 2; et

Q représente $-CH_2-$, -O- ou $NR^8$ où $R^8$ représente un atome d'hydrogène, un groupe alkyle en $C_1$-$C_6$, un groupe cycloalkyle en $C_3$-$C_8$, un groupe alcényle en $C_2$-$C_6$, $-SO_2CH_3$ ou $-(CH_2)_n$-Y où n représente un entier de 0 à 3, les deux étant inclus, et Y représente un groupe cyano, $OR^9$,

$$\overset{\overset{O}{\|}}{\text{-C}}R^{10},$$

un groupe tétrazolyle, $-NR^{11}R^{12}$, -SH, -S(alkyle en $C_1$-$C_4$) ou

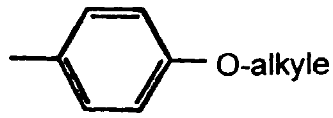

en $C_1$-$C_4$ où $R^9$ représente un atome d'hydrogène, un groupe alkyle en $C_1$-$C_4$, un groupe tosyle ou un groupe

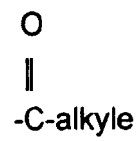

en $C_1$-$C_4$; $R^{10}$ représente un groupe alkyle en $C_1$-$C_4$, un groupe alcoxy en $C_1$-$C_4$ ou $-NH_2$; $R^{11}$ et $R^{12}$ représentent, chacun indépendamment l'un de l'autre, un atome d'hydrogène, un groupe alkyle en $C_1$-$C_6$, un groupe alcényle en $C_2$-$C_6$, un groupe alcynyle en $C_2$-$C_6$, $-(CH_2)_qOH$, $-(CH_2)_q$-N(alkyle en $C_1$-$C_4$)$_2$, $-(CH_2)_q$-S-(alkyle en $C_1$-$C_4$) ou

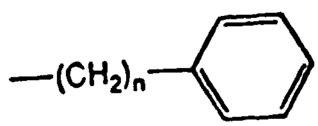

où q représente un entier de 1 à 6, les deux étant inclus, et n est tel que défini ci-dessus; ou bien $R^{11}$ et $R^{12}$ pris ensemble forment un noyau morpholinyle, un noyau pipéridinyle, un noyau pipérazinyle ou un noyau N-méthylpipérazinyle;

avec cette réserve que, lorsque Q représente -O- ou $NR^8$ ($R^8$ représente un atome d'hydrogène ou un groupe alkyle en $C_1$-$C_4$), $R^3$ représente un atome d'hydrogène, $R^4$ et $R^5$ représentent chacun un atome d'hydrogène ou, lorsqu'ils sont pris ensemble, forment une liaison, et $R^6$ et $R^7$ représentent chacun un atome d'hydrogène ou, lorsqu'ils sont pris ensemble, représentent =S, et X représente

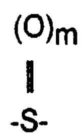

(où m est égal à 0), $R^1$ et $R^2$ ne peuvent représenter tous deux un groupe t-butyle;

et lorsque Q représente $NR^8$ ($R^8$ représente un atome d'hydrogène), $R^3$ représente un atome d'hydrogène, $R^6$ et $R^7$ pris ensemble représentent =S et
X représente $-S(O)_m$- (où m est égal à 0), $R^4$ et $R^5$ doivent représenter tous deux un atome d'hydrogène; et lorsque Q représente $NR^8$ [$R^8$ représente un atome d'hydrogène ou $-(CH_2)_n$-Y où n représente 0 et Y représente un groupe CO(alkyle en $C_1$-$C_4$)], $R^3$ représente un atome d'hydrogène, $R^4$ et $R^5$ pris ensemble forment une liaison, $R^6$ et $R^7$ représentent chacun un atome d'hydrogène et X représente $-S(O)_m$- (où m est égal à 0), $R^1$ et $R^2$ ne peuvent représenter tous deux un groupe isopropyle.

**2.** Composé selon la revendication 1, dans lequel $R^1$ et $R^2$ représentent chacun un groupe alkyle en $C_1$-$C_6$; $R^3$ représente un atome d'hydrogène; $R^4$ et $R^5$ pris ensemble forment une liaison; $R^6$ et $R^7$ représentent chacun un atome d'hydrogène;

X représente

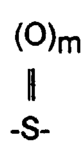

où m est égal à 0; et

Q représente -O- ou $NR^8$ où $R^8$ est tel que défini à la revendication 1.

**3.** Composé selon la revendication 2, dans lequel $R^1$ et $R^2$ représentent chacun un groupe alkyle en $C_1$-$C_4$; $R^3$ représente un atome d'hydrogène; $R^4$ et $R^5$ pris ensemble forment une liaison; $R^6$ et $R^7$ représentent chacun un atome d'hydrogène;

X représente

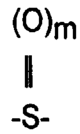

où m est égal à 0; et Q représente $NR^8$ où $R^8$ représente un atome d'hydrogène, un groupe alkyle en $C_1$-$C_6$ ou $-(CH_2)_n$-Y où n et Y sont tels que définis à la revendication 1.

**4.** Composé de formule I, tel que défini dans l'une quelconque des revendications 1, 2 ou 3, à utiliser dans le traitement de l'inflammation et de l'arthrite chez les mammifères.

**5.** Composition pharmaceutique comprenant, comme ingrédient actif, un composé de formule (I) selon l'une quelconque des revendications 1, 2 ou 3, en association avec un ou plusieurs diluants, excipients ou supports pharmaceutiquement acceptables pour le composé.

**6.** Utilisation d'un composé de formule (II)

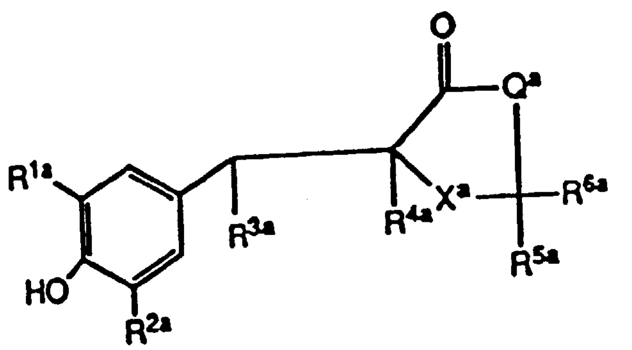

(II)

dans laquelle

| | |
|---|---|
| $R^{1a}$ et $R^{2a}$ | représentent, chacun indépendamment l'un de l'autre, un groupe alkyle en $C_1$-$C_6$, |
| $R^{3a}$ et $R^{4a}$ | représentent chacun un atome d'hydrogène ou, lorsqu'ils sont pris ensemble, forment une liaison; |
| $R^{5a}$ et $R^{6a}$ | représentent chacun un atome d'hydrogène ou, lorsqu'ils sont pris ensemble, représentent =O; et |
| $X^a$ | représente $-CH_2$- ou |

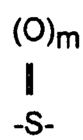

où m est égal à 0, 1 ou 2; et

| | |
|---|---|
| $Q^a$ | représente $NR^{7a}$; |
| $R^{7a}$ | représente un atome d'hydrogène, un groupe alkyle en $C_1$-$C_6$ ou $-(CH_2)_n$-$Y^a$ où n représente un entier de 0 à 3, les deux étant inclus, et $Y^a$ représente un groupe cyano, $OR^{8a}$, |

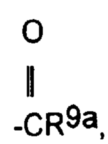

-SH, -S(alkyle en $C_1$-$C_4$), un groupe tétrazolyle, $-NR^{10a}R^{11a}$ ou

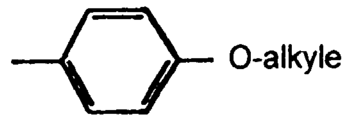

en $C_1$-$C_4$ où $R^{8a}$ représente un atome d'hydrogène, un groupe alkyle en $C_1$-$C_4$ ou un groupe

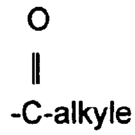

en $C_1$-$C_4$ $R^{9a}$ représente $-NH_2$ ou -OH; et $R^{10a}$ et $R^{11a}$ représentent, chacun indépendamment l'un de l'autre, un atome d'hydrogène, un groupe alkyle en $C_1$-$C_6$, un groupe alcényle en $C_2$-$C_6$ ou un groupe alcynyle en $C_2$-$C_6$; avec cette réserve que, lorsque $R^{3a}$ et $R^{4a}$ pris ensemble forment une liaison, $R^{5a}$ et $R^{6a}$ représentent chacun un atome d'hydrogène,
$X^a$ représente

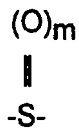

(où m est égal à 0), et $R^{7a}$ représente un atome d'hydrogène ou un groupe alkyle en $C_1$-$C_4$, $R^{1a}$ et $R^{2a}$ ne peuvent représenter ensemble un groupe t-butyle, pour la préparation d'un médicament pour empêcher la dégradation cellulaire induite par ischémie chez les mammifères.

**7.** Composé de formule (II)

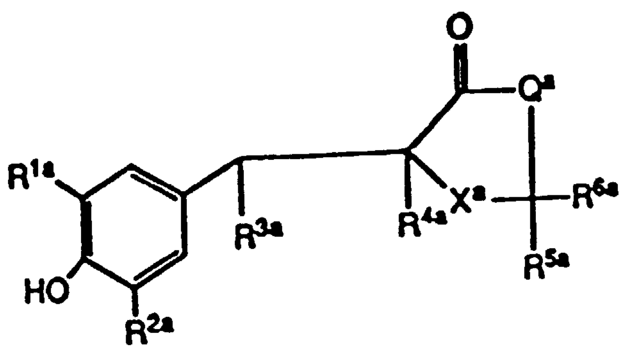

(II)

dans laquelle

| | |
|---|---|
| $R^{1a}$ et $R^{2a}$ | représentent, chacun indépendamment l'un de l'autre, un groupe alkyle en $C_1$-$C_6$, |
| $R^{3a}$ et $R^{4a}$ | représentent chacun un atome d'hydrogène ou, lorsqu'ils sont pris ensemble, forment une liaison; |
| $R^{5a}$ et $R^{6a}$ | représentent chacun un atome d'hydrogène ou, lorsqu'ils sont pris ensemble, représentent =O; et |
| $X^a$ | représente $-CH_2-$ ou |

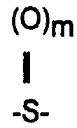

où m est égal à 0, 1 ou 2;

$Q^a$ représente $NR^{7a}$;

$R^{7a}$ représente un atome d'hydrogène, un groupe alkyle en $C_1$-$C_6$ ou $-(CH_2)_n-Y^a$ où n représente un entier de 0 à 3, les deux étant inclus, et $Y^a$ représente un groupe cyano, $OR^{8a}$,

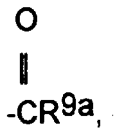

-SH, -S(alkyle en $C_1$-$C_4$), un groupe tétrazolyle, $-NR^{10a}R^{11a}$ ou

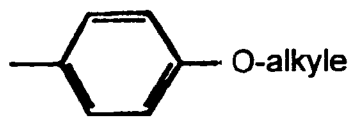

en $C_1$-$C_4$ où $R^{8a}$ représente un atome d'hydrogène, un groupe alkyle en $C_1$-$C_4$ ou un groupe

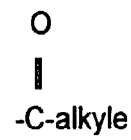

en $C_1$-$C_4$; $R^{9a}$ représente $-NH_2$ ou -OH; et $R^{10a}$ et $R^{11a}$ représentent, chacun indépendamment l'un de l'autre, un atome d'hydrogène, un groupe alkyle en $C_1$-$C_6$, un groupe alcényle en $C_2$-$C_6$ ou un groupe alcynyle en $C_2$-$C_6$; avec cette réserve que, lorsque $R^{3a}$ et $R^{4a}$ pris ensemble forment une liaison, $R^{5a}$ et $R^{6a}$ représentent chacun un atome d'hydrogène ou, lorsqu'ils sont pris ensemble, représentent =O;
$X^a$ représente

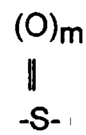

(où m est égal à 0), et $R^{7a}$ représente un atome d'hydrogène ou un groupe alkyle en $C_1$-$C_4$, $R^{1a}$ et $R^{2a}$ ne peuvent représenter tous deux un groupe isopropyle ou un groupe t-butyle, à utiliser pour empêcher la dégradation cellulaire induite par ischémie chez les mammifères.

**8.** Composé de formule (III)

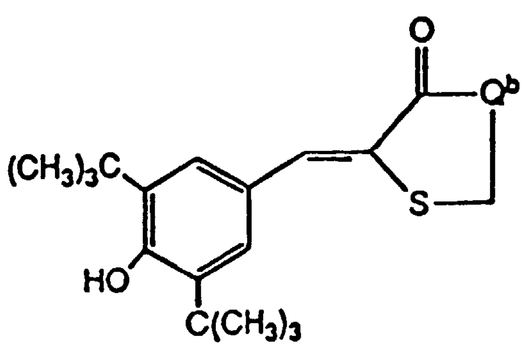

(III)

dans laquelle

$Q^b$ représente -O- ou $NR^{7b}$ où $R^{7b}$ représente un atome d'hydrogène, un groupe alkyle en $C_1$-$C_6$, $NR^{8b}R^{9b}$ ou $-(CH_2)_n-OH$ où $R^{8b}$ et $R^{9b}$ représentent, chacun indépendamment l'un de l'autre, un atome d'hydrogène, un groupe alkyle en $C_1$-$C_4$, et n représente un entier de 0 à 3, les deux étant inclus, à utiliser dans le traitement d'un mammifère dystrophique.

**9.** Procédé pour préparer un composé de formule (I) selon l'une quelconque des revendications 1, 2 ou 3, qui comprend le fait de :

(A) faire réagir un composé de formule

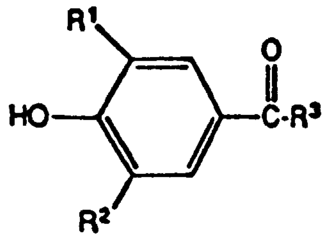

avec un composé de formule

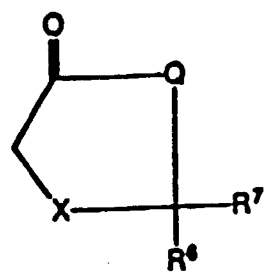

dans lesquelles $R^1$, $R^2$, $R^3$ et X sont tels qu'indiqués dans la formule I, Q représente $-CH_2-$ ou $NR^8$ (où $R^8$ est tel que défini dans la formule I), et $R^6$ et $R^7$ pris ensemble représentent =S pour obtenir un composé de formule

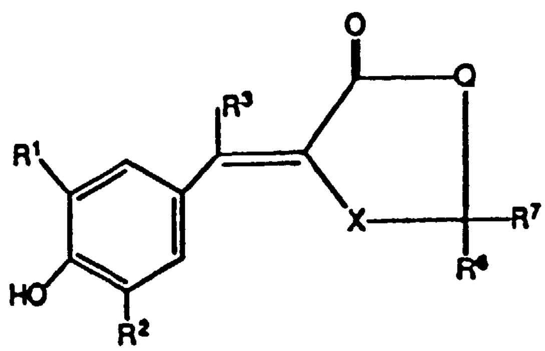

dans laquelle $R^1$, $R^2$, $R^3$, $R^6$, $R^7$, X et Q sont tels qu'indiqués ci-dessus;

(B) réduire un composé de formule I dans laquelle $R^6$ et $R^7$ pris ensemble représentent =S pour préparer un composé de formule I dans laquelle $R^6$ et $R^7$ représentent un atome d'hydrogène;

(C) réduire un composé de formule I dans laquelle $R^4$ et $R^5$ pris ensemble forment une liaison pour préparer un composé de formule I dans laquelle $R^4$ et $R^5$ représentent un atome d'hydrogène;

(D) réduire un composé de formule I dans laquelle $R^4$ et $R^5$ pris ensemble forment une liaison, et $R^6$ et $R^7$ pris ensemble représentent =S pour préparer un composé de formule I dans laquelle $R^4$, $R^5$, $R^6$ et $R^7$ représentent tous un atome d'hydrogène;

(E) alkyler un composé de formule I dans laquelle $R^8$ représente un atome d'hydrogène pour préparer un composé de formule I dans laquelle $R^8$ représente un groupe alkyle en $C_1$-$C_6$, un groupe alcényle en $C_2$-$C_6$, un groupe cycloalkyle en $C_3$-$C_8$ ou $-(CH_2)_n-Y$ (où n représente un entier de 0 à 3, les deux étant inclus, et Y représente un groupe cyano, $OR^9$, -SH, -S(alkyle en $C_1$-$C_4$), $-NR^{11}R^{12}$ ou

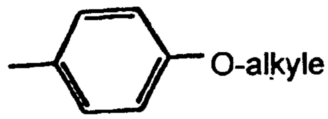

en $C_1$-$C_4$, où $R^9$, $R^{11}$ et $R^{12}$ sont tels que définis dans la formule I);

(F) acyler un composé de formule I dans laquelle $R^8$ représente un atome d'hydrogène pour préparer un composé de formule I dans laquelle $R^8$ représente $-(CH_2)_n-Y$ où n représente un entier de 0 à 3, les deux étant inclus, et

Y représente

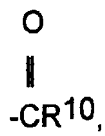

où $R^{10}$ est tel que défini à la formule I;

(G) oxyder un composé de formule I dans laquelle

X représente

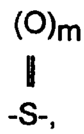

où m est égal à 0, pour préparer un composé de formule I dans laquelle X représente

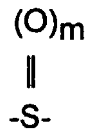

et m est égal à 1;

(H) oxyder un composé de formule I dans laquelle X représente

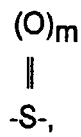

où m est égal à 0, pour préparer un composé de formule I dans laquelle

X représente

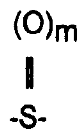

et m est égal à 2;

(I) oxyder un composé de formule I dans laquelle X représente

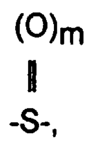

où m est égal à 1, pour préparer un composé de formule I dans laquelle

X représente

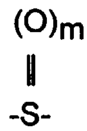

et m est égal à 2;

(J) faire réagir un composé de formule

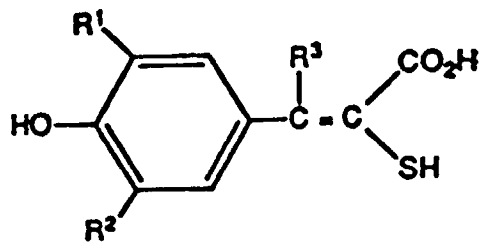

avec

i) de l'acide formique pour obtenir un composé de formule I dans laquelle Q représente O, $R^4$ et $R^5$ pris ensemble forment une liaison et $R^6$ et $R^7$ représentent un atome d'hydrogène; ou

ii) du disulfure de carbone pour obtenir un composé de formule I dans laquelle Q représente O, $R^3$ et $R^4$ pris ensemble forment une liaison et $R^6$ et $R^7$ pris ensemble représentent =S;

(K) faire réagir un composé de formule

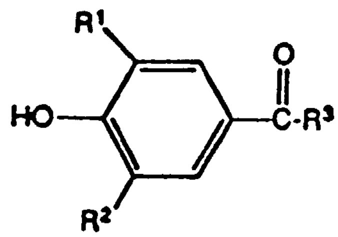

avec un composé de formule

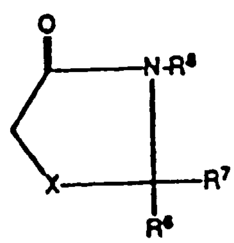

dans lesquelles $R^1$, $R^2$, $R^3$ et X sont tels que définis à la formule I, $R^6$ et $R^7$ pris ensemble représentent =S et $R^8$ représente $-(CH_2)_n-Y$ (où n représente un entier de 0 à 3, tous deux étant inclus, et Y représente $OR^9$ où $R^9$ représente un atome d'hydrogène) pour obtenir un composé de formule

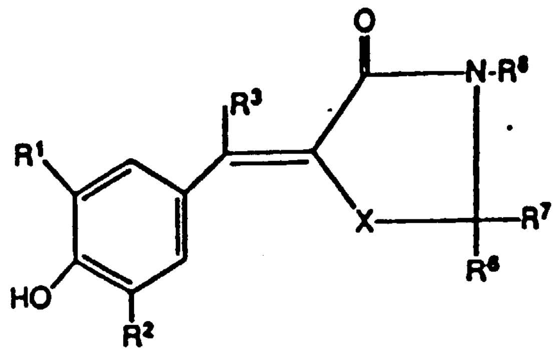

dans laquelle $R^1$, $R^2$, $R^3$, $R^6$, $R^7$ et X sont tels qu'indiqués ci-dessus et $R^8$ représente $-(CH_2)_n$-Y (où n représente un entier de 0 à 3, tous deux étant inclus, et Y représente $OR^9$ où
$R^9$ représente

O
‖
$-C-CH_3$);

(L) réduire un composé de formule I dans laquelle $R^8$ représente $-(CH_2)_n$-Y dans laquelle n représente de 0 à 3, tous deux étant inclus, et Y représente $-OR^9$ où
$R^9$ représente un groupe

O
‖
-C-alkyle

en $C_1$-$C_4$, pour préparer un composé de formule I dans laquelle $R^8$ représente $-(CH_2)_n$-Y dans laquelle n représente de 0 à 3, tous deux étant inclus, et Y représente $OR^9$ où $R^9$ représente un atome d'hydrogène;
(M) faire réagir un composé de formule I dans laquelle $R^8$ représente $-(CH_2)_n$-Y dans laquelle n représente de 0 à 3, tous deux étant inclus, et Y représente $-OR^9$ où $R^9$ représente un atome d'hydrogène, avec un halogénure de tosyle pour préparer un composé de formule I dans laquelle $R^8$ représente $-(CH_2)_n$-Y dans laquelle n représente de 0 à 3, tous deux étant inclus, et Y représente $OR^9$ où $R^9$ représente un groupe tosyle;
(N) faire réagir un composé de formule I dans laquelle $R^8$ représente $-(CH_2)_n$-Y dans laquelle n représente de 0 à 3, tous deux étant inclus, et Y représente $-OR^9$ où $R^9$ représente un groupe tosyle, avec une amine de formule $HNR^{11}R^{12}$ (où $R^{11}$ et $R^{12}$ sont tels que définis pour la formule I), pour préparer un composé de formule I dans laquelle $R^8$ représente $-(CH_2)_n$-Y dans laquelle n représente de 0 à 3, tous deux étant inclus, et Y représente $-NR_{11}R_{12}$;
(O) traiter un composé de formule I dans laquelle $R^8$ représente $-(CH_2)_n$-Y dans laquelle n représente de 0 à 3, tous deux étant inclus, et Y représente un groupe cyano, avec de l'azide de tri-n-butylétain, pour préparer un composé de formule I dans laquelle $R^8$ représente $-(CH_2)_n$-Y dans laquelle n représente de 0 à 3, tous deux étant inclus, et Y représente un groupe tétrazolyle;
(P) faire réagir un composé de formule

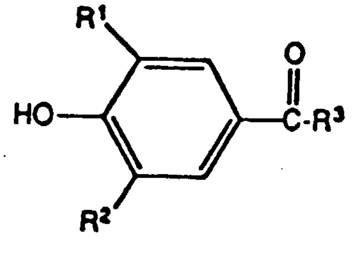

avec un composé de formule

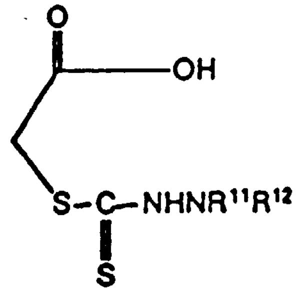

dans lesquelles $R^1$, $R^2$, $R^3$, $R^{11}$ et $R^{12}$ sont tels qu'indiqués en formule I, pour obtenir un composé de formule

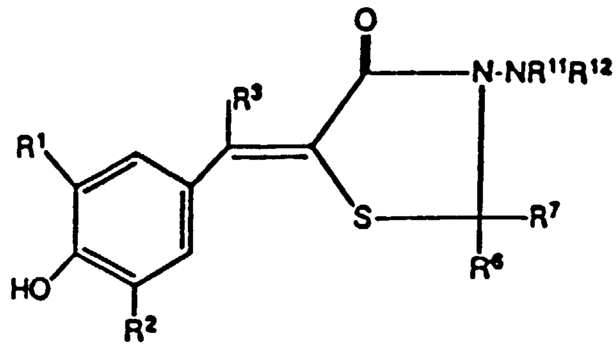

dans laquelle $R^6$ et $R^7$ pris ensemble représentent =S et $R^1$, $R^2$, $R^3$, $R^{11}$ et $R^{12}$ sont tels que définis à la formule I;

(Q) chauffer un composé de formule I dans laquelle $R^8$ représente $-(CH_2)_n-Y$ et Y représente $-NR^{11}R^{12}$ (aucun des radicaux $R^{11}$ ou $R^{12}$ ne représentant un atome d'hydrogène) dans un mélange éthanol/eau en présence d'un catalyseur pour préparer un composé de formule I dans laquelle $R^8$ représente $-(CH_2)_n-Y$ et Y représente $-NR^{11}R^{12}$ (où un des radicaux $R^{11}$ ou $R^{12}$ représente un atome d'hydrogène et l'autre ne représente pas un atome d'hydrogène);

(R) faire réagir un composé de formule I dans laquelle $R^6$ et $R^7$ représentent tous deux un atome d'hydrogène, avec de l'anhydride trifluoroacétique, pour préparer un composé de formule I dans laquelle un des radicaux $R^6$ et $R^7$ représente un atome d'hydrogène et l'autre représente -OH;

(S) salifier un composé de formule I en faisant réagir la forme non saline du composé, soit avec un acide fort, soit avec une base forte.

**10.** Composé de formule I, chaque fois préparé par un procédé selon la revendication 9.

**Revendications pour les Etats contractants suivants : ES, GR**

**1.** Procédé pour préparer un composé, ainsi que ses sels pharmaceutiquement acceptables, répondant à la formule (I) :

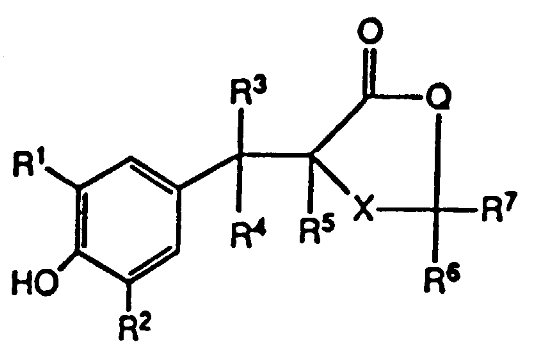

(I)

dans laquelle

$R^1$ et $R^2$ représentent, chacun indépendamment l'un de l'autre, un atome d'hydrogène, un groupe alkyle en $C_1$-$C_6$, un groupe alcoxy en $C_1$-$C_6$ ou un groupe alkyl(en $C_1$-$C_4$)

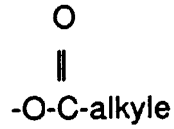

en $C_1$-$C_4$;

$R^3$ représente un atome d'hydrogène ou un groupe alkyle en $C_1$-$C_6$;

$R^4$ et $R^5$ représentent, chacun indépendamment l'un de l'autre, un atome d'hydrogène ou, lorsqu'ils sont pris ensemble, forment une liaison;

$R^6$ et $R^7$ représentent, chacun indépendamment l'un de l'autre, un atome d'hydrogène ou, lorsqu'ils sont pris ensemble, =S ou =O , ou encore lorsqu'un des radicaux $R^6$ ou $R^7$ représente un atome d'hydrogène, l'autre représente -OH ou $-SCH_3$;

X représente

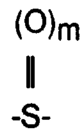

où m est égal à 0, 1 ou 2; et

Q représente $-CH_2-$, -O- ou $NR^8$ où $R^8$ représente un atome d'hydrogène, un groupe alkyle en $C_1$-$C_6$, un groupe cycloalkyle en $C_3$-$C_8$, un groupe alcényle en $C_2$-$C_6$, $-SO_2CH_3$ ou $-(CH_2)_n-Y$ où n représente un entier de 0 à 3, les deux étant inclus, et Y représente un groupe cyano, $OR^9$,

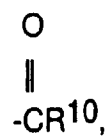

un groupe tétrazolyle, $-NR^{11}R^{12}$, -SH, -S(alkyle en $C_1$-$C_4$) ou

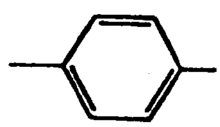

O-alkyle en $C_1$-$C_4$ où $R^9$ représente un atome d'hydrogène, un groupe alkyle en $C_1$-$C_4$, un groupe tosyle ou
un groupe

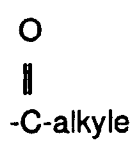

en $C_1$-$C_4$; $R^{10}$ représente un groupe OH, un alkyle en $C_1$-$C_4$, un groupe alcoxy en $C_1$-$C_4$ ou $-NH_2$; $R^{11}$ et $R^{12}$ représentent, chacun indépendamment l'un de l'autre, un atome d'hydrogène, un groupe alkyle en $C_1$-$C_6$, un groupe alcényle en $C_2$-$C_6$, un groupe alcynyle en $C_2$-$C_6$, $-(CH_2)_qOH$, $-(CH_2)_q$-N(alkyle en $C_1$-$C_4$)$_2$, $-(CH_2)_q$-S-(alkyle en $C_1$-$C_4$) ou

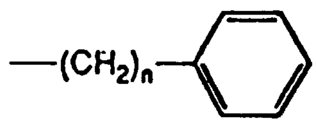

où q représente un entier de 1 à 6, les deux étant inclus, et n est tel que défini ci-dessus; ou bien $R^{11}$ et $R^{12}$ pris ensemble forment un noyau morpholinyle, un noyau pipéridinyle, un noyau pipérazinyle ou un noyau N-méthylpipérazinyle;

avec cette réserve que lorsque Q représente -O- ou $NR^8$ ($R^8$ représente un atome d'hydrogène ou un groupe alkyle en $C_1$-$C_4$), $R^3$ représente un atome d'hydrogène, $R^4$ et $R^5$ représentent chacun un atome d'hydrogène ou, lorsqu'ils sont pris ensemble, forment une liaison, et $R^6$ et $R^7$ représentent chacun un atome d'hydrogène ou, lorsqu'ils sont pris ensemble, représentent =S, et X représente

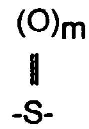

(où m est égal à 0), $R^1$ et $R^2$ ne peuvent représenter tous deux un groupe t-butyle;

et lorsque Q représente $NR^8$ ($R^8$ représente un atome d'hydrogène), $R^3$ représente un atome d'hydrogène, $R^6$ et $R^7$ pris ensemble représentent =S et
X représente $-S(O)_m-$ (où m est égal à 0), $R^4$ et $R^5$ doivent représenter tous deux un atome d'hydrogène; et lorsque Q représente $NR^8$ [$R^8$ représente un atome d'hydrogène ou $-(CH_2)_n-Y$ où n représente 0 et Y représente un groupe CO(alkyle en $C_1$-$C_4$)], $R^3$ représente un atome d'hydrogène, $R^4$ et $R^5$ pris ensemble forment une liaison, $R^6$ et $R^7$ représentent chacun un atome d'hydrogène et X représente $-S(O)_m-$ (où m est égal à 0), $R^1$ et $R^2$ ne peuvent représenter tous deux un groupe isopropyle,

qui comprend le fait de :

(A) faire réagir un composé de formule

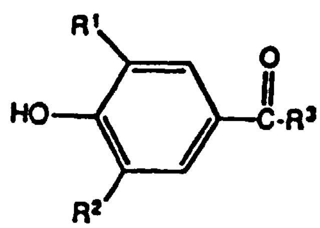

avec un composé de formule

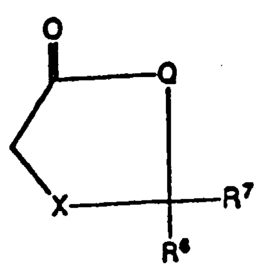

dans lesquelles $R^1$, $R^2$, $R^3$ et X sont tels qu'indiqués dans la formule I, Q représente $-CH_2-$ ou $NR^8$ (où $R^8$ est tel que défini dans la formule I), et $R^6$ et $R^7$ pris ensemble représentent =S pour obtenir un composé de formule

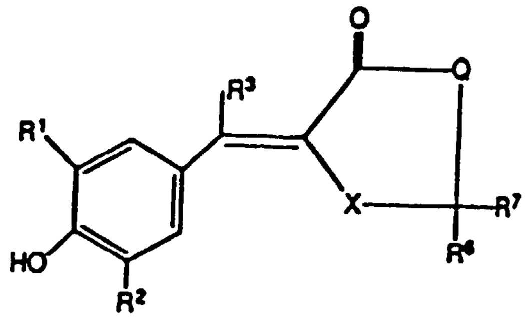

dans laquelle $R^1$, $R^2$, $R^3$, $R^6$, $R^7$, X et Q sont tels qu'indiqués ci-dessus;

(B) réduire un composé de formule I dans laquelle $R^6$ et $R^7$ pris ensemble représentent =S pour préparer un composé de formule I dans laquelle $R^6$ et $R^7$ représentent un atome d'hydrogène;

(C) réduire un composé de formule I dans laquelle $R^4$ et $R^5$ pris ensemble forment une liaison pour préparer un composé de formule I dans laquelle $R^4$ et $R^5$ représentent un atome d'hydrogène;

(D) réduire un composé de formule I dans laquelle $R^4$ et $R^5$ pris ensemble forment une liaison, et $R^6$ et $R^7$ pris ensemble représentent =S pour préparer un composé de formule I dans laquelle $R^4$, $R^5$, $R^6$ et $R^7$ représentent tous un atome d'hydrogène;

(E) alkyler un composé de formule I dans laquelle $R^8$ représente un atome d'hydrogène pour préparer un composé de formule I dans laquelle $R^8$ représente un groupe alkyle en $C_1$-$C_6$, un groupe alcényle en $C_2$-$C_6$, un groupe cycloalkyle en $C_3$-$C_8$ ou $-(CH_2)_n$-Y (où n représente un entier de 0 à 3, les deux étant inclus, et Y représente un groupe cyano, $OR^9$, -SH, -S(alkyle en $C_1$-$C_4$), $-NR^{11}R^{12}$ ou

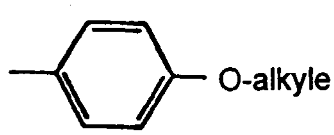

en $C_1$-$C_4$, où $R^9$, $R^{11}$ et $R^{12}$ sont tels que définis dans la formule I);

(F) acyler un composé de formule I dans laquelle $R^8$ représente un atome d'hydrogène pour préparer un composé de formule I dans laquelle $R^8$ représente $-(CH_2)_n$-Y où n représente un entier de 0 à 3, les deux étant inclus, et Y représente

$$\overset{\displaystyle O}{\overset{\|}{-CR^{10}}},$$

où $R^{10}$ est tel que défini à la formule I;

(G) oxyder un composé de formule I dans laquelle X représente

$$\overset{\displaystyle (O)_m}{\overset{\|}{-S-}},$$

où m est égal à 0, pour préparer un composé de formule I dans laquelle X représente

$$\overset{\displaystyle (O)_m}{\overset{\|}{-S-}}$$

et m est égal à 1;

(H) oxyder un composé de formule I dans laquelle X représente

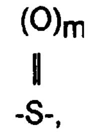

où m est égal à 0, pour préparer un composé de formule I dans laquelle X représente

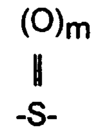

et m est égal à 2;

(I) oxyder un composé de formule I dans laquelle X représente

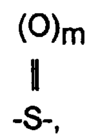

où m est égal à 1, pour préparer un composé de formule I dans laquelle X représente

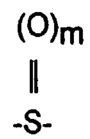

et m est égal à 2;

(J) faire réagir un composé de formule

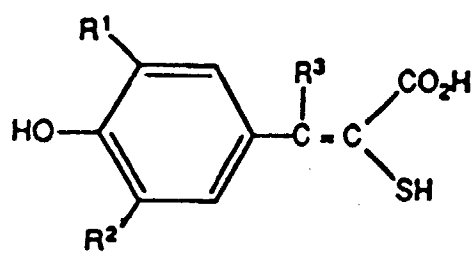

avec

i) de l'acide formique pour procurer un composé de formule I dans laquelle Q représente O, $R^4$ et $R^5$ pris ensemble forment une liaison et $R^6$ et $R^7$ représentent un atome d'hydrogène; ou
ii) du disulfure de carbone pour obtenir un composé de formule I dans laquelle Q représente O, $R^3$ et $R^4$ pris ensemble forment une liaison et $R^6$ et $R^7$ pris ensemble représentent =S;

(K) faire réagir un composé de formule

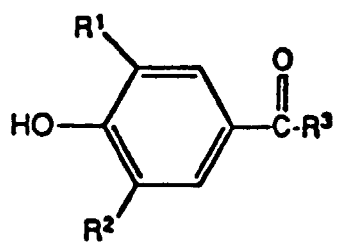

avec un composé de formule

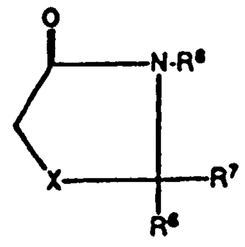

dans lesquelles $R^1$, $R^2$, $R^3$ et X sont tels que définis à la formule I, $R^6$ et $R^7$ pris ensemble représentent =S et $R^8$ représente $-(CH_2)_n-Y$ (où n représente un entier de 0 à 3, tous deux étant inclus, et Y représente $OR^9$ où $R^9$ représente un atome d'hydrogène) pour obtenir un composé de formule

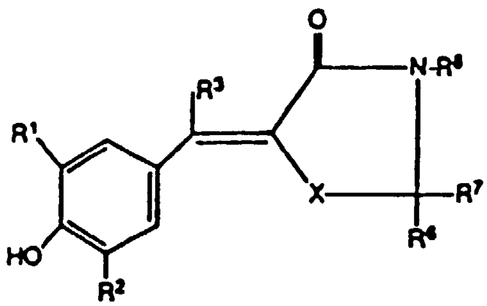

dans laquelle $R^1$, $R^2$, $R^3$, $R^6$, $R^7$ et X sont tels qu'indiqués ci-dessus et $R^8$ représente $-(CH_2)_n-Y$ (où n représente un entier de 0 à 3, tous deux étant inclus, et Y représente $OR^9$ où
$R^9$ représente

O

‖

$-C-CH_3$);

(L) réduire un composé de formule I dans laquelle $R^8$ représente $-(CH_2)_n-Y$ dans laquelle n représente de 0 à 3, tous deux étant inclus, et Y représente $-OR^9$ où $R^9$ représente un groupe

O

‖

-C-alkyle

en $C_1$-$C_4$, pour préparer un composé de formule I dans laquelle $R^8$ représente $-(CH_2)_n-Y$ dans laquelle n représente de 0 à 3, tous deux étant inclus, et Y représente $OR^9$ où $R^9$ représente un atome d'hydrogène;
(M) faire réagir un composé de formule I dans laquelle $R^8$ représente $-(CH_2)_n-Y$ dans laquelle n représente de 0 à 3, tous deux étant inclus, et Y représente $-OR^9$ où $R^9$ représente un atome d'hydrogène, avec un halogénure de tosyle pour préparer un composé de formule I dans laquelle $R^8$ représente $-(CH_2)_n-Y$ dans laquelle n représente de 0 à 3, tous deux étant inclus, et Y représente $OR^9$ où $R^9$ représente un groupe tosyle;
(N) faire réagir un composé de formule I dans laquelle $R^8$ représente $-(CH_2)_n-Y$ dans laquelle n représente de 0 à 3, tous deux étant inclus, et Y représente $-OR^9$ où $R^9$ représente un groupe tosyle, avec une amine de formule $HNR^{11}R^{12}$ (où $R^{11}$ et $R^{12}$ sont tels que définis par la formule I), pour préparer un composé de formule I dans laquelle $R^8$ représente $-(CH_2)_n-Y$ dans laquelle n représente de 0 à 3, tous deux étant inclus, et Y représente $-NR_{11}R_{12}$;
(O) traiter un composé de formule I dans laquelle $R^8$ représente $-(CH_2)_n-Y$ dans laquelle n représente de 0 à 3, tous deux étant indus, et Y représente un groupe cyano, avec de l'azide de tri-n-butylétain, pour préparer un composé de formule I dans laquelle $R^8$ représente $-(CH_2)_n-Y$ dans laquelle n représente de 0 à 3, tous deux étant inclus, et Y représente un groupe tétrazolyle;
(P) faire réagir un composé de formule

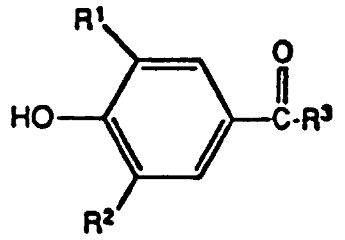

avec un composé de formule

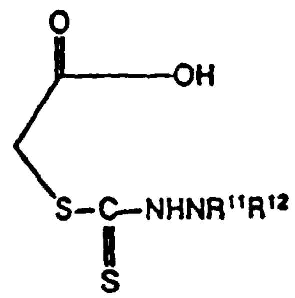

dans lesquelles $R^1$, $R^2$, $R^3$, $R^{11}$ et $R^{12}$ sont tels qu'indiqués en formule I, pour obtenir un composé de formule

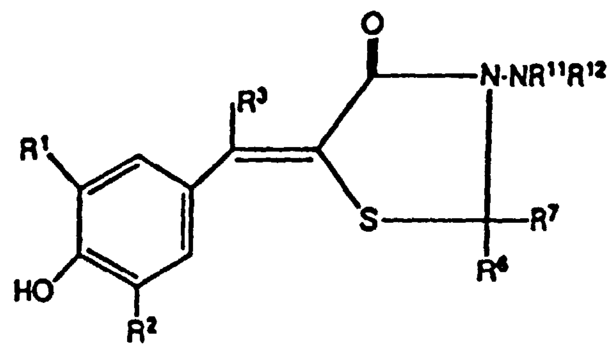

dans laquelle $R^6$ et $R^7$ pris ensemble représentent =S et $R^1$, $R^2$, $R^3$, $R^{11}$ et $R^{12}$ sont tels que définis à la formule I;

(Q) chauffer un composé de formule I dans laquelle $R^8$ représente $-(CH_2)_n$-Y et Y représente $-NR^{11}R^{12}$ (aucun des radicaux $R^{11}$ ou $R^{12}$ ne représentant un atome d'hydrogène) dans un mélange éthanol/eau en présence d'un catalyseur pour préparer un composé de formule I dans laquelle $R^8$ représente $-(CH_2)_n$-Y et Y représente $-NR^{11}R^{12}$ (où un des radicaux $R^{11}$ ou $R^{12}$ représente un atome d'hydrogène et l'autre ne représente pas un atome d'hydrogène);

(R) faire réagir un composé de formule I dans laquelle $R^6$ et $R^7$ représentent tous deux un atome d'hydrogène, avec de l'anhydride trifluoracétique, pour préparer un composé de formule I dans laquelle un des radicaux $R^6$ et $R^7$ représente un atome d'hydrogène et l'autre représente -OH;

(S) salifier un composé de formule I en faisant réagir la forme non saline du composé, soit avec un acide fort, soit avec une base forte.

**2.** Composition pharmaceutique comprenant, comme ingrédient actif, un composé de formule (I) tel que défini à la revendication 1, en association avec un ou plusieurs diluants, excipients ou supports pharmaceutiquement acceptables pour le composé.

**3.** Procédé pour préparer un médicament de formule (I), tel que défini à la revendication 1, à utiliser dans le traitement de l'inflammation et de l'arthrite chez les mammifères.

**4.** Procédé pour préparer un médicament de formule (II)

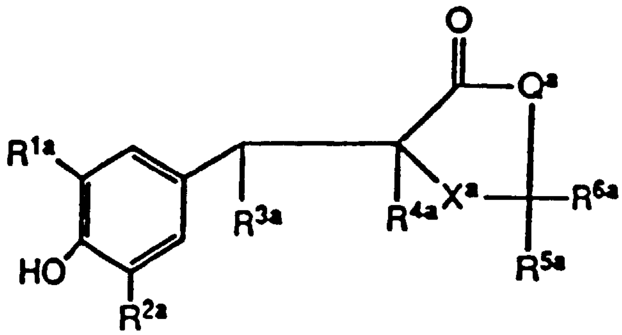

(II)

dans laquelle

| | |
|---|---|
| $R^{1a}$ et $R^{2a}$ | représentent, chacun indépendamment l'un de l'autre, un groupe alkyle en $C_1$-$C_6$, |
| $R^{3a}$ et $R^{4a}$ | représentent chacun un atome d'hydrogène ou, lorsqu'ils sont pris ensemble, forment une liaison; |
| $R^{5a}$ et $R^{6a}$ | représentent chacun un atome d'hydrogène ou, lorsqu'ils sont pris ensemble, représentent =O; et |
| $X^a$ | représente $-CH_2-$ ou 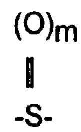 où m est égal à 0, 1 ou 2; et |
| $Q^a$ | représente $NR^{7a}$; |
| $R^{7a}$ | représente un atome d'hydrogène, un groupe alkyle en $C_1$-$C_6$ ou $-(CH_2)_n-Y^a$ où n représente un entier de 0 à 3, les deux étant inclus, et $Y^a$ représente un groupe cyano, $OR^{8a}$, 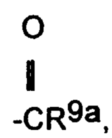 -SH, -S(alkyle en $C_1$-$C_4$), un groupe tétrazolyle, $-NR^{10a}R^{11a}$ ou 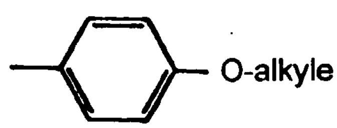 en $C_1$-$C_4$; où $R^{8a}$ représente un atome d'hydrogène, un groupe alkyle en $C_1$-$C_4$ ou un groupe 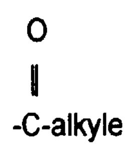 en $C_1$-$C_4$; $R^{9a}$ représente $-NH_2$ ou -OH; et $R^{10a}$ et $R^{11a}$ représentent, chacun indépendamment l'un de l'autre, un atome d'hydrogène, un groupe alkyle en $C_1$-$C_6$, un groupe alcényle en $C_2$-$C_6$ ou un groupe alcynyle en $C_2$-$C_6$; avec cette réserve que, lorsque $R^{3a}$ et $R^{4a}$ pris ensemble forment une liaison, $R^{5a}$ et $R^{6a}$ représentent chacun un atome d'hydrogène,<br>$X^a$ représente |

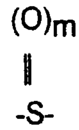

(où m est égal à 0), et $R^{7a}$ représente un atome d'hydrogène ou un groupe alkyle en $C_1$-$C_4$, $R^{1a}$ et $R^{2a}$ ne peuvent représenter ensemble un groupe t-butyle, à utiliser pour empêcher la dégradation cellulaire induite par ischémie chez les mammifères.

**5.** Procédé pour préparer un médicament de formule (III)

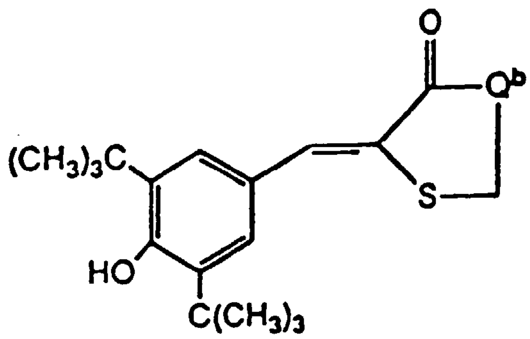

(III)

dans laquelle

$Q^b$ représente -O- ou $NR^{7b}$ où $R^{7b}$ représente un atome d'hydrogène, un groupe alkyle en $C_1$-$C_6$, $NR^{8b}R^{9b}$ ou $-(CH_2)_n$-OH où $R^{8b}$ et $R^{9b}$ représentent, chacun indépendamment l'un de l'autre, un atome d'hydrogène ou un groupe alkyle en $C_1$-$C_4$, et n représente un entier de 0 à 3, les deux étant inclus, à utiliser dans le traitement d'un mammifère dystrophique.